# EUROPEAN PATENT SPECIFICATION

(11) **EP 4 218 409 B1**
(45) Date of publication and mention of the grant of the patent: **12.02.2025**
(21) Application number: 23151398.7
(22) Date of filing: 13.03.2014
(51) Int. Cl.: A01K 67/027, C12N 15/85, C07K 16/00, C07K 16/46

(54) **MICE EXPRESSING A LIMITED IMMUNOGLOBULIN LIGHT CHAIN REPERTOIRE**
MÄUSE, DIE EIN BEGRENZTES IMMUNGLOBULIN-LEICHTKETTENREPERTOIR EXPRIMIEREN
SOURIS EXPRIMANT UN RÉPERTOIRE DE CHAÎNES LÉGÈRES D'IMMUNOGLOBULINE LIMITÉ

(30) Priority: 13.03.2013 US 201313798455
(43) Date of publication of application: 02.08.2023
(62) Divisional of application: 18210518.9
(73) Proprietor: Regeneron Pharmaceuticals, Inc., Tarrytown, NY 10591-6706 (US)
(72) Inventor: BABB, Robert, New Jersey, 07661 (US); MCWHIRTER, John, New Jersey, 10591 (US); MACDONALD, Lynn, New York, 10528 (US); STEVENS, Sean, California, 92122 (US); DAVIS, Samuel, New York, 10591 (US); BUCKLER, David R., New York, 10591 (US); MEAGHER, Karolina A., 10598, New York (US); MURPHY, Andrew J., New York, 10520 (US)
(74) Representative: J A Kemp LLP

(56) References cited:
- WO-A1-2010/070263
- WO-A1-2011/163314
- WO-A1-2013/022782
- WO-A2-2012/148873
- US-A1- 2013 045 492

## Description

### FIELD

The present invention relates to mouse ES cells and mouse embryos, as well as methods for making genetically modified mouse cells and mice. A genetically modified mouse is described that expresses antibodies having a common human variable/mouse constant light chain associated with diverse human variable/mouse constant heavy chains. Also described are methods for making a human bispecific antibody from human variable region gene sequences of B cells of the mouse described.

### BACKGROUND

Antibodies typically comprise a homodimeric heavy chain component, wherein each heavy chain monomer is associated with an identical light chain. Antibodies having a heterodimeric heavy chain component (*e*.*g*., bispecific antibodies) are desirable as therapeutic antibodies. But making bispecific antibodies having a suitable light chain component that can satisfactorily associate with each of the heavy chains of a bispecific antibody has proved problematic.

In one approach, a light chain might be selected by surveying usage statistics for all light chain variable domains, identifying the most frequently employed light chain in human antibodies, and pairing that light chain *in vitro* with the two heavy chains of differing specificity.

In another approach, a light chain might be selected by observing light chain sequences in a phage display library (*e*.*g*., a phage display library comprising human light chain variable region sequences, *e*.*g*., a human scFv library) and selecting the most commonly used light chain variable region from the library. The light chain can then be tested on the two different heavy chains of interest.

In another approach, a light chain might be selected by assaying a phage display library of light chain variable sequences using the heavy chain variable sequences of both heavy chains of interest as probes. A light chain that associates with both heavy chain variable sequences might be selected as a light chain for the heavy chains.

In another approach, a candidate light chain might be aligned with the heavy chains' cognate light chains, and modifications are made in the light chain to more closely match sequence characteristics common to the cognate light chains of both heavy chains. If the chances of immunogenicity need to be minimized, the modifications preferably result in sequences that are present in known human light chain sequences, such that proteolytic processing is unlikely to generate a T cell epitope based on parameters and methods known in the art for assessing the likelihood of immunogenicity (*i.e., in silico* as well as wet assays).

All of the above approaches rely on in vitro methods that subsume a number of a priori restraints, e.g., sequence identity, ability to associate with specific pre-selected heavy chains, *etc.* There is a need in the art for compositions and methods that do not rely on manipulating in vitro conditions, but that instead employ more biologically sensible approaches to making human epitope-binding proteins that include a common light chain.

### SUMMARY

Genetically modified mice that express human immunoglobulin heavy and light chain variable domains, wherein the mice have a limited light chain variable repertoire, are described. The present invention provides a mouse embryonic stem (ES) cell that has been genetically modified so that it comprises in its genome:
exactly two unrearranged human immunoglobulin Vκ gene segments and five unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment.

The present invention further provides a mouse embryo derived from such a genetically modified ES cell.

The present invention further provides a method of making a genetically modified mouse ES cell, the method comprising genetically modifying an ES cell so that it comprises in its genome exactly two unrearranged human immunoglobulin Vκ gene segments and five unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment.

The present invention also provides a method of making a genetically modified mouse comprising genetically modifying the germline genome of the mouse so that it includes exactly two unrearranged human immunoglobulin Vκ gene segments and five

unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment.

Also referred to is a biological system for generating a human light chain variable domain that associates and expresses with a diverse repertoire of affinity-matured human heavy chain variable domains. Also referred to are methods for making binding proteins comprising immunoglobulin variable domains, comprising immunizing mice that have a limited immunoglobulin light chain repertoire with an antigen of interest, and employing an immunoglobulin variable region gene sequence of the mouse in a binding protein that specifically binds the antigen of interest. Methods include methods for making human immunoglobulin heavy chain variable domains suitable for use in making multi-specific antigen-binding proteins.

Also referred to are genetically engineered mice that select suitable affinity-matured human immunoglobulin heavy chain variable domains derived from a repertoire of unrearranged human heavy chain variable region gene segments, wherein the affinity-matured human heavy chain variable domains associate and express with a single human light chain variable domain derived from one human light chain variable region gene segment. Genetically engineered mice that present a choice of two human light chain variable region gene segments are described. The two gene segments are human Vκ1-39 and human Vκ3-20.

Also referred to are genetically engineered mice that express a limited repertoire of human light chain variable domains, or a single human light chain variable domain, from a limited repertoire of human light chain variable region gene segments. The mice are genetically engineered to include two unrearranged human light chain variable region gene segments that rearrange to form two rearranged light chain variable region genes that expresses either or both of two light chains. The rearranged human light chain variable domains are capable of pairing with a plurality of affinity-matured human heavy chains selected by the mice, wherein the heavy chain variable regions specifically bind different epitopes.

Also referred to are genetically engineered mice that express a limited repertoire of human light chain variable domains, or a single human light chain variable domain, from a limited repertoire of human light chain variable region sequences. Also referred to are mice are genetically engineered to include a single V/J human light chain sequence (or two V/J sequences) that express a variable region of a single light chain (or that express either or both of two variable regions). A light chain comprising the variable sequence is capable of pairing with a plurality of affinity-matured human heavy chains clonally selected by the mice, wherein the heavy chain variable regions specifically bind different epitopes.

Also referred to is a genetically modified mouse that comprises a single human immunoglobulin light chain variable (V_{L}) region gene segment that is capable of rearranging with a human J gene segment (selected from one or a plurality of J_{L} segments) and encoding a human V_{L} domain of an immunoglobulin light chain. The genetically modified mouse employed in the present invention comprises exactly two human V_{L} gene segments, each of which is capable of rearranging with a human J gene segment (selected from one or a plurality of J_{L} segments) and encoding a human V_{L} domain of an immunoglobulin light chain. In some instances, the two human V_{L} gene segments are juxtaposed in the genome of the mouse. In some instances, the two human V_{L} gene segments are at different loci (e.g., a heterozygote, comprising a first human V_{L} segment at a first light chain allele, and a second human V_{L} segment at a second light chain allele, wherein the first and the second human V_{L} segments are not identical) in the genome of the mouse. The two human V_{L} gene segments are a human Vκ1-39 gene segment and a human Vκ3-20 gene segment. The human J_{L} gene are human Jκ1, Jκ2, Jκ3, Jκ4, Jκ5. In various embodiments, the genetically engineered mouse is incapable of expressing an immunoglobulin light chain that contains an endogenous V_{L} gene segment. For example, in some embodiments, a genetically engineered mouse employed contains a genetic modification that inactivates and/or removes part or all of an endogenous V_{L} gene segment.

Also referred to is a genetically modified mouse that comprises an immunoglobulin light chain locus that does not comprise an endogenous mouse V_{L} gene segment that is capable of rearranging to form an immunoglobulin light chain gene, wherein the V_{L} locus contains a single human V_{L} gene segment that is capable of rearranging to encode a V_{L} region of a light chain gene. In specific instances, the human V_{L} gene segment is a human Vκ1-39Jκ5 gene segment or a human Vκ3-20Jκ1 gene segment. Also referred to is a genetically modified mouse comprises a V_{L} locus that does not comprise an endogenous mouse V_{L} gene segment that is capable of rearranging to form an immunoglobulin light chain gene, wherein the V_{L} locus comprises no more than two human V_{L} gene segments that are capable of rearranging to encode a V_{L} region of a light chain gene. The no more than two human V_{L} gene segments are selected from the group consisting of a human Vκ1-39 gene segment, a human Vκ3-20 gene segment, and a combination thereof. In some certain instances, the no more than two human V_{L} gene segments are a human Vκ1-39Jκ5 gene segment and a human Vκ3-20Jκ1 gene segment.

Also referred to is a genetically modified mouse that comprises a single rearranged (V/J) human immunoglobulin light chain variable (V_{L}) region *(i.e.,* a V_{L}/J_{L} region) that encodes a human V_{L} domain of an immunoglobulin light chain. In another instance, the mouse comprises no more than two rearranged human V_{L} regions that are capable of encoding a human V_{L} domain of an immunoglobulin light chain.

In one instance, the V_{L} region is a rearranged human Vκ1-39/J sequence or a rearranged human Vκ3-20/J sequence. In one instance, the human J_{L} segment of the rearranged V_{L}/J_{L} sequence is selected from Jκ1, Jκ2, Jκ3, Jκ4, and Jκ5. In a specific instance, the V_{L} region is a human Vκ1-39Jκ5 sequence or a human Vκ3-20Jκ1 sequence. The mouse has both a human Vκ1-39Jκ5 sequence and a human Vκ3-20Jκ1 sequence.

In one embodiment, the human V_{L} gene segment is operably linked to a human or mouse leader sequence. In one embodiment, the leader sequence is a mouse leader sequence. In a specific embodiment, the mouse leader sequence is a mouse Vκ3-7 leader sequence. In a specific embodiment, the leader sequence is operably linked to an unrearranged human V_{L} gene segment.

In one embodiment, the V_{L} gene segment is operably linked to an immunoglobulin promoter sequence. In one embodiment, the promoter sequence is a human promoter sequence. In a specific embodiment, the human immunoglobulin promoter is a human Vκ3-15 promoter. In a specific embodiment, the promoter is operably linked to an unrearranged human V_{L} gene segment.

In one embodiment, the light chain locus comprises a leader sequence flanked 5' (with respect to transcriptional direction of a V_{L} gene segment) with a human immunoglobulin promoter and flanked 3' with a human V_{L} gene segment that rearranges with a human J segment and encodes a V_{L} domain of a reverse chimeric light chain comprising an endogenous mouse light chain constant region (C_{L}). In a specific embodiment, the V_{L} gene segment is at the mouse Vκ locus, and the mouse C_{L} is a mouse Cκ.

In one instance, the light chain locus comprises a leader sequence flanked 5' (with respect to transcriptional direction of a V_{L} gene segment) with a human immunoglobulin promoter and flanked 3' with a rearranged human V_{L} region (V_{L}/J_{L} sequence) and encodes a V_{L} domain of a reverse chimeric light chain comprising an endogenous mouse light chain constant region (C_{L}). In a specific instance, the rearranged human V_{L}/J_{L} sequence is at the mouse kappa (κ) locus, and the mouse C_{L} is a mouse Cκ.

In one embodiment, the V_{L} locus of the modified mouse is a κ light chain locus, and the κ light chain locus comprises a mouse κ intronic enhancer, a mouse κ 3' enhancer, or both an intronic enhancer and a 3' enhancer.

In one embodiment, the mouse comprises a nonfunctional immunoglobulin lambda (λ) light chain locus. In a specific instance, the λ light chain locus comprises a deletion of one or more sequences of the locus, wherein the one or more deletions renders the λ light chain locus incapable of rearranging to form a light chain gene. In another embodiment, all or substantially all of the V_{L} gene segments of the λ light chain locus are deleted.

In one embodiment, mouse makes a light chain that comprises a somatically mutated V_{L} domain derived from a human V_{L} gene segment. In one embodiment, the light chain comprises a somatically mutated V_{L} domain derived from a human V_{L} gene segment, and a mouse Cκ region. In one embodiment, the mouse does not express a λ light chain.

In one embodiment, the genetically modified mouse is capable of somatically hypermutating the human V_{L} region sequence. In a specific embodiment, the mouse comprises a cell that comprises a rearranged immunoglobulin light chain gene derived from a human V_{L} gene segment that is capable of rearranging and encoding a V_{L} domain, and the rearranged immunoglobulin light chain gene comprises a somatically mutated V_{L} domain.

In one embodiment, the mouse comprises a cell that expresses a light chain comprising a somatically mutated human V_{L} domain linked to a mouse Cκ, wherein the light chain associates with a heavy chain comprising a somatically mutated V_{H} domain derived from a human V_{H} gene segment and wherein the heavy chain comprises a mouse heavy chain constant region (C_{H}). In a specific embodiment, the heavy chain comprises a mouse C_{H}1, a mouse hinge, a mouse C_{H}2, and a mouse C_{H}3. In a specific embodiment, the heavy chain comprises a human C_{H}1, a hinge, a mouse C_{H}2, and a mouse C_{H}3.

In one embodiment, the mouse comprises a replacement of endogenous mouse V_{H} gene segments with one or more human V_{H} gene segments, wherein the human V_{H} gene segments are operably linked to a mouse C_{H} region gene, such that the mouse rearranges the human V_{H} gene segments and expresses a reverse chimeric immunoglobulin heavy chain that comprises a human V_{H} domain and a mouse C_{H}. In one embodiment, 90-100% of unrearranged mouse V_{H} gene segments are replaced with at least one unrearranged human V_{H} gene segment. In a specific embodiment, all or substantially all of the endogenous mouse V_{H} gene segments are replaced with at least one unrearranged human V_{H} gene segment. In one embodiment, the replacement is with at least 19, at least 39, or at least 80 or 81 unrearranged human V_{H} gene segments. In one embodiment, the replacement is with at least 12 functional unrearranged human V_{H} gene segments, at least 25 functional unrearranged human V_{H} gene segments, or at least 43 functional unrearranged human V_{H} gene segments. In one embodiment, the mouse comprises a replacement of all mouse D_{H} and J_{H} segments with at least one unrearranged human D_{H} segment and at least one unrearranged human J_{H} segment. In one embodiment, the at least one unrearranged human D_{H} segment is selected from 1-1, D1-7, 1-26, 2-8, 2-15, 3-3, 3-10, 3-16, 3-22, 5-5, 5-12, 6-6, 6-13, 7-27, and a combination thereof. In one embodiment, the at least one unrearranged human J_{H} segment is selected from 1, 2, 3, 4, 5, 6, and a combination thereof. In a specific embodiment, the one or more human V_{H} gene segment is selected from a 1-2, 1-8, 1-24, 1-69, 2-5, 3-7, 3-9, 3-11, 3-13, 3-15, 3-20, 3-23, 3-30, 3-33, 3-48, 3-53, 4-31, 4-39, 4-59, 5-51, a 6-1 human V_{H} gene segment, and a combination thereof.

In one embodiment, the mouse comprises a B cell that expresses a binding protein that specifically binds an antigen of interest, wherein the binding protein comprises a light chain derived from a human Vκ1-39/Jκ5 rearrangement or a human Vκ3-20/Jκ1 rearrangement, and wherein the cell comprises a rearranged immunoglobulin heavy chain gene derived from a rearrangement of human V_{H} gene segments selected from a 1-69, 2-5, 3-13, 3-23, 3-30, 3-33, 3-53, 4-39, 4-59, and 5-51 gene segment. In one embodiment, the one or more human V_{H} gene segments are rearranged with a human heavy chain J_{H} gene segment selected from 1, 2, 3, 4, 5, and 6. In one embodiment, the one or more human V_{H} and J_{H} gene segments are rearranged with a human D_{H} gene segment selected from 1-1, 1-7, 1-26, 2-8, 2-15, 3-3, 3-10, 3-16, 3-22, 5-5, 5-12, 6-6, 6-13, and 7-27. In a specific embodiment, the light chain gene has 1, 2, 3, 4, or 5 or more somatic hypermutations.

In one embodiment, the mouse comprises a B cell that comprises a rearranged immunoglobulin heavy chain variable region gene sequence comprising a V_{H}/D_{H}/J_{H} region selected from 2-5/6-6/1, 2-5/3-22/1, 3-13/6-6/5, 3-23/2-8/4, 3-23/3-3/4, 3-23/3-10/4, 3-23/6-6/4, 3-23/7-27/4, 3-30/1-1/4, 3-30/1-7/4, 3-30/3-3/3, 3-30/3-3/4, 3-30/3-22/5, 3-30/5-5/2, 3-30/5-12/4, 3-30/6-6/1, 3-30/6-6/3, 3-30/6-6/4, 3-30/6-6/5, 3-30/6-13/4, 3-30/7-27/4, 3-30/7-27/5, 3-30/7-27/6, 3-33/1-7/4, 3-33/2-15/4, 4-39/1-26/3, 4-59/3-16/3, 4-59/3-16/4, 4-59/3-22/3, 5-51/3-16/6, 5-51/5-5/3, 5-51/6-13/5, 3-53/1-1/4, 1-69/6-6/5, and 1-69/6-13/4. In a specific embodiment, the B cell expresses a binding protein comprising a human immunoglobulin heavy chain variable region fused with a mouse heavy chain constant region, and a human immunoglobulin light chain variable region fused with a mouse light chain constant region.

In one embodiment, the rearranged human V_{L} region is a human Vκ1-39Jκ5 sequence, and the mouse expresses a reverse chimeric light chain comprising (i) a V_{L} domain derived from the human V_{L}/J_{L} sequence and (ii) a mouse C_{L}; wherein the light chain is associated with a reverse chimeric heavy chain comprising (i) a mouse C_{H} and (ii) a somatically mutated human V_{H} domain derived from a human V_{H} gene segment selected from a 1-2, 1-8, 1-24, 1-69, 2-5, 3-7, 3-9, 3-11, 3-13, 3-15, 3-20, 3-23, 3-30, 3-33, 3-48, 3-53, 4-31, 4-39, 4-59, 5-51, a 6-1 human V_{H} gene segment, and a combination thereof. In one embodiment, the mouse expresses a light chain that is somatically mutated. In one embodiment the C_{L} is a mouse Cκ. In a specific embodiment, the human V_{H} gene segment is selected from a 2-5, 3-13, 3-23, 3-30, 4-59, 5-51, and 1-69 gene segment. In a specific embodiment, the somatically mutated human V_{H} domain comprises a sequence derived from a D_{H} segment selected from 1-1, 1-7, 2-8, 3-3, 3-10, 3-16, 3-22, 5-5, 5-12, 6-6, 6-13, and 7-27. In a specific embodiment, the somatically mutated human V_{H} domain comprises a sequence derived from a J_{H} segment selected from 1, 2, 3, 4, 5, and 6. In a specific embodiment, the somatically mutated human V_{H} domain is encoded by a rearranged human V_{H}/D_{H}/J_{H} sequence selected from 2-5/6-6/1, 2-5/3-22/1, 3-13/6-6/5, 3-23/2-8/4, 3-23/3-3/4, 3-23/3-10/4, 3-23/6-6/4, 3-23/7-27/4, 3-30/1-1/4, 3-30/1-7/4, 3-30/3-3/4, 3-30/3-22/5, 3-30/5-5/2, 3-30/5-12/4, 3-30/6-6/1, 3-30/6-6/3, 3-30/6-6/4, 3-30/6-6/5, 3-30/6-13/4, 3-30/7-27/4, 3-30/7-27/5, 3-30/7-27/6, 4-59/3-16/3, 4-59/3-16/4, 4-59/3-22/3, 5-51/5-5/3, 1-69/6-6/5, and 1-69/6-13/4.

In one embodiment, the rearranged human V_{L} region is a human Vκ3-20Jκ1 sequence, and the mouse expresses a reverse chimeric light chain comprising (i) a V_{L} domain derived from the rearranged human V_{L}/J_{L} sequence, and (ii) a mouse C_{L}; wherein the light chain is associated with a reverse chimeric heavy chain comprising (i) a mouse C_{H}, and (ii) a somatically mutated human V_{H} derived from a human V_{H} gene segment selected from a 1-2, 1-8, 1-24, 1-69, 2-5, 3-7, 3-9, 3-11, 3-13, 3-15, 3-20, 3-23, 3-30, 3-33, 3-48, 3-53, 4-31, 4-39, 4-59, 5-51, a 6-1 human V_{H} gene segment, and a combination thereof. In one embodiment, the mouse expresses a light chain that is somatically mutated. In one embodiment the C_{L} is a mouse Cκ. In a specific embodiment, the human V_{H} gene segment is selected from a 3-30, 3-33, 3-53, 4-39, and 5-51 gene segment. In a specific embodiment, the somatically mutated human V_{H} domain comprises a sequence derived from a D_{H} segment selected from 1-1, 1-7, 1-26, 2-15, 3-3, 3-16, and 6-13. In a specific embodiment, the somatically mutated human V_{H} domain comprises a sequence derived from a J_{H} segment selected from 3, 4, 5, and 6. In a specific embodiment, the somatically mutated human V_{H} domain is encoded by a rearranged human V_{H}/D_{H}/J_{H} sequence selected from 3-30/1-1/4, 3-30/3-3/3, 3-33/1-7/4, 3-33/2-15/4, 4-39/1-26/3, 5-51/3-16/6, 5-51/6-13/5, and 3-53/1-1/4.

Also referred to is a mouse that comprises both a rearranged human Vκ1-39Jκ5 sequence and a rearranged human Vκ3-20Jκ1 sequence, and the mouse expresses a reverse chimeric light chain comprising (i) a V_{L} domain derived from the human Vκ1-39Jκ5 sequence or the human Vκ3-20Jκ1 sequence, and (ii) a mouse C_{L}; wherein the light chain is associated with a reverse chimeric heavy chain comprising (i) a mouse C_{H}, and (ii) a somatically mutated human V_{H} derived from a human V_{H} gene segment selected from a 1-2, 1-8, 1-24, 1-69, 2-5, 3-7, 3-9, 3-11, 3-13, 3-15, 3-20, 3-23, 3-30, 3-33, 3-48, 3-53, 4-31, 4-39, 4-59, 5-51, a 6-1 human V_{H} gene segment, and a combination thereof. In one instance, the mouse expresses a light chain that is somatically mutated. In one instance the C_{L} is a mouse Cκ.

In one embodiment, 90-100% of the endogenous unrearranged mouse V_{H} gene segments are replaced with at least one unrearranged human V_{H} gene segment. In a specific embodiment, all or substantially all of the endogenous unrearranged mouse V_{H} gene segments are replaced with at least one unrearranged human V_{H} gene segment. In one embodiment, the replacement is with at least 18, at least 39, at least 80, or 81 unrearranged human V_{H} gene segments. In one embodiment, the replacement is with at least 12 functional unrearranged human V_{H} gene segments, at least 25 functional unrearranged human V_{H} gene segments, or at least 43 unrearranged human V_{H} gene segments.

In one embodiment, the genetically modified mouse is a C57BL strain, in a specific embodiment selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, and C57BL/Ola. In a specific embodiment, the genetically modified mouse is a mix of an aforementioned 129 strain and an aforementioned C57BL/6 strain. In another specific embodiment, the mouse is a mix of aforementioned 129 strains, or a mix of aforementioned BL/6 strains. In a specific embodiment, the 129 strain of the mix is a 129S6 (129/SvEvTac) strain.

Also described is a mouse that expresses a reverse chimeric antibody comprising a light chain that comprises a mouse Cκ and a somatically mutated human V_{L} domain derived from a rearranged human Vκ1-39Jκ5 sequence or a rearranged human Vκ3-20Jκ1 sequence, and a heavy chain that comprises a mouse C_{H} and a somatically mutated human V_{H} domain derived from a human V_{H} gene segment selected from a 1-2, 1-8, 1-24, 1-69, 2-5, 3-7, 3-9, 3-11, 3-13, 3-15, 3-20, 3-23, 3-30, 3-33, 3-48, 3-53, 4-31, 4-39, 4-59, 5-51, and a 6-1 human V_{H} gene segment, wherein the mouse does not express a fully mouse antibody and does not express a fully human antibody. In one instance the mouse comprises a κ light chain locus that comprises a replacement of endogenous mouse κ light chain gene segments with the rearranged human Vκ1-39Jκ5 sequence or the rearranged human Vκ3-20Jκ1 sequence, and comprises a replacement of all or substantially all endogenous mouse V_{H} gene segments with a complete or substantially complete repertoire of human V_{H} gene segments.

Also described is a population of antigen-specific antibodies derived from a mouse as described herein, wherein the antibodies comprise a light chain gene derived from a human Vκ1-39/Jκ5 rearrangement or a human Vκ3-20/Jκ1 rearrangement, and wherein the antibodies comprise a rearranged immunoglobulin heavy chain gene derived from a rearrangement of a human V_{H} gene segment selected from a 1-2, 1-3, 1-8, 1-18, 1-24, 1-46, 1-58, 1-69, 2-5, 2-26, 2-70, 3-7, 3-9, 3-11, 3-13, 3-15, 3-16, 3-20, 3-21, 3-23, 3-30, 3-33, 3-43, 3-48, 3-53, 3-64, 3-72, 3-73, 4-31, 4-34, 4-39, 4-59, 5-51, and a 6-1 human V_{H} gene segment. In one instance, the one or more human V_{H} gene segments are rearranged with a human heavy chain J_{H} gene segment selected from 1, 2, 3, 4, 5, and 6. In a specific instance, the light chain has 1, 2, 3, 4, or 5 or more somatic hypermutations.

In one instance, the light chain has 1, 2, 3, or 4 somatic hypermutations. In one instance, the light chain gene has 1 or 2 mutations. In various instances, the light chain gene is capable of incurring multiple mutations along its sequence.

In one instance, the light chain is derived from a human Vκ1-39/Jκ5 rearrangement and the light chain has at least one or no more than four somatic hypermutations. In one instance, the light chain comprises at least two somatic hypermutations. In one instance, the light chain comprises at least three somatic hypermutations. In one instance the light chain comprises at least four somatic hypermutations. In a specific instance, at least one such somatic hypermutation is present in one or more framework regions (FWs) of the light chain. In a specific instance, at least one such somatic hypermutation is present in one or more complementarity determining regions (CDRs) of the light chain. In a specific instance, at least one such somatic hypermutation is present in one or more FWs and/or one or more CDRs of the light chain. In various instances, the framework regions are selected from framework 1 (FW1), framework 2 (FW2), framework 3 (FW3), and/or a combination thereof. In various instances, the CDRs are selected from CDR1, CDR2, CDR3, and/or a combination thereof.

In one instance, the heavy chain comprises at least one mutation in one or more FWs or one or more CDRs. In one instance, the heavy chain comprises at least one mutation in one or more FWs and one or more CDRs. In one instance, the heavy chain comprises at least two mutations in one or more FWs and one or more CDRs. In one instance, the heavy chain comprises at least three mutations in one or more FWs and one or more CDRs. In one instance, the heavy chain comprises at least four mutations in one or more FWs and one or more CDRs. In one instance, the heavy chain comprises at least five or more than five mutations in one or more FWs and one or more CDRs; in a specific instance, the heavy chain comprises at least five or more than five mutations in two FWs; in a specific instance, the heavy chain comprises at least five or more than five mutations in one FW and one CDR.

In one instance, the light chain is derived from a human Vκ1-39/Jκ5 rearrangement and about 9% of the Vκ1-39/Jκ5-derived light chains have at least one mutation present in FW1; in one instance, at least 9% of the light chains comprise one mutation present in FW1. In one instance, the light chain is derived from a human Vκ1-39/Jκ5 rearrangement and about 25% of the Vκ1-39/Jκ5-derived light chains have at least one or no more than two mutations present in CDR1; in one instance, at least 19% of the light chains have one mutation present in CDR1; in one instance, at least 5% of the light chains have two mutations present in CDR1.

In one instance, the light chain is derived from a human Vκ1-39/Jκ5 rearrangement and about 20% of the Vκ1-39/Jκ5-derived light chains have at least one or no more than three mutations present in FW2; in one instance, at least 17% of the light chains have one mutation present in FW2; in one instance, at least 1% of the light chains have two mutations present in FW2; in one instance, at least 1% of the light chains have three mutations present in FW2.

In one instance, the light chain is derived from a human Vκ1-39/Jκ5 rearrangement and about 10% of the Vκ1-39/Jκ5-derived light chains have at least one or no more than two mutations present in CDR2; in one instance, at least 10% of the light chains have one mutation present in CDR2; in one instance, at least 1% of the light chains have two mutations present in CDR2.

In one instance, the light chain is derived from a human Vκ1-39/Jκ5 rearrangement and about 29% of the Vκ1-39/Jκ5-derived light chains have at least one or no more than four mutations present in FW3; in one instance, at least 21% of the light chains have one mutation present in FW3; in one instance, at least 5% of the light chains have two mutations present in FW3; in one instance, at least 2% of the light chains have three mutations present in FW3; in one instance, at least 2% of the light chains have four mutations present in FW3.

In one instance, the light chain is derived from a human Vκ1-39/Jκ5 rearrangement and about 37% of the Vκ1-39/Jκ5-derived light chains have at least one or no more than four mutations present in CDR3; in one instance, at least 27% of the light chains have one mutation present in CDR3; in one instance, at least 8% of the light chains have two mutations present in CDR3; in one instance, at least 1% of the light chains have three mutations present in CDR3; in one instance, at least 1% of the light chains have four mutations present in CDR3.

Also described is a population of antigen-specific antibodies derived from a mouse as described herein, wherein the antibodies comprise a light chain derived from a human Vκ1-39/Jκ5 rearrangement and about 9% of the Vκ1-39/Jκ5-derived light chains have one or more mutations present in FW1, about 25% of the Vκ1-39/Jκ5-derived light chains have one or more mutations present in CDR1, about 20% of the Vκ1-39/Jκ5-derived light chains have one or more mutations present in FW2, about 10% of the Vκ1-39/Jκ5-derived light chains have one or more mutations present in CDR2, about 29% of the Vκ1-39/Jκ5-derived light chains have one or more mutations present in FW3, and about 37% of the Vκ1-39/Jκ5-derived light chains have one or more mutations present in CDR3.

In one instance, the light chain is derived from a human Vκ1-39/Jκ5 rearrangement and about 35% of the heavy chains have at least one mutation present in FW1; in one instance, at least 25% of the heavy chains have one mutation present in FW1; in one instance, at least 9 % of the heavy chains have two mutations present in FW1; in one instance, at least 1% of the heavy chains have three mutations present in FW1; in one instance, at least 1% of the heavy chains have more than five mutations present in FW1.

In one instance, the light chain is derived from a human Vκ1-39/Jκ5 rearrangement and about 92% of the heavy chains have at least one or no more than four mutations present in CDR1; in one instance, at least 92% of the heavy chains have at least one, at least two, at least three, or at least four mutations present in CDR1; in one instance, at least 26% of the heavy chains have one mutation present in CDR1; in one instance, at least 44% of the heavy chains have two mutations present in CDR1; in one instance, at least 19% of the heavy chains have three mutations present in CDR1; in one instance, at least 3% of the heavy chains have four mutations present in CDR1.

In one instance, the light chain is derived from a human Vκ1-39/Jκ5 rearrangement and about 66% of the heavy chains have at least one or no more than three mutations present in FW2; in one instance, at least 66% of the heavy chains have at least one, at least two, or at least three mutations present in FW2; in one instance, at least 35% of the heavy chains have one mutation present in FW2; in one instance, at least 23% of the heavy chains have two mutations present in FW2; in one instance, at least 8% of the heavy chains have three mutations present in FW2.

In one instance, the light chain is derived from a human Vκ1-39/Jκ5 rearrangement and about 70% of the heavy chains have at least one or no more than four mutations present in CDR2; in one instance, at least 70% of the heavy chains have at least two, at least three, or at least four mutations present in CDR2; in one instance, at least 34% have one mutation present in CDR2; in one instance, at least 20% of the heavy chains have two mutations present in CDR2; in one instance, at least 12% of the heavy chains have three mutations present in CDR2; in one instance, at least 5% of the heavy chains have four mutations present in CDR2.

In one instance, the light chain is derived from a human Vκ1-39/Jκ5 rearrangement and about 91% of the heavy chains have at least one or up to five or more mutations present in FW3; in one instance, at least 91% of the heavy chains have at least two, at least three, at least four, or at least five or more mutations present in FW3; in one instance, at least 19% of the heavy chains have one mutation present in FW3; in one instance, at least 33% of the heavy chains have two mutations present in FW3; in one instance, at least 22% of the heavy chains have three mutations present in FW3; in one instance, at least 11% of the heavy chains have four mutations present in FW3; in one instance, at least 7% of the heavy chains have five or more mutations present in FW3.

In one instance, the light chain is derived from a human Vκ1-39/Jκ5 rearrangement and about 63% of the heavy chains have at least one or no more than two mutations present in CDR3; in one instance, at least 63% of the heavy chains have at one mutation present in CDR3; in one instance, at least 54% of the heavy chains have one mutation present in CDR3; in one instance, at least 9% of the heavy chains have two mutations present in CDR3.

Also described is a population of antigen-specific antibodies derived from a mouse as described herein, wherein the antibodies comprise a light chain derived from a human Vκ1-39/Jκ5 rearrangement and at least 35% of the heavy chains have one or more mutations present in FW1, about 92% of the heavy chains have one or more mutations present in CDR1, about 66% of the heavy chains have one or more mutations present in FW2, about 70% of the heavy chains have one or more mutations present in CDR2, about 91% of the heavy chains have one or more mutations present in FW3, and about 63% of the heavy chains have one or more mutations present in CDR3.

In one instance, the light chain is derived from a human Vκ3-20/Jκ1 rearrangement and the light chain gene has at least one or no more than two somatic hypermutations; in one instance, the light chain gene has at least two, at least three, at least four or more somatic hypermutations. In a specific instance, the mutations are present in one or more framework regions of the light chain. In a specific instance, the mutations are present in one or more CDR regions of the light chain. In a specific instance, the mutations are present in one or more framework regions and/or one or more CDR regions of the light chain. In various instances, the framework regions are selected from framework 1 (FW1), framework 2 (FW2), framework 3 (FW3), and/or a combination thereof.

In one instance, the light chain is derived from a human Vκ3-20/Jκ1 rearrangement and about 10% of the Vκ3-20/Jκ1-derived light chains have at least one mutation present in FW1; in one instance, at least 10% of the light chains have one mutation in FW1.

In one instance, the light chain is derived from a human Vκ3-20/Jκ1 rearrangement and about 53% of the Vκ3-20/Jκ1-derived light chains have at least one or no more than two mutations present in CDR1; in one instance, at least 27% of the light chains have one or more mutations in CDR1; in one instance, about 54% of the light chains have one or two mutations present in CDR1.

In one instance, the light chain is derived from a human Vκ3-20/Jκ1 rearrangement and about 6% of the Vκ3-20/Jκ1-derived light chains have at least one or no more than two mutations present in FW2; in one instance, at least 6% of light chains have at least one mutation present in FW2; in one instance, at least 3% of the light chains have one mutation present in FW2; in one instance, at least 3% of the light chains have two mutations present in FW2.

In one instance, the light chain is derived from a human Vκ3-20/Jκ1 rearrangement and at least about 3% of the Vκ3-20/Jκ1-derived light chains have at least one mutation present in CDR2; in one instance, at least 3% of the light chains have one mutation in CDR2.

In one instance, the light chain is derived from a human Vκ3-20/Jκ1 rearrangement and about 17% or more of the Vκ3-20/Jκ1-derived light chains have at least one or no more than two mutations present in FW3; in one instance, at least 20% of the light chain have one mutation present in FW3; in one instance, at least 17% of the light chains have two mutations present in FW3.

In one instance, the light chain is derived from a human Vκ3-20/Jκ1 rearrangement and at least 43% of the Vκ3-20/Jκ1-derived light chains have at least one mutation present in CDR3; in one instance, at least 43% of the light chains have one mutation in CDR3.

Also described is a population of antigen-specific antibodies derived from a mouse as described herein, wherein the antibodies comprise a light chain derived from a human Vκ3-20/Jκ1 rearrangement and about 10% of the Vκ3-20/Jκ1-derived light chains have one or more mutations present in at least, about 53% of the Vκ3-20/Jκ1-derived light chains have one or more mutations present in CDR1, about 6% of the Vκ3-20/Jκ1-derived light chains have one or more mutations present in FW2, about 3% of the Vκ3-20/Jκ1-derived light chains have one or more mutations present in CDR2, about 37% of the Vκ3-20/Jκ1-derived light chains have one or more mutations present in FW3, and about 43% of the Vκ3-20/Jκ1-derived light chains have one or more mutations present in CDR3.

In one instance, the light chain is derived from a human Vκ3-20/Jκ1 rearrangement and about 43% of the heavy chains have at least one or no more than two mutations present in FW1; in one instance, at least 41% of the heavy chains have at least one mutation present in FW1; in one instance, about 41% of the heavy chains have one mutation present in FW1; in one instance, about 2% of the heavy chains have two mutations present in FW1.

In one instance, the light chain is derived from a human Vκ3-20/Jκ1 rearrangement and about 92% of the heavy chains have at least one or no more than four mutations present in CDR1; in one instance, at least 43% of heavy chains have at least one mutation present in CDR1; in one instance, at least 25% of heavy chains have at least two mutations present in CDR1; in one instance, at least 15% of heavy chains have at least 3 mutations present in CDR1; in one instance, at least 10% of heavy chains have 4 or more mutations present in CDR1.

In one instance, the light chain is derived from a human Vκ3-20/Jκ1 rearrangement and about 46% of the heavy chains have at least one or no more than three mutations present in FW2; in one instance, at least 34% of heavy chains have at least one mutation present in FW2; in one instance, at least 10% of heavy chains have two or more mutations present in FW2; in one instance, at least 2% of heavy chains have three or more mutations present in FW2.

In one instance, the light chain is derived from a human Vκ3-20/Jκ1 rearrangement and about 84% of the heavy chains have at least one or up to five or more than five mutations present in CDR2; in one instance, at least 39% of the heavy chains have one or more mutations present in CDR2; in one instance, at least 18% of the heavy chains have two or more mutations present in CDR2; in one instance, at least 21% of the heavy chains have three or more mutations present in CDR2; in one instance, at least 3% of the heavy chains have four or more mutations present in CDR2; in one instance, at least 2% of the heavy chains have five or more mutations present in CDR2.

In one instance, the light chain is derived from a human Vκ3-20/Jκ1 rearrangement and about 92% of the heavy chains have at least one or up to five or more than five mutations present in FW3; in one instance, at least 21% of the light chains have at least one mutation present in FW3; in one instance, at least 20% of heavy chains have at least two mutations present in FW3; in one instance, at least 13% of the heavy chains have at least three mutations present in FW3; in one instance, at least 20% of the heavy chains have at least four mutations in FW3; in one instance, at least 18% of the heavy chains have at lest 5 mutations in FW3.

In one instance, the light chain is derived from a human Vκ3-20/Jκ1 rearrangement and about 7% of the heavy chains have at least one mutation present in CDR3; in one instance, about 7% of the heavy chains have one mutation in CDR3.

Also described is a population of antigen-specific antibodies derived from a mouse as described herein, wherein the antibodies comprise a light chain derived from a human Vκ3-20/Jκ1 rearrangement and about 43% of the heavy chains have one or more mutations present in FW1, about 92% of the heavy chains have one or more mutations present in CDR1, about 46% of the heavy chains have one or more mutations present in FW2, about 84% of the heavy chains have one or more mutations present in CDR2, about 92% of the heavy chains have one or more mutations present in FW3, and about 7% of the heavy chains have one or more mutations present in CDR3.

Also referred to is a mouse that expresses an immunoglobulin light chain from a rearranged immunoglobulin light chain sequence, wherein the rearranged immunoglobulin light chain sequence is present in the germline of the mouse, wherein the immunoglobulin light chain comprises a human variable sequence. In one instance, the germline of the mouse comprises a rearranged immunoglobulin light chain sequence that is derived from the same V segment and the same J segment as all non-surrogate light chain sequences present in every B cell of the mouse that comprises a rearranged light chain sequence.

In one instance, the germline of the mouse lacks a functional unrearranged immunoglobulin light chain V gene segment. In one instance, the germline of the mouse lacks a functional unrearranged immunoglobulin light chain J gene segment.

In one instance, the germline of the mouse comprises no more than one, no more than two, or no more than three rearranged (V/J) light chain sequences.

In one instance, the rearranged V/J sequence comprises a κ light chain sequence. In a specific instance, the κ light chain sequence is a human κ light chain sequence. In a specific instance, the κ light chain sequence is selected from a human Vκ1-39/J sequence, a human Vκ3-20/J sequence, and a combination thereof. In a specific instance, the κ light chain sequence is a human Vκ1-39/Jκ5 sequence. In a specific instance, the κ light chain sequence is a human Vκ3-20/Jκ1 sequence.

In one embodiment, the mouse further comprises in its germline a sequence selected from a mouse κ intronic enhancer 5' with respect to the rearranged immunoglobulin light chain sequence, a mouse κ 3' enhancer, and a combination thereof.

In one embodiment, the mouse comprises an unrearranged human V_{H} gene segment, an unrearranged human D_{H} gene segment, and an unrearranged human J_{H} gene segment, wherein said V_{H}, D_{H}, and J_{H} gene segments are capable of rearranging to form an immunoglobulin heavy chain variable gene sequence operably linked to a heavy chain constant gene sequence. In one embodiment, the mouse comprises a plurality of human V_{H}, D_{H}, and J_{H} gene segments. In a specific embodiment, the human V_{H}, D_{H}, and J_{H} gene segments replace endogenous mouse V_{H}, D_{H}, and J_{H} gene segments at the endogenous mouse immunoglobulin heavy chain locus. In a specific embodiment, the mouse comprises a replacement of all or substantially all functional mouse V_{H}, D_{H}, and J_{H} gene segments with all or substantially all functional human V_{H}, D_{H}, and J_{H} gene segments.

In one embodiment, the mouse expresses an immunoglobulin light chain that comprises a mouse constant sequence. In one embodiment, the mouse expresses an immunoglobulin light chain that comprises a human constant sequence.

In one embodiment, the mouse expresses an immunoglobulin heavy chain that comprises a mouse sequence selected from a C_{H}1 sequence, a hinge sequence, a C_{H}2 sequence, a C_{H}3 sequence, and a combination thereof.

In one embodiment, the mouse expresses an immunoglobulin heavy chain that comprises a human sequence selected from a C_{H}1 sequence, a hinge sequence, a C_{H}2 sequence, a C_{H}3 sequence, and a combination thereof.

In one instance, the rearranged immunoglobulin light chain sequence in the germline of the mouse is at an endogenous mouse immunoglobulin light chain locus. In a specific instance, the rearranged immunoglobulin light chain sequence in the germline of the mouse replaces all or substantially all mouse light chain V and J sequences at the endogenous mouse immunoglobulin light chain locus.

Also referred to is a mouse that comprises a B cell population characterized by each B cell that comprises a non-surrogate light chain sequence, which sequence comprises a rearranged light chain gene that is generated from a single human V gene segment and a single human J gene segment, wherein the only light chain variable sequence in the germline of the mouse is a rearranged sequence generated from the single human V segment and the single human J segment, and wherein each B cell that comprises the rearranged light chain gene further comprises a gene encoding a cognate human heavy chain variable domain, and wherein the rearranged light chain gene comprises at least one, at least two, at least three, or at least four somatic hypermutations.

Also referred to is a mouse whose mature B cell population is characterized in that each mature B cell comprises a non-surrogate light chain sequence on its surface, which sequence comprises a rearranged light chain gene that is generated through rearrangement of one of two human V_{L} gene segments and one of no more than five human J_{L} gene segments, wherein the only light chain variable sequence (V_{L}J_{L} sequence) in the germline of the mouse is a rearranged sequence that is generated through rearrangement of one of the two human V_{L} gene segments and one of the no more than five human J_{L} gene segments, and wherein each B cell that comprises the rearranged light chain gene further comprises a gene encoding a cognate human heavy chain variable domain, and wherein the rearranged light chain gene comprises at least one, at least two, at least three, at least four, or five or more somatic hypermutations. In some embodiments, a rearranged light chain gene comprises one, two, three, four, or five somatic hypermutations. In some embodiments, mice as described herein have been immunized with an antigen of interest, and, in some embodiments, a mature B cell population is enriched with B cells that bind the antigen of interest.

In some embodiments, the mouse made using a method of the invention is one whose mature B cell population is characterized in that each mature B cell comprises a non-surrogate light chain sequence on its surface, which sequence comprises a rearranged light chain gene that is generated through rearrangement of one of two human V_{L} gene segments and one of 5 human J_{L} gene segments, wherein the V_{L} gene segments consist essentially of two V_{L} gene segments that are not identical and the V_{L} locus comprises 5 human J_{L} gene segments, and wherein each B cell that comprises the rearranged light chain gene further comprises a gene encoding a cognate human heavy chain variable domain, and wherein the rearranged light chain gene comprises at least one, at least two, at least three, at least four, or five or more somatic hypermutations. In some embodiments, a rearranged light chain gene comprises one, two, three, four, or five somatic hypermutations. In some embodiments, mice as described herein have been immunized with an antigen of interest, and in some embodiments, a mature B cell population is enriched with B cells that bind the antigen of interest.

In one aspect, a pluripotent, induced pluripotent, or totipotent cell derived from a mouse as described herein is provided. In a specific embodiment, the cell is a mouse embryonic stem (ES) cell. The present invention provides a mouse embryonic stem (ES) cell that has been genetically modified so that it comprises in its genome:
exactly two unrearranged human immunoglobulin Vκ gene segments and five unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment.

In one instance, a tissue derived from a mouse as described herein is disclosed. In one instance, the tissue is derived from spleen, lymph node or bone marrow of a mouse as described herein.

Also refererred to is a nucleus derived from a mouse as described herein. In one instance, the nucleus is from a diploid cell that is not a B cell.

In one embodiment, a mouse ES cell of the invention is isolated from a mouse as described herein Also referred to is a mouse cell that is isolated from a mouse as described herein.. In one instance, the cell is a lymphocyte. In one instance, the lymphocyte is a B cell. In one Instance, the B cell expresses a chimeric heavy chain comprising a variable domain derived from a human gene segment; and a light chain derived from a rearranged human Vκ1-39/J sequence, rearranged human Vκ3-20/J sequence, or a combination thereof; wherein the heavy chain variable domain is fused to a mouse constant region and the light chain variable domain is fused to a mouse or a human constant region.

Also referred to is a hybridoma, wherein the hybridoma is made with a B cell of a mouse as described herein. In a specific instance, the B cell is from a mouse as described herein that has been immunized with an immunogen comprising an epitope of interest, and the B cell expresses a binding protein that binds the epitope of interest, the binding protein has a somatically mutated human V_{H} domain and a mouse C_{H}, and has a human V_{L} domain derived from a rearranged human Vκ1-39Jκ5 or a rearranged human Vκ3-20Jκ1 and a mouse C_{L}.

In one aspect, a mouse embryo is provided, wherein the embryo comprises a donor ES cell that is derived from a mouse as described herein. The present invention provides a mouse embryo that is derived from a genetically modified ES cell of the present invention.

Also referred to is a targeting vector, comprising, from 5' to 3' in transcriptional direction with reference to the sequences of the 5' and 3' mouse homology arms of the vector, a 5' mouse homology arm, a human or mouse immunoglobulin promoter, a human or mouse leader sequence, and a human V_{L} region selected from a rearranged human Vκ1-39Jκ5 or a rearranged human Vκ3-20Jκ1, and a 3' mouse homology arm. In one instance, the 5' and 3' homology arms target the vector to a sequence 5' with respect to an enhancer sequence that is present 5' and proximal to the mouse Cκ gene. In one instance, the promoter is a human immunoglobulin variable region gene segment promoter. In a specific instance, the promoter is a human Vκ3-15 promoter. In one instance, the leader sequence is a mouse leader sequence. In a specific instance, the mouse leader sequence is a mouse Vκ3-7 leader sequence.

Also referred to is a targeting vector as described above, but in place of the 5' mouse homology arm the human or mouse promoter is flanked 5' with a site-specific recombinase recognition site (SRRS), and in place of the 3' mouse homology arm the human V_{L} region is flanked 3' with an SRRS.

In one aspect, a reverse chimeric antibody made by a mouse as described herein, wherein the reverse chimeric antibody comprises a light chain comprising a human V_{L} and a mouse C_{L}, and a heavy chain comprising a human V_{H} and a mouse C_{H}.

Also disclosed is a method for making an antibody, comprising expressing in a single cell (a) a first V_{H} gene sequence of an immunized mouse as described herein fused with a human C_{H} gene sequence; (b) a V_{L} gene sequence of an immunized mouse as described herein fused with a human C_{L} gene sequence; and, (c) maintaining the cell under conditions sufficient to express a fully human antibody, and isolating the antibody. In one instance, the cell comprises a second V_{H} gene sequence of a second immunized mouse as described herein fused with a human C_{H} gene sequence, the first V_{H} gene sequence encodes a V_{H} domain that recognizes a first epitope, and the second V_{H} gene sequence encodes a V_{H} domain that recognizes a second epitope, wherein the first epitope and the second epitope are not identical.

Also disclosed is a method for making an epitope-binding protein, comprising exposing a mouse as described herein with an immunogen that comprises an epitope of interest, maintaining the mouse under conditions sufficient for the mouse to generate an immunoglobulin molecule that specifically binds the epitope of interest, and isolating the immunoglobulin molecule that specifically binds the epitope of interest; wherein the epitope-binding protein comprises a heavy chain that comprises a somatically mutated human V_{H} and a mouse C_{H}, associated with a light chain comprising a mouse C_{L} and a human V_{L} derived from a rearranged human Vκ1-39Jκ5 or a rearranged human Vκ3-20Jκ1.

Also referred to is a cell that expresses an epitope-binding protein, wherein the cell comprises: (a) a human nucleotide sequence encoding a human V_{L} domain that is derived from a rearranged human Vκ1-39Jκ5 or a rearranged human Vκ3-20Jκ1, wherein the human nucleotide sequence is fused (directly or through a linker) to a human immunoglobulin light chain constant domain cDNA sequence (e.g., a human κ constant domain DNA sequence); and, (b) a first human V_{H} nucleotide sequence encoding a human V_{H} domain derived from a first human V_{H} nucleotide sequence, wherein the first human V_{H} nucleotide sequence is fused (directly or through a linker) to a human immunoglobulin heavy chain constant domain cDNA sequence; wherein the epitope-binding protein recognizes a first epitope. In one instance, the epitope-binding protein binds the first epitope with a dissociation constant of lower than 10⁻⁶ M, lower than 10⁻⁸ M, lower than 10⁻⁹ M, lower than 10⁻¹⁰ M, lower than 10⁻¹¹ M, or lower than 10⁻¹² M.

In one instance, the cell comprises a second human nucleotide sequence encoding a second human V_{H} domain, wherein the second human sequence is fused (directly or through a linker) to a human immunoglobulin heavy chain constant domain cDNA sequence, and wherein the second human V_{H} domain does not specifically recognize the first epitope (e.g., displays a dissociation constant of, e.g., 10⁻⁶ M, 10⁻⁵ M, 10⁻⁴ M, or higher), and wherein the epitope-binding protein recognizes the first epitope and the second epitope, and wherein the first and the second immunoglobulin heavy chains each associate with an identical light chain of (a).

In one instance, the second V_{H} domain binds the second epitope with a dissociation constant that is lower than 10⁻⁶ M, lower than 10⁻⁷ M, lower than 10⁻⁸ M, lower than 10⁻⁹ M, lower than 10⁻¹⁰ M, lower than 10⁻¹¹ M, or lower than 10⁻¹² M.

In oneinstance, the epitope-binding protein comprises a first immunoglobulin heavy chain and a second immunoglobulin heavy chain, each associated with an identical light chain derived from a rearranged human V_{L} region selected from a human Vκ1-39Jκ5 or a human Vκ3-20Jκ1, wherein the first immunoglobulin heavy chain binds a first epitope with a dissociation constant in the nanomolar to picomolar range, the second immunoglobulin heavy chain binds a second epitope with a dissociation constant in the nanomolar to picomolar range, the first epitope and the second epitope are not identical, the first immunoglobulin heavy chain does not bind the second epitope or binds the second epitope with a dissociation constant weaker than the micromolar range (e.g., the millimolar range), the second immunoglobulin heavy chain does not bind the first epitope or binds the first epitope with a dissociation constant weaker than the micromolar range (e.g., the millimolar range), and one or more of the V_{L}, the V_{H} of the first immunoglobulin heavy chain, and the V_{H} of the second immunoglobulin heavy chain, are somatically mutated.

In one instance, the first immunoglobulin heavy chain comprises a protein A-binding residue, and the second immunoglobulin heavy chain lacks the protein A-binding residue.

In one instance, the cell is selected from CHO, COS, 293, HeLa, and a retinal cell expressing a viral nucleic acid sequence (e.g., a PERC.6^{™} cell).

Also referred to is a reverse chimeric antibody, comprising a human V_{H} and a mouse heavy chain constant domain, a human V_{L} and a mouse light chain constant domain, wherein the antibody is made by a process that comprises immunizing a mouse as described herein with an immunogen comprising an epitope, and the antibody specifically binds the epitope of the immunogen with which the mouse was immunized. In one instance, the V_{L} domain is somatically mutated. In one instance ihe V_{H} domain is somatically mutated. In one instance, both the V_{L} domain and the V_{H} domain are somatically mutated. In one instance, the V_{L} is linked to a mouse Cκ domain.

Also referred to is a mouse, comprising human V_{H} gene segments replacing all or substantially all mouse V_{H} gene segments at the endogenous mouse heavy chain locus; no more than one or two rearranged human light chain V_{L}/J_{L} sequences selected from a rearranged Vκ1-39/J and a rearranged Vκ3-20/J or a combination thereof, replacing all mouse light chain gene segments; wherein the human heavy chain variable gene segments are linked to a mouse constant gene, and the rearranged human light chain sequences are linked to a human or mouse constant gene.

Also referred to is a mouse comprising human immunoglobulin V_{H} gene segments replacing all or substantially all mouse immunoglobulin V_{H} gene segments at the endogenous mouse immunoglobulin heavy chain locus; no more than two unrearranged human immunoglobulin V_{L} gene segments and two or more (e.g., 2, 3, 4 or 5) unrearranged human immunoglobulin J_{L} gene segments or five human immunoglobulin J_{L} gene segments, replacing all mouse immunoglobulin light chain gene segments; wherein the human immunoglobulin V_{H} gene segments are linked to a mouse immunoglobulin constant gene, and the unrearranged human immunoglobulin V_{L} and J_{L} gene segments are linked to a human or non-human immunoglobulin constant gene. In some instances, a non-human constant gene is a mouse immunoglobulin constant gene. In some embodiments, a non-human immunoglobulin constant gene is a rat immunoglobulin constant gene.

Also referred to is a mouse ES cell comprising a replacement of all or substantially all mouse heavy chain variable gene segments with human heavy chain variable gene segments, and no more than one or two rearranged human light chain V_{L}/J_{L} sequences, wherein the human heavy chain variable gene segments are linked to a mouse immunoglobulin heavy chain constant gene, and the rearranged human light chain V_{L}/J_{L} sequences are linked to a mouse or human immunoglobulin light chain constant gene. In a specific instance, the light chain constant gene is a mouse constant gene.

Also referred to is a mouse ES cell, comprising a replacement of all or substantially all mouse immunoglobulin V_{H} gene segments with human immunoglobulin V_{H} gene segments and two unrearranged human immunoglobulin V_{L} gene segments and 5 unrearranged human immunoglobulin J_{L} gene segments, wherein the human immunoglobulin V_{H} gene segments are linked to a mouse immunoglobulin heavy chain constant gene, and the unrearranged human immunoglobulin V_{L} and J_{L} gene segments are linked to a non-human or human immunoglobulin light chain constant gene. In some certain instances, the non-human immunoglobulin light chain constant gene is a mouse immunoglobulin constant gene. In some certain instances, the mouse comprises five unrearranged immunoglobulin J_{L} gene segments.

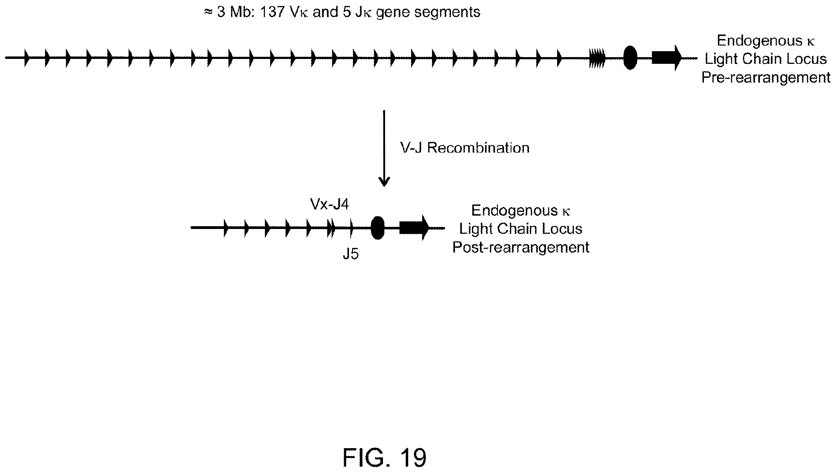

Also referred to is a mouse comprising a light chain locus whose structure is different from that of the reference structure of FIG. 19 in that at least one, and in some instances all, mouse V_{L} gene segments are replaced by one human V_{L} gene segment or no more than two human V_{L} gene segments. In some instances, human V_{L} gene segments of a mouse are capable of rearranging to one of two or more human J_{L} gene segments to encode an immunoglobulin V_{L} domain of an antibody. In some instance, human V_{L} gene segment(s) of a light chain locus of a mouse as described herein is/are operably linked to two or more (e.g., two, three, four, or five) human J_{L} gene segments.

Also referred to is a mouse, comprising a light chain locus whose structure is different from that of the reference structure of FIG. 19 in that it does not contain a nucleotide sequence before rearrangement that encodes an endogenous V_{L} gene segment. Also referred to is a mouse, comprising a light chain locus whose structure is different from that of the reference structure of FIG. 19 in that it does not contain a nucleotide sequence before rearrangement that encodes an endogenous J_{L} gene segment. Also referred to is a mouse, comprising a light chain locus whose structure is different from that of the reference structure of FIG. 19 in that it does not contain a nucleotide before rearrangement that encodes endogenous V_{L} and J_{L} gene segments.

Also referred to is a mouse, comprising a light chain locus whose structure is different from that of the reference structure of FIG. 19 in that it does not contain a nucleotide sequence after rearrangement that encodes an endogenous V_{L} gene segment. Also referred to is a mouse, comprising a light chain locus whose structure is different from that of the reference structure of FIG. 19 in that it does not contain a nucleotide sequence after rearrangement that encodes an endogenous J_{L} gene segment. Also referred to is a mouse, comprising a light chain locus whose structure is different from that of the reference structure of FIG. 19 in that it does not contain a nucleotide sequence after rearrangement that encodes endogenous V_{L} and J_{L} gene segments.

Also referred to is a mouse, comprising a light chain locus whose structure is different from that of the reference structure of FIG. 19 in that it contains no more than two human V_{L} gene segments and two or more (e.g., two, three, four, or five) human J_{L} gene segments before rearrangement. Also referred to is a mouse, comprising a light chain locus whose structure is different from that of the reference structure of FIG. 19 in that it contains two human V_{L} gene segments and five human J_{L} gene segments before rearrangement.

Also referred to is a mouse, comprising a light chain locus whose structure is different from that of the reference structure of FIG. 19 in that it contains two human V_{L} gene segments and five human J_{L} gene segments after rearrangement. Also referred to is a mouse, comprising a light chain locus whose structure is different from that of the reference structure of FIG. 19 in that it contains two human V_{L} gene segments and five human J_{L} gene segments after rearrangement.

Also referred to is a mouse, comprising a light chain locus whose structure is different from that of the reference structure of FIG. 19 in that it contains one human V_{L} gene segment and five or less (e.g., 5, 4, 3, 2, or 1) human J_{L} gene segments after rearrangement. Also referred to is a mouse, comprising a light chain locus whose structure is different from that of the reference structure of FIG. 19 in that it contains one human V_{L} gene segment and one, two, three, four, or five human J_{L} gene segments after rearrangement.

In various embodiments, human V_{L} and J_{L} gene segments are human Vκ and Jκ gene segments. Human Vκ segments are selected from a human Vκ1-39 gene segment and a human Vκ3-20 gene segment. Human Jκ gene segments are human Jκ1, Jκ2, Jκ3, Jκ4, and Jκ5.

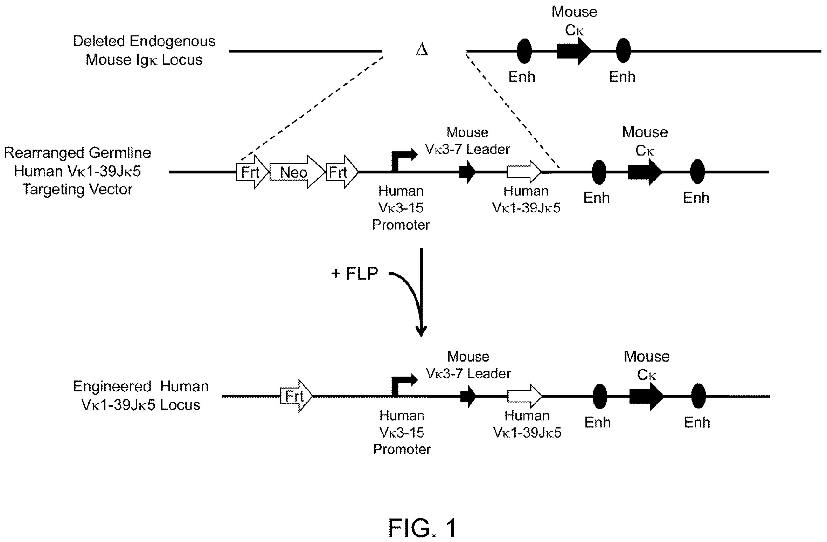

Also referred to is a mouse comprising a light chain locus whose structure is different from that of the reference structure of FIG. 19 in that it contains a structure that is substantially the same as that of the structure of FIG. 1, FIG. 2, FIG. 3, or FIG. 9 before rearrangement. In some embodiments, a mouse comprises a light chain locus whose structure is identical to the structure of FIG. 1, FIG. 2, FIG. 3 or FIG. 9 before rearrangement.

Also referred to is an antigen-binding protein made by a mouse as described herein. In a specific instance, the antigen-binding protein comprises a human immunoglobulin heavy chain variable region fused with a mouse constant region, and a human immunoglobulin light chain variable region derived from a Vκ1-39 gene segment or a Vκ3-20 gene segment, wherein the light chain constant region is a mouse constant region.

Also referred to is a fully human antigen-binding protein made from an immunoglobulin variable region gene sequence from a mouse as described herein, wherein the antigen-binding protein comprises a fully human heavy chain comprising a human variable region derived from a sequence of a mouse as described herein, and a fully human light chain comprising a Vκ1-39 or a Vκ3-20. In one instance, the light chain variable region comprises one to five somatic mutations. In one instance, the light chain variable region is a cognate light chain variable region that is paired in a B cell of the mouse with the heavy chain variable region.

In one instance, the fully human antigen-binding protein comprises a first heavy chain and a second heavy chain, wherein the first heavy chain and the second heavy chain comprise non-identical variable regions independently derived from a mouse as described herein, and wherein each of the first and second heavy chains express from a host cell associated with a human light chain derived from a Vκ1-39 gene segment or a Vκ3-20 gene segment. In one instance, the first heavy chain comprises a first heavy chain variable region that specifically binds a first epitope of a first antigen, and the second heavy chain comprises a second heavy chain variable region that specifically binds a second epitope of a second antigen. In a specific instance, the first antigen and the second antigen are different. In a specific instance, the first antigen and the second antigen are the same, and the first epitope and the second epitope are not identical; in a specific embodiment, binding of the first epitope by a first molecule of the binding protein does not block binding of the second epitope by a second molecule of the binding protein.

Also referred to is a fully human binding protein derived from a human immunoglobulin sequence of a mouse as described herein that comprises a first immunoglobulin heavy chain and a second immunoglobulin heavy chain, wherein the first immunoglobulin heavy chain comprises a first variable region that is not identical to a variable region of the second immunoglobulin heavy chain, and wherein the first immunoglobulin heavy chain comprises a wild type protein A binding determinant, and the second heavy chain lacks a wild type protein A binding determinant. In one instance, the first immunoglobulin heavy chain binds protein A under isolation conditions, and the second immunoglobulin heavy chain does not bind protein A or binds protein A at least 10-fold, a hundred-fold, or a thousand-fold weaker than the first immunoglobulin heavy chain binds protein A under isolation conditions. In a specific instance, the first and the second heavy chains are IgG1 isotypes, wherein the second heavy chain comprises a modification selected from 95R (EU 435R), 96F (EU 436F), and a combination thereof, and wherein the first heavy chain lacks such modification.

Also referred to is a method for making a bispecific antigen-binding protein, comprising exposing a first mouse as described herein to a first antigen of interest that comprises a first epitope, exposing a second mouse as described herein to a second antigen of interest that comprises a second epitope, allowing the first and the second mouse to each mount immune responses to the antigens of interest, identifying in the first mouse a first human heavy chain variable region that binds the first epitope of the first antigen of interest, identifying in the second mouse a second human heavy chain variable region that binds the second epitope of the second antigen of interest, making a first fully human heavy chain gene that encodes a first heavy chain that binds the first epitope of the first antigen of interest, making a second fully human heavy chain gene that encodes a second heavy chain that binds the second epitope of the second antigen of interest, expressing the first heavy chain and the second heavy chain in a cell that expresses a single fully human light chain derived from a human Vκ1-39 or a human Vκ3-20 gene segment to form a bispecific antigen-binding protein, and isolating the bispecific antigen-binding protein.

In one instance, the first antigen and the second antigen are not identical.

In one instance, the first antigen and the second antigen are identical, and the first epitope and the second epitope are not identical. In one instance, binding of the first heavy chain variable region to the first epitope does not block binding of the second heavy chain variable region to the second epitope.

In one instance, the human light chain when paired with the first heavy chain specifically binds the first epitope of the first antigen and when paired the second heavy chain specifically binds the second epitope of the second antigen.

In one instance, the first antigen is selected from a soluble antigen and a cell surface antigen (*e*.*g*., a tumor antigen), and the second antigen comprises a cell surface receptor. In a specific embodiment, the cell surface receptor is an immunoglobulin receptor. In a specific embodiment, the immunoglobulin receptor is an Fc receptor. In one instance, the first antigen and the second antigen are the same cell surface receptor, and binding of the first heavy chain to the first epitope does not block binding of the second heavy chain to the second epitope.

In one instance, the light chain variable domain of the light chain comprises 2 to 5 somatic mutations. In one instance, the light chain variable domain is a somatically mutated cognate light chain expressed in a B cell of the first or the second immunized mouse with either the first or the second heavy chain variable domain. In one instance, the light chain of the cell comprises a germline sequence.

In one instance, the first fully human heavy chain bears an amino acid modification that reduces its affinity to protein A, and the second fully human heavy chain does not comprise a modification that reduces its affinity to protein A.

Also referred to is a method of preparing a bispecific antibody that specifically binds to a first and a second antigen, wherein the method comprises (a) identifying a first nucleic acid sequence that encodes a first human heavy chain variable (V_{H}) domain that is specific for the first antigen; (b) identifying a second nucleic acid sequence that encodes a second human heavy chain variable (V_{H}) domain that is specific for the second antigen; (c) providing a third nucleic acid sequence that encodes a human light chain variable (V_{L}) region which, when paired with the V_{H} region of (a) specifically binds the first antigen, and when paired with the V_{H} region of (b) specifically binds to the second antigen; (d) culturing a host cell comprising the first, second, and third nucleic acid sequences to allow expression of the first and second human V_{H} regions and the human V_{L} region to form the bispecific antibody; and (d) recovering said bispecific antibody. In various instances, the first and second antigens are different from one another. In various instances the first and second nucleic acid sequences are isolated from an immunized mouse that expresses a human immunoglobulin V_{L} region from a rearranged immunoglobulin light chain sequence, wherein the rearranged immunoglobulin sequence is in the germline of the mouse.

In one instance, the human V_{L} region is derived from a rearranged human light chain sequence comprising a human Vκ1-39 gene segment or a human Vκ3-20 gene segment. In a specific instance, the rearranged human light chain sequence is a germline sequence *(i.e.,* does not comprise a somatic hypermutation within the V gene segment sequence).

In one instance, the third nucleic acid sequence is isolated from a mouse that expresses a human immunoglobulin V_{L} region from a rearranged immunoglobulin light chain sequence in the germline of the mouse. In one instance, the rearranged immunoglobulin light chain sequence comprises a human Vκ1-39 or human Vκ3-20 gene segment. In a specific instances, the rearranged immunoglobulin light chain sequence comprises a human Vκ1-39 gene segment. In one instances, the human immunoglobulin V_{L} region is expressed from a modified endogenous immunoglobulin light chain locus.

In one instance, the first and second antigens are present on one molecule. In one embodiment, the first and second antigens are present on different molecules. In various instances, the first or second nucleic acid sequence comprises a modification that reduces the affinity of the encoded heavy chain to protein A.

In one instance, the first or second nucleic acid sequences comprise a rearranged human heavy chain variable region sequence comprising a human heavy chain gene segment selected from V_{H}1-2, V_{H}1-3, V_{H}1-8, V_{H}1-18, V_{H}1-24, V_{H}1-46, V_{H}1-58, V_{H}1-69, V_{H}2-5, V_{H}2-26, V_{H}2-70, V_{H}3-7, V_{H}3-9, V_{H}3-11, V_{H}3-13, V_{H}3-15, V_{H}3-20, V_{H}3-21, V_{H}3-23, V_{H}3-30, V_{H}3-33, V_{H}3-43, V_{H}3-48, V_{H}3-53, V_{H}3-64, V_{H}3-72, V_{H}3-73, V_{H}4-31, V_{H}4-34, V_{H}4-39, V_{H}4-59, V_{H}5-51, and V_{H}6-1. In a specific instance, the heavy chain gene segment is V_{H}2-5, V_{H}3-23 or V_{H}3-30.

also referred to is a method of preparing a bispecific antibody that specifically binds to a first and a second antigen, wherein the method comprises (a) identifying a first nucleic acid sequence that encodes a first human heavy chain variable (V_{H}) domain that is specific for the first antigen; (b) identifying a second nucleic acid sequence that encodes a second human heavy chain variable (V_{H}) domain that is specific for the second antigen; (c) providing a third nucleic acid sequence that encodes a human light chain variable (V_{L}) region derived from a human Vκ1-39 or human Vκ3-20 gene segment which, when paired with the V_{H} region of (a) specifically binds the first antigen, and when paired with the V_{H} region of (b) specifically binds to the second antigen; (d) culturing a host cell comprising the first, second, and third nucleic acid sequences to allow expression of the first and second human V_{H} regions and the human V_{L} region to form the bispecific antibody; and (d) recovering said bispecific antibody. In various instances, the first and second antigens are different from one another. In variousinstances, the first and second nucleic acid sequences are isolated from an immunized mouse that expresses a human immunoglobulin V_{L} region from a rearranged immunoglobulin sequence that is derived from a human Vκ1-39 or human Vκ3-20 gene segment, wherein the rearranged human Vκ1-39 or Vκ3-30 gene segment is in the germline of the mouse.

In one instance, the third nucleic acid sequence is a germline sequence *(i.e.,* does not comprise a somatic hypermutation within the V gene segment sequence). In one instance, the third nucleic acid sequence is isolated from the mouse that expresses a human immunoglobulin V_{L} region derived from a human Vκ1-39 or human Vκ3-20 gene segment from a rearranged immunoglobulin light chain sequence in the germline of the mouse. In a specific instance, the third nucleic acid sequence comprises two to five somatic hypermutations in a complementary determining region (CDR) and/or a framework region (FWR). In one instance, the human immunoglobulin V_{L} region is expressed from a modified endogenous immunoglobulin light chain locus.

In one instance, the first and second antigens are present on one molecule. In one instance the first and second antigens are present on different molecules. In one instance, the first or second nucleic acid sequence comprises a modification that reduces the affinity of the encoded heavy chain to protein A.

In one instance, the first or second nucleic acid sequences comprise a rearranged human heavy chain variable region sequence comprising a human heavy chain gene segment selected from V_{H}1-2, V_{H}1-3, V_{H}1-8, V_{H}1-18, V_{H}1-24, V_{H}1-46, V_{H}1-58, V_{H}1-69, V_{H}2-5, V_{H}2-26, V_{H}2-70, V_{H}3-7, V_{H}3-9, V_{H}3-11, V_{H}3-13, V_{H}3-15, V_{H}3-20, V_{H}3-21, V_{H}3-23, V_{H}3-30, V_{H}3-33, V_{H}3-43, V_{H}3-48, V_{H}3-53, V_{H}3-64, V_{H}3-72, V_{H}3-73, V_{H}4-31, V_{H}4-34, V_{H}4-39, V_{H}4-59, V_{H}5-51, and V_{H}6-1. In a specific embodiment, the heavy chain gene segment is V_{H}2-5, V_{H}3-23 or V_{H}3-30.

Also referred to is a method for making a bispecific antibody, comprising exposing a mouse as described herein to an antigen of interest, allowing the mouse to mount an immune response to the antigen of interest, identifying a first human heavy chain variable region that binds a first epitope of the antigen of interest, identifying a second human heavy chain variable region that binds a second epitope of the antigen of interest, making a first fully human heavy chain gene that encodes the first heavy chain that binds the first epitope of the antigen of interest, making a second fully human heavy chain gene that encodes a second heavy chain that binds the second epitope of the antigen of interest, expressing the first heavy chain and the second heavy chain in a cell that expresses a single fully human light chain derived from a human Vκ1-39 or a human Vκ3-20 gene segment to form a bispecific antibody, and isolating the bispecific antigen-binding protein.

In one instance, the first epitope and the second epitope are not identical. In one embodiment, binding of the first heavy chain variable region to the first epitope does not block binding of the second heavy chain variable region to the second epitope. In one instance, the first and second heavy chains are capable of binding the first and second epitopes simultaneously.

In one instrance, the bispecific antibody binds the first and second epitopes simultaneously. In one instrance, the bispecific antibody binds the first epitope and second epitope independently.

In one instance, the binding response of the bispecific antibody to the antigen is about 2-fold higher than the binding response of the first heavy chain variable region to the antigen. In one instance, the binding response of the bispecific antibody to the antigen is about 2-fold higher than the binding response of the second heavy chain variable region to the antigen. In one instance, the binding response of the bispecific antibody to the antigen is about the same as, or about equal to, the binding response of the first heavy chain variable region and or the second heavy chain variable region to the antigen.

In one instance, the antigen is selected from a soluble antigen, a cell surface antigen (e.g., a tumor antigen) and a cell surface receptor. In a specific instance, the cell surface receptor is an immunoglobulin receptor. In a specific instance, the immunoglobulin receptor is an Fc receptor.

In one instance, the light chain variable domain of the light chain comprises 2 to 5 somatic mutations. In one instance, the light chain variable domain is a somatically mutated cognate light chain expressed in a B cell of the immunized mouse with either the first or the second heavy chain variable domain.

In one instance, the first fully human heavy chain bears an amino acid modification that reduces its affinity to protein A, and the second fully human heavy chain does not comprise a modification that reduces its affinity to protein A.

In various instances, methods for making bispecific antibodies are enhanced by employing a common light chain to pair with each heavy chain variable regions of the bispecific antibodies. In various instance, employing a common light chain as described herein reduces the number of inappropriate species of immunoglobulins lacking bispecificity as compared to employing original cognate light chains. In various instances, the heavy chain variable regions of the bispecific antibodies are identified from monospecific antibodies comprising a common light chain. In various instances, the heavy chain variable regions of the bispecific antibodies comprise human heavy chain variable gene segments that are rearranged *in vivo* within mouse B cells that have been previously engineered to express a limited human light chain repertoire, or a single human light chain, cognate with human heavy chains and, in response to exposure with an antigen of interest, generate a chimeric antibody repertoire containing a plurality of human heavy chain variable regions that are cognate with one or one of two possible human light chain variable regions, wherein the chimeric antibodies are specific for the antigen of interest.

Also referred to is a method of preparing a bispecific antibody, the bispecific antibody comprising 1) a first polypeptide and a second polypeptide, wherein the first and second polypeptides each include a multimerization domain (*e*.*g*., an immunoglobulin Fc domain) allowing the first and second polypeptides to form a dimer, and the multimerization domains promote stable interaction between first and second polypeptides, and wherein one of the multimerization domains bears an amino acid modification that reduces its affinity to protein A and the other multimerization domain lacks the modification, 2) a binding domain in each of the first and second polypeptide, each binding domain comprising a variable heavy chain and a variable light chain, wherein the variable light chain of the first polypeptide and the variable light chain of the second polypeptide have a common amino acid sequence, which common sequence has an amino acid sequence identity to an original light chain of each of the polypeptides of at least 80%, of at least 85%, preferably at least 90%, more preferably at least 95% and most preferably 100% sequence identity. In various instances, the variable light chain is derived from a human Vκ1-39 or a human Vκ3-20 gene segment. In various instances, the variable light chain is a rearranged human light chain sequence. In various instances, the variable light chain is isolated from a mouse as described herein.

In various instances, the method comprises the steps of (i) culturing a host cell comprising a nucleic acid encoding the first polypeptide, the second polypeptide, and the common light chain, wherein the nucleic acid is expressed; and (ii) recovering the bispecific antibody from the host cell culture; in one instance, the nucleic acid encoding the first polypeptide or the nucleic acid encoding the second polypeptide, bears an amino acid modification that reduces its affinity to protein A. In one instance, the nucleic acid encoding the first polypeptide, the second polypeptide, and the common light chain is present in a single vector or in separate vectors. In one instance, the host cell is used to make a bispecific antibody according to the preceding paragraph.

Also disclosed is a method of preparing a bispecific antibody, comprising (a) selecting a first nucleic acid encoding a first human heavy chain variable region isolated from a mouse as described herein; (b) selecting a second nucleic acid encoding a second human heavy chain variable region isolated from the same or separate mouse as described herein; (c) providing a third nucleic acid encoding a human light chain variable region isolated from a mouse as described herein or derived from a rearranged human light chain variable region as described herein; (c) introducing into a host cell the first, second and third nucleic acids and culturing the host cell so that expression of the first, second and third nucleic acid occurs; and (d) recovering the bispecific antibody formed from the cell culture.

In one instance, the first and second human heavy chain variable regions are somatically mutated. In a specific instance, the first and second human heavy chain variable regions are independently derived from a rearranged human V_{H} gene segment selected from 1-2, 1-3, 1-8, 1-18, 1-24, 1-46, 1-58, 1-69, 2-5, 2-26, 2-70, 3-7, 3-9, 3-11, 3-13, 3-15, 3-16, 3-20, 3-21, 3-23, 3-30, 3-33, 3-43, 3-48, 3-53, 3-64, 3-72, 3-73, 4-31, 4-34, 4-39, 4-59, 5-51, and a 6-1 human V_{H} gene segment. In one instance, the first and second human heavy chain variable regions are independently derived from a rearranged human V_{H} gene segment selected from 2-5, 3-30 and 3-23. In one instance, the first human heavy chain variable region is derived from a human V_{H}2-5 gene segment and the second human heavy chain variable region is derived from a human V_{H}3-30 gene segment. In one instance, the first human heavy chain variable region is derived from a human V_{H}3-30 gene segment and the second human heavy chain variable region is derived from a human V_{H}3-23 gene segment. In one instance, the first human heavy chain variable region is derived from a human V_{H}3-23 gene segment and the second human heavy chain variable region is derived from a human V_{H}3-30 gene segment.

In one instance, the first or second nucleic acid is modified prior to step (c), wherein the first or second nucleic acid is modified such that it has a reduced affinity to protein A.

In one instance, the third nucleic acid is isolated from a mouse as described herein. In one instance, the third nucleic acid comprises 2 to 5 somatic mutations. In one instance, the third nucleic acid encodes a human light chain variable region derived from a human Vκ1-39 gene segment. In one instance, the third nucleic acid encodes a human light chain variable region derived from a human Vκ3-20 gene segment.

In one instance, the third nucleic acid is derived from a rearranged human light chain variable region. In one instance, the rearranged human light chain variable region comprises a sequence derived from a human Vκ1-39 gene segment or a human Vκ3-20 gene segment. In one instance, the rearranged human light chain variable region comprises a germline human Vκ1-39 sequence (*i.e.,* does not comprise a somatic hypermutation within the V gene segment sequence). In one instance, the rearranged human light chain variable region comprises a germline human Vκ3-20 sequence.

Also disclosed is a method of preparing a bispecific antibody that incorporates a first human heavy chain comprising a variable domain derived from a modified mouse that lacks a rearranged human light chain sequence in its germline wherein the first human heavy chain is paired with a cognate human light chain that comprises a rearranged human light chain variable region derived from a human Vκ1-39 or a human Vκ3-20 gene segment. In various instances, a second human heavy chain with a different specificity from the first human heavy chain is identified from an immunized mouse as described herein. Nucleic acids encoding the two heavy chains and the common light chain are introduced into a host

cell as described in the preceding paragraphs so that expression of all three chains occurs and the bispecific antibody is recovered from the cell culture.

In one instance, the mouse is immunized with the same antigen used to generate the first human heavy chain variable domain. In one instancet, the mouse is immunized with a different antigen used to generate the first human heavy chain variable domain.

Also disclosed is a method of selecting human heavy chains that can pair with a single human light chain to make a bispecific antibody, including nucleic acids that encode the bispecific antibody and a host cell comprising the nucleic acids.

Also disclosed is a method of increasing the amount of a desired bispecific antibody in a cell culture over undesired products such as monospecific antibodies, wherein one of the heavy chains of the bispecific antibody is modified to reduce its affinity to protein A.

Also referred to is an isolated host cell, wherein the host cell comprises (a) a first nucleic acid sequence encoding a first human heavy chain variable region that binds a first antigen, wherein the first nucleic acid sequence is isolated from a mouse immunized with the first antigen that expresses a human immunoglobulin V_{L} region from a rearranged immunoglobulin light chain sequence in the germline of the mouse; (b) a second nucleic acid sequence encoding a second human heavy chain variable region that binds a second antigen, wherein the second nucleic acid sequence is isolated from a mouse immunized with the second antigen that expresses a human immunoglobulin V_{L} region from a rearranged immunoglobulin light chain sequence in the germline of the mouse; (c) a third nucleic acid sequence encoding a human light chain variable region which, when paired with the heavy chain variable region of (a) specifically binds the first antigen, and when paired with the heavy chain variable region of (b) specifically binds to the second antigen.

In various instances, the first and second antigens are different from one another. In various instanbces, the expression of the first, second and third nucleic acid sequences leads to the formation of a bispecific antibody that specifically binds to the first and second antigens.

In one instance, the human V_{L} region is derived from a rearranged human light chain sequence comprising a human Vκ1-39 gene segment or a human Vκ3-20 gene segment. In a specific instance, the rearranged human light chain sequence is a germline sequence *(i.e.,* does not comprise a somatic hypermutation within the variable domain). In one instance, the third nucleic acid sequence is isolated from a mouse that expresses a human immunoglobulin V_{L} region from a rearranged immunoglobulin light chain sequence, wherein the rearranged human light chain sequence is present in the germline of the mouse. In one instance, the rearranged immunoglobulin light chain sequence comprises a human Vκ1-39 gene segment or a human Vκ3-20 gene segment. In a specific instance, the human Vκ1-39 gene segment or human Vκ3-20 gene segment comprises at least one somatic hypermutation in a complementary determining region (CDR) or framework region (FWR). In a specific instance, the first, second and third nucleic acid sequences are isolated from a mouse that expresses a human immunoglobulin V_{L} region derived from a human Vκ1-39 or human Vκ3-20 gene segment from a rearranged immunoglobulin light chain sequence, wherein the rearranged immunoglobulin light chain sequence is present in the germline of the mouse.

In various embodiments, the mouse does not contain an endogenous light chain variable region gene segment that is capable of rearranging to form an immunoglobulin light chain.

In one embodiment, the human immunoglobulin V_{L} region is expressed from a modified endogenous immunoglobulin light chain locus. In one embodiment, the first and second antigens are present on one molecule. In one instance, the first and second antigens are present on different molecules. In one instance, the first or second nucleic acid sequence comprises a modification that reduces the affinity of the encoded heavy chain to protein A.

In one instance, the first or second nucleic acid sequences comprise a rearranged human heavy chain variable region sequence comprising a human heavy chain gene segment selected from V_{H}1-2, V_{H}1-3, V_{H}1-8, V_{H}1-18, V_{H}1-24, V_{H}1-46, V_{H}1-58, V_{H}1-69, V_{H}2-5, V_{H}2-26, V_{H}2-70, V_{H}3-7, V_{H}3-9, V_{H}3-11, V_{H}3-13, V_{H}3-15, V_{H}3-20, V_{H}3-21, V_{H}3-23, V_{H}3-30, V_{H}3-33, V_{H}3-43, V_{H}3-48, V_{H}3-53, V_{H}3-64, V_{H}3-72, V_{H}3-73, V_{H}4-31, V_{H}4-34, V_{H}4-39, V_{H}4-59, V_{H}5-51, and V_{H}6-1. In a specific instance, the heavy chain gene segment is V_{H}2-5, V_{H}3-23 or V_{H}3-30.

Also referred to is an antibody or a bispecific antibody comprising a human heavy chain variable domain made in accordance with the invention. In another instance, use of a mouse as described herein to make a fully human antibody or a fully human bispecific antibody is also described.

In one aspect, a genetically modified mouse, embryo, or cell described herein comprises a κ light chain locus that retains endogenous regulatory or control elements, e.g., a mouse κ intronic enhancer, a mouse κ 3' enhancer, or both an intronic enhancer and a 3' enhancer, wherein the regulatory or control elements facilitate somatic mutation and affinity maturation of an expressed sequence of the κ light chain locus.

Also referred to is a mouse that comprises a B cell population characterized by having immunoglobulin light chains derived from no more than one, or no more than two, rearranged or unrearranged immunoglobulin light chain V and J gene segments, wherein the mouse exhibits a κ:λ light chain ratio that is about the same as a mouse that comprises a wild type complement of immunoglobulin light chain V and J gene segments.

In one instance the immunoglobulin light chains are derived from no more than one, or no more than two, rearranged immunoglobulin light chain V and J gene segments.

In a specific instance, the light chains are derived from no more than one rearranged immunoglobulin light chain V and J gene segments. In one instance, the immunoglobulin light chains are generated from one of two unrearranged immunoglobulin V_{L} gene segments and one of 1, 2, 3, 4, or 5 immunoglobulin J_{L} gene segments. In one instance, the immunoglobulin light chains are generated from one of two unrearranged immunoglobulin V_{L} gene segments and one immunoglobulin J_{L} gene segment.

Also referred to is a mouse as described herein that expresses an immunoglobulin light chain derived from no more than one, or no more than two, human Vκ/Jκ sequences, wherein the mouse comprises a replacement of all or substantially all endogenous mouse heavy chain variable region gene segments with one or more human heavy chain variable region gene segments, and the mouse exhibits a ratio of (a) CD19⁺ B cells that express an immunoglobulin having a λ light chain, to (b) CD19⁺ B cells that express an immunoglobulin having a κ light chain, of about 1 to about 20.

In one instance, the mouse expresses a single κ light chain derived from a human Vκ1-39Jκ5 sequence, and the ratio of CD19⁺ B cells that express an immunoglobulin having a λ light chain to CD19⁺ B cells that express an immunoglobulin having a κ light chain is about 1 to about 20; in one instance, the ratio is about 1 to at least about 66; in a specific embodiment, the ratio is about 1 to 66.

Also referred to is a mouse that expresses a single κ light chain derived from a human Vκ3-20Jκ5 sequence, and the ratio of CD19⁺ B cells that express an immunoglobulin having a λ light chain to CD19⁺ B cells that express an immunoglobulin having a κ light chain is about 1 to about 20; in one instance, the ratio is about 1 to about 21. In specific embodiments, the ratio is 1 to 20, or 1 to 21.

Also referred to is a mouse that expresses an immunoglobulin light chain whose sequence is identical to that achieved by rearrangement of one of two human Vκ gene segments with 1, 2, 3, 4, or 5 human Jκ gene segments.

In some embodiments, a mouse expresses an immunoglobulin light chain generated from a rearrangement of two human Vκ gene segments and 5 human Jκ gene segments, wherein the mouse comprises a replacement of all or substantially all endogenous immunoglobulin V_{H} gene segments with one or more human immunoglobulin V_{H}, one or more D_{H}, and one or more J_{H} gene segments, and the mouse exhibits a ratio of (a) B cells in the bone marrow that express an immunoglobulin having a λ light chain, to (b) B cells in the bone marrow that express an immunoglobulin having a κ light chain, of about 1 to about 15. In some embodiments, the rearrangement includes a human Vκ1-39 gene segment. In some embodiments, the rearrangement includes a human Vκ3-20 gene segment. In some embodiments, the replacement of the endogenous immunoglobulin V_{H} gene segments is at an endogenous immunoglobulin V_{H} locus. In some embodiments, the two human Vκ gene segments is at an endogenous immunoglobulin Vκ locus, and, in some embodiments, the two human Vκ gene segments replace all or substantially all mouse immunoglobulin Vκ gene segments. In some embodiments, the two human Vκ gene segments are at an endogenous immunoglobulin Vκ locus, and, in some embodiments, the two human Vκ gene segments replace all or substantially all mouse immunoglobulin Vκ and Jκ gene segments. The two human Vκ gene segments are operably linked to 5 human Jκ gene segments.

In some embodiments, a mouse made by a method of the present invention expresses a light chain generated through a rearrangement of a human Vκ1-39 gene segment or a human Vκ3-20 gene segment and 5 human Jκ gene segments, and the ratio of immature B cells in the bone marrow that express an immunoglobulin having a λ light chain to immature B cells that express an immunoglobulin having a κ light chain is about 1 to about 13.

In some embodiments, a mouse made by a method of the present invention expresses a light chain generated through a rearrangement of a human Vκ1-39 gene segment or a human Vκ3-20 gene segment and 5 human Jκ gene segments, and the ratio of mature B cells in the bone marrow that express an immunoglobulin having a λ light chain to immature B cells that express an immunoglobulin having a κ light chain is about 1 to about 7.

In some embodiments, a mouse made by a method of the present invention expresses a light chain generated through a rearrangement of a human Vκ1-39 gene segment or a human Vκ3-20 gene segment and 5 human Jκ gene segments, and has a pro B cell population in the bone marrow within in the range of about 2.5×10⁴ to about 1.5×10⁵ cells, inclusive, for example about 2.5×10⁴, 3.0×10⁴, 3.5×10⁴, 4.0×10⁴, 4.5×10⁴, 5.0×10⁴, 5.5×10⁴, 6.0×10⁴, 6.5×10⁴, 7.0×10⁴, 7.5×10⁴, 8.0×10⁴, 8.5×10⁴, 9.0×10⁴, 9.5×10⁴, 1.0×10⁵, or 1.5×10⁵ cells; in some embodiments, a mouse made by a method of the present invention comprises a pro B cell population in the bone marrow of about 2.88×10⁴ cells; in some embodiments, a mouse made by a method of the present invention comprises a pro B cell population in the bone marrow of about 6.42×10⁴ cells; in some embodiments, a mouse of the present invention comprises a pro B cell population in the bone marrow of about 9.16×10⁴ cells; in some embodiments, a mouse made by a method of the present invention comprises a pro B cell population in the bone marrow of about 1.19×10⁵ cells. Exemplary pro B cells in the bone marrow of genetically modified mice as described herein are characterized by expression of CD19, CD43, c-kit and/or a combination thereof (e.g., CD19⁺, CD43⁺, c-kit⁺).

In some embodiments, a mouse made by a method of the present invention expresses a light chain generated through a rearrangement of a human Vκ1-39 gene segment or a human Vκ3-20 gene segment and 5 human Jκ gene segments, and has a pre B cell population in the bone marrow within in the range of about 1×10⁶ to about 2×10⁶ cells, inclusive, for example, about 1.0×10⁶, 1.1×10⁶, 1.2×10⁶, 1.3×10⁶, 1.4×10⁶, 1.5×10⁶, 1.6×10⁶, 1.7×10⁶, 1.8×10⁶, 1.9×10⁶, or 2.0×10⁶ cells; in some embodiments, a mouse made by a method of the present invention comprises a pre B cell population in the bone marrow of about 1.25×10⁶ cells; in some embodiments, a mouse made by a method of the present invention comprises a pre B cell population in the bone marrow of about 1.46×10⁶ cells; in some embodiments, a mouse made by a method of the present invention comprises a pre B cell population in the bone marrow of about 1.64×10⁶ cells; in some embodiments, a mouse made by a method of the present invention comprises a pre B cell population in the bone marrow of about 2.03×10⁶ cells. Exemplary pre B cells in the bone marrow of genetically modified mice as described herein are characterized by expression of CD19, CD43, c-kit and/or a combination thereof (e.g., CD19⁺, CD43⁻, c-kit).

In some embodiments, a mouse made by a method of the present invention expresses a light chain generated through a rearrangement of a human Vκ1-39 gene segment or a human Vκ3-20 gene segment and 5 human Jκ gene segments, and has an immature B cell population in the bone marrow within the range of about 5×10⁵ to about 7×10⁵ cells, inclusive, for example, about 5.0x105, 5.1x105, 5.2×10⁵, 5.3×10⁵, 5.4×10⁵, 5.5×10⁵, 5.6×10⁵, 5.7×10⁵, 5.8×10⁵, 5.9×10⁵, 6.0×10⁵, 6.1×10⁵, 6.2×10⁵, 6.3×10⁵, 6.4×10⁵, 6.5×10⁵, 6.6×10⁵, 6.7×10⁵, 6.8×10⁵, 6.9×10⁵, or 7.0×10⁵ cells; in some embodiments, a mouse employed in the present invention comprises an immature B cell population in the bone marrow of about 5.33×10⁵ cells; in some embodiments, a mouse employed in the present invention comprises an immature B cell population in the bone marrow of about 5.80×10⁵ cells; in some embodiments, a mouse employed the present invention comprises an immature B cell population in the bone marrow of about 5.92×10⁵ cells; in some embodiments, the mouse comprises an immature B cell population in the bone marrow of about 6.67×10⁵ cells. Exemplary immature B cells in the bone marrow of genetically modified mice as described herein are characterized by expression of IgM, B220 and/or a combination thereof (e.g., IgM⁺, B220^{int}).

In some embodiments, a mouse made by a method of the present invention expresses a light chain generated through a rearrangement of a human Vκ1-39 gene segment or a human Vκ3-20 gene segment and one of 5 human Jκ gene segments, and has a mature B cell population in the bone marrow within the range of about 3×10⁴ to about 1.5×10⁵ cells, inclusive, for example about 3.0×10⁴, 3.5×10⁴, 4.0×10⁴, 4.5×10⁴, 5.0×10⁴, 5.5×10⁴, 6.0×10⁴, 6.5×10⁴, 7.0×10⁴, 7.5×10⁴, 8.0×10⁴, 8.5×10⁴, 9.0×10⁴, 9.5×10⁴, 1.0×10⁵, or 1.5×10⁵ cells; in some embodiments, a mouse made by a method of the present invention comprises a mature B cell population in the bone marrow of about 3.11×10⁴ cells; in some embodiments, a mouse made by a method of the present invention comprise a mature B cell population in the bone marrow of about 1.09×10⁵ cells; in some embodiments, a mouse made by a method of the present invention comprises a mature B cell population in the bone marrow of about 1.16×10⁵ cells; in some embodiments, a mouse made by a method of the present invention comprises a mature B cell population in the bone marrow of about 1.44×10⁵ cells. Exemplary mature B cells in the bone marrow of genetically modified mice as described herein are characterized by expression of IgM, B220 and/or a combination thereof (e.g., IgM⁺, B220^{hi}).

In some embodiments, a mouse made by a method of the present invention expresses a light chain generated through a rearrangement of a human Vκ1-39 gene segment or a human Vκ3-20 gene segment and 5 human Jκ gene segments, and has a total B cell population in the bone marrow within the range of about 1×10⁶ to about 3×10⁶ cells, inclusive, for example about 1.0×10⁶, 1.1×10⁶, 1.2×10⁶, 1.3×10⁶, 1.4×10⁶, 1.5×10⁶, 1.6×10⁶, 1.7×10⁶, 1.8×10⁶, 1.9×10⁶, 2.0×10⁶, 2.1×10⁶, 2.2×10⁶, 2.3×10⁶, 2.4×10⁶, 2.5×10⁶, 2.6×10⁶, 2.7×10⁶, 2.8×10⁶, 2.9×10⁶ or 2.0×10⁶ cells; in some embodiments, a mouse made by a method of the present invention comprises a total B cell population in the bone marrow of about 1.59×10⁶ cells; in some embodiments, a mouse made by a method of the present invention comprises a total B cell population in the bone marrow of about 1.75×10⁶ cells; in some embodiments, a mouse made by a method of the present invention comprises a total B cell population in the bone marrow of about 2.13×10⁶ cells; in some embodiments, a mouse made by a method of the present invention comprises a total B cell population in the bone marrow of about 2.55×10⁶ cells. An exemplary total B cells in the bone marrow of genetically modified mice as described herein are characterized by expression CD19, CD20 and/or a combination thereof (*e*.*g*., CD19⁺).

Also referred to is a genetically modified mouse that expresses a single rearranged κ light chain, wherein the mouse comprises a functional λ light chain locus, and wherein the mouse expresses a B cell population that comprises Igκ⁺ cells that express a κ light chain derived from the same single rearranged κ light chain. In one instance, the percent of Igκ⁺Igλ⁺ B cells in the mouse is about the same as in a wild type mouse. In a specific instance, the percent of Igκ⁺Igλ⁺ B cells in the mouse is about 2 to about 6 percent. In a specific instance, the percent of Igκ⁺Igλ⁺ B cells in a mouse wherein the single rearranged κ light chain is derived from a Vκ1-39Jκ5 sequence is about 2 to about 3; in a specific embodiment, the percent is about 2.6. In a specific instance, the percent of Igκ⁺Igλ⁺ B cells in a mouse wherein the single rearranged κ light chain is derived from a Vκ3-20Jκ1 sequence is about 4 to about 8; in a specific instance, the percent is about 6.

In some embodiments, the genetically modified mouse made by a method of the present invention expresses an immunoglobulin light chain comprising a rearranged human immunoglobulin Vκ/Jκ sequence, wherein the mouse comprises a functional immunoglobulin λ light chain locus, and wherein the mouse comprises a splenic B cell population that comprises a ratio of Igλ⁺ B cells to Igκ⁺ B cells that is about 1 to about 8; in some embodiments, about 1 to about 5. The rearranged human immunoglobulin Vκ/Jκ sequence is generated through a rearrangement of one of two human immunoglobulin Vκ gene segments and one of 5 human immunoglobulin Jκ gene segments. In some embodiments, the rearranged human immunoglobulin Vκ/Jκ sequence is generated through a rearrangement of a human immunoglobulin Vκ1-39 gene segment and a human immunoglobulin Jκ gene segment selected from Jκ1, Jκ2, Jκ3, Jκ4, Jκ5, and a combination thereof. In some embodiments, the rearranged human immunoglobulin Vκ/Jκ sequence is generated through a rearrangement of a human immunoglobulin Vκ3-20 gene segment and a human immunoglobulin Jκ gene segment selected from Jκ1, Jκ2, Jκ3, Jκ4, Jκ5, and a combination thereof.

In some embodiments, a mouse made by a method of the present invention comprises a CD19⁺ splenic B cell population within the range of about 2×10⁶ to about 7×10⁶ cells, inclusive, for example about 2.0×10⁶, 2.5×10⁶, 3.0×10⁶, 3.5×10⁶, 4.0×10⁶, 4.5×10⁶, 5.0×10⁶, 5.5×10⁶, 6.0×10⁶, 6.5×10⁶, or 7.0×10⁶ cells; in some embodiments, a mouse made by a method of the present invention comprises a CD19⁺ splenic B cell population of about 2.74×10⁶ cells; some embodiments, a mouse made by a method of the present invention comprises a CD19⁺ splenic B cell population of about 4.30×10⁶ cells; in some embodiments, a mouse made by a method of the present invention comprises a CD19⁺ splenic B cell population of about 5.53×10⁶ cells; in some embodiments, a mouse made by a method of the present invention comprises a CD19⁺ splenic B cell population of about 6.18×10⁶ cells.

In some embodiments, a mouse made by a method of the present invention comprises a CD19⁺, IgD^{hi}, IgM^{lo} splenic B cell population within the range of about 1×10⁶ to about 4×10⁶ cells, inclusive, for example about 1.0×10⁶, 1.5×10⁶, 2.0×10⁶, 2.5×10⁶, 3.0×10⁶,

3.5×10⁶, 4.0×10⁶ cells; in some embodiments, a mouse made by a method of the present invention comprises a CD19⁺, IgD^{hi}, IgM^{lo} splenic B cell population of about 1.30×10⁶; in some embodiments, a mouse made by a method of the present invention comprises a CD19⁺, IgD^{hi}, IgM^{lo} splenic B cell population of about 2.13×10⁶ cells; in some embodiments, a mouse made by a method of the present invention comprises CD19⁺, IgD^{hi}, IgM^{lo} splenic B cell population of about 3.15×10⁶ cells; in some embodiments, a mouse made by a method of the present invention comprises a CD19⁺, IgD^{hi}, IgM^{lo} splenic B cell population of about 3.93×10⁶ cells.

In some embodiment, a mouse made by a method of the present invention comprises a CD19⁺, IgD^{lo}, IgM^{hi} splenic B cell population within the range of about 9×10⁵ to about 2×10⁶ cells, inclusive, for example about 9.0×10⁵, 9.25×10⁵, 9.5×10⁵, 9.75×10⁵, 1.0×10⁶, 1.25×10⁶, 1.50×10⁶, 1.75×10⁶, 2.0×10⁶ cells; in some embodiments, a mouse made by a method of the present invention comprises a CD19⁺, IgD^{lo}, IgM^{hi} splenic B cell population of about 9.52×10⁵; in some embodiments, a mouse made by a method of the present invention comprises a CD19⁺, IgD^{lo}, IgM^{hi} splenic B cell population of about 1.23×10⁶ cells; in some embodiments, a mouse made by a method o the present invention comprises CD19⁺, IgD^{lo}, IgM^{hi} splenic B cell population of about 1.40×10⁶ cells; in some embodiments, a mouse made by a method of the present invention comprises a CD19⁺, IgD^{lo}, IgM^{hi} splenic B cell population of about 1.42×10⁶ cells.

In some embodiments, the genetically modified mouse made by a method of the present invention comprises an immunoglobulin κ light chain locus that comprises two unrearranged human immunoglobulin Vκ gene segments and 5 unrearranged human Jκ gene segments, and wherein the mouse comprises a peripheral splenic B cell population comprising transitional (*e*.*g*., T1, T2 and T3) B cell populations that are about the same as a mouse that comprises a wild type complement of immunoglobulin κ light chain V and J gene segments. Exemplary transitional B cell populations (*e*.*g*., T1, T2 and T3) in the spleen of a genetically modified mouse as described herein are characterized by expression of IgM, CD23, CD93, B220 and/or a combination thereof.

In some embodiments, a mouse made by a method of the present invention comprises a T1 B cell population in the spleen (e.g., CD93⁺, B220⁺, IgM^{hi}, CD23⁻) within the range of about 2×10⁶ to about 7×10⁶ cells, inclusive, for example about 2.0×10⁶, 2.5×10⁶, 3.0×10⁶, 3.5×10⁶, 4.0×10⁶, 4.5×10⁶, 5.0×10⁶, 5.5×10⁶, 6.0×10⁶, 6.5×10⁶, or 7.0×10⁶ cells; in some embodiments, a mouse made by a method of the present invention comprises a T1 B cell population in the spleen of about 2.16×10⁶ cells; in some embodiments, a mouse made by a method of the present invention comprises a T1 B cell population in the spleen of about 3.63×10⁶ cells; in some embodiments, a mouse made by a method of the present invention comprises a T1 B cell population in the spleen of about 3.91×10⁶; in some embodiments, a mouse made by a method of the present invention comprises a T1 B cell population in the spleen of about 6.83×10⁶ cells.

In some embodiments, a mouse made by a method of the present invention comprises a T2 B cell population in the spleen (e.g., CD93⁺, B220⁺, IgM^{hi}, CD23⁺) within the range of about 1×10⁶ to about 7×10⁶ cells, inclusive, for example about 1.0×10⁶, 1.5×10⁶, 2.0×10⁶, 2.5×10⁶, 3.0×10⁶, 3.5×10⁶, 4.0×10⁶, 4.5×10⁶, 5.0×10⁶, 5.5×10⁶, 6.0×10⁶, 6.5×10⁶, or 7.0×10⁶ cells; in some embodiments, a mouse made by a method of the present invention mouse comprises a T2 B cell population in the spleen of about 1.30×10⁶ cells; in some embodiments, a mouse made by a method of the present invention comprises a T2 B cell population in the spleen of about 2.46×10⁶ cells; in some embodiments, a mouse made by a method of the present invention comprises a T2 B cell population in the spleen of about 3.24×10⁶; in some embodiments, a made by a method mouse of the present invention comprises a T2 B cell population in the spleen of about 6.52×10⁶ cells.

In some embodiments, a mouse made by a method of the present invention comprises a T3 B cell population in the spleen (e.g., CD93⁺, B220⁺, IgM^{lo}, CD23⁺) within the range of about 1×10⁶ to about 4×10⁶ cells, inclusive, for example about 1.0×10⁶, 1.5×10⁶, 2.0×10⁶, 2.5×10⁶, 3.0×10⁶, 3.5×10⁶, or 4.0×10⁶ cells; in some embodiments, a mouse made by a method of the present invention comprises a T3 B cell population in the spleen of about 1.08×10⁶ cells; in some embodiments, a mouse made by a method of the present invention comprises a T3 B cell population in the spleen of about 1.35×10⁶ cells; in some embodiments, a mouse made by a method of the present invention comprises a T3 B cell population in the spleen of about 3.37×10⁶; in some embodiments, a mouse made by a method of the present invention comprises a T1 B cell population in the spleen of about 3.63×10⁶ cells.

In some embodiments, a genetically modified mouse made by a method of the present invention is one wherein the mouse comprises an immunoglobulin κ light chain locus that comprises two unrearranged human immunoglobulin Vκ gene segments and 5 unrearranged human immunoglobulin Jκ gene segments, and wherein the mouse comprises a peripheral splenic B cell population comprising marginal zone and marginal zone precursor B cell populations that are about the same as a mouse that comprises a wild type complement of immunoglobulin Vκ and Jκ gene segments. Exemplary marginal zone B cell populations in the spleen of a genetically modified mouse as described herein are characterized by expression of IgM, CD21/35, CD23, CD93, B220 and/or a combination thereof.

In some embodiments, a mouse made by a of the present invention comprises marginal zone B cell population in the spleen (e.g., CD93⁻, B220⁺, IgM^{hi}, CD21/35^{hi}, CD23⁻) within the range of about 1×10⁶ to about 3×10⁶ cells, inclusive, for example, about 1.0×10⁶, 1.5×10⁶, 2.0×10⁶, 2.5×10⁶, or 3.0×10⁶ cells; in some embodiments, a mouse made by a method of the present invention comprises a marginal zone B cell population in the spleen of about 1.47×10⁶ cells; in some embodiments, a mouse made by a method of the present invention comprises a marginal zone B cell population in the spleen of about 1.49×10⁶ cells; in some embodiments, a mouse made by a method of the present invention comprises a marginal zone B cell population in the spleen of about 2.26×10⁶ cells; in some embodiments, a mouse made by a of the present invention comprises a marginal zone B cell population in the spleen of about 2.33×10⁶ cells.

In some embodiments, a genetically modified mouse made by a method of the present invention comprises an immunoglobulin κ light chain locus that comprises two unrearranged human immunoglobulin Vκ gene segments and 5 unrearranged human immunoglobulin Jκ gene segments, and wherein the mouse comprises a peripheral splenic B cell population comprising follicular (*e*.*g*., FO-I and FO-II) B cell population(s) that are about the same as a mouse that comprises a wild type complement of immunoglobulin Vκ and Jκ gene segments. Exemplary follicular B cell populations (*e*.*g*., FO-I and FO-II) in the spleen of a genetically modified mouse as described herein are characterized by expression of IgM, IgD, CD21/35, CD93, B220 and/or a combination thereof.

In some embodiments, a mouse made by a method of the present invention comprises a follicular type 1 B cell population in the spleen (e.g., CD93⁻, B220⁺, CD21/35^{int}, IgM^{lo}, IgD^{hi}) within the range of about 3×10⁶ to about 1.5×10⁷ cells, inclusive, for example about 3.0×10⁶, 3.5×10⁶, 4.0×10⁶, 4.5×10⁶, 5.0×10⁶, 5.5×10⁶, 6.0×10⁶, 6.5×10⁶, 7.0×10⁶, 7.5×10⁶, 8.0×10⁶, 8.5×10⁶, 9.0×10⁶, 9.5×10⁶, 1.0×10⁷, or 1.5×10⁷ cells; in some embodiments, a mouse made by a method of the present invention comprises a follicular type 1 B cell population in the spleen of about 3.57×10⁶ cells; in some embodiments, a mouse made by a method of the present invention comprises a follicular type 1 B cell population in the spleen of about 6.31×10⁶ cells; in some embodiments, a mouse made by a method of the present invention comprises a follicular type 1 B cell population in the spleen of about 9.42×10⁶ cells; in some embodiments, a mouse made by a method of the present invention comprise a follicular type 1 B cell population in the spleen of about 1.14×10⁷ cells.

In some embodiments, a mouse made by a method of the present invention comprises a follicular type 2 B cell population in the spleen (e.g., CD93⁻, B220⁺, CD21/35^{int}, IgM^{int}, IgD^{hi}) within the range of about 1×10⁶ to about 2×10⁶ cells, inclusive, for example, 1.0×10⁶, 1.25×10⁶, 1.5×10⁶, 1.75×10⁶, or 2.0×10⁶ cells; in some embodiments, a mouse made by a method of the present invention comprises a follicular type 2 B cell population in the spleen of about 1.14×10⁶ cells; in some embodiments, a mouse made by a method of the present invention comprises a follicular type 2 B cell population in the spleen of about 1.45×10⁶ cells; in some embodiments, a mouse made by a method of the present invention comprises a follicular type 2 B cell population in the spleen of about 1.80×10⁶; in some embodiments, a mouse made by a method of the present invention comprise a follicular type 2 B cell population in the spleen of about 2.06×10⁶ cells.

In one aspect, the genetically modified mouse made by a method expresses a single rearranged κ light chain derived from a human Vκ and Jκ gene segment, wherein the mouse expresses a B cell population that comprises a single κ light chain derived from the single rearranged κ light chain sequence, wherein the genetically modified mouse has not been rendered resistant to somatic hypermutations. In one em, at least 90% of the κ light chains expressed on a B cell of the mouse exhibit from at least one to about five somatic hypermutations.

Also referred to is a genetically modified mouse that is modified to express a single κ light chain derived from no more than one, or no more than two, rearranged κ light chain sequences, wherein the mouse exhibits a κ light chain usage that is about two-fold or more, at least about three-fold or more, or at least about four-fold or more greater than the κ light chain usage exhibited by a wild type mouse, or greater than the κ light chain usage exhibited by a mouse of the same strain that comprises a wild type repertoire of κ light chain gene segments. In a specific instance, the mouse expresses the single κ light chain from no more than one rearranged κ light chain sequence. In a more specific instance, the rearranged κ light chain sequence is selected from a Vκ1-39Jκ5 and Vκ3-20Jκ1 sequence. In one instance, the rearranged κ light chain sequence is a Vκ1-39Jκ5 sequence. In one instance, the rearranged κ light chain sequence is a Vκ3-20Jκ1 sequence.

Also referred to is a genetically modified mouse that expresses a single κ light chain derived from no more than one, or no more than two, rearranged κ light chain sequences, wherein the mouse exhibits a κ light chain usage that is about 100-fold or more, at least about 200-fold or more, at least about 300-fold or more, at least about 400-fold or more, at least about 500-fold or more, at least about 600-fold or more, at least about 700-fold or more, at least about 800-fold or more, at least about 900-fold or more, at least about 1000-fold or more greater than the same κ light chain usage exhibited by a mouse bearing a complete or substantially complete human κ light chain locus. In a specific instance, the mouse bearing a complete or substantially complete human κ light chain locus lacks a functional unrearranged mouse κ light chain sequence. In a specific instance, the mouse expresses the single κ light chain from no more than one rearranged κ light chain sequence. In one instance, the mouse comprises one copy of a rearranged κ light chain sequence (*e*.*g*., a heterozygote). In one instance, the mouse comprises two copies of a rearranged κ light chain sequence (*e*.*g*., a homozygote). In a more specific embodiment, the rearranged κ light chain sequence is selected from a Vκ1-39Jκ5 and Vκ3-20Jκ1 sequence. In one embodiment, the rearranged κ light chain sequence is a Vκ1-39Jκ5 sequence. In one embodiment, the rearranged κ light chain sequence is a Vκ3-20Jκ1 sequence.

Also referred to is a genetically modified mouse that expresses a single light chain derived from no more than one, or no more than two, rearranged light chain sequences, wherein the light chain in the genetically modified mouse exhibits a level of expression that is at least 10-fold to about 1,000-fold, 100-fold to about 1,000-fold, 200-fold to about 1,000-fold, 300-fold to about 1,000-fold, 400-fold to about 1,000-fold, 500-fold to about 1,000-fold, 600-fold to about 1,000-fold, 700-fold to about 1,000-fold, 800-fold to about 1,000-fold, or 900-fold to about 1,000-fold higher than expression of the same rearranged light chain exhibited by a mouse bearing a complete or substantially complete light chain locus. In one instance, the light chain comprises a human sequence. In a specific instance, the human sequence is a κ sequence. In one instance, the human sequence is a λ sequence. In one instance, the light chain is a fully human light chain.

In one embodiment, the level of expression is characterized by quantitating mRNA of transcribed light chain sequence, and comparing it to transcribed light chain sequence of a mouse bearing a complete or substantially complete light chain locus.

Also referred to is a genetically modified mouse that expresses a single κ light chain derived from no more than one, or no more than two, rearranged κ light chain sequences, wherein the mouse, upon immunization with antigen, exhibits a serum titer that is comparable to a wild type mouse immunized with the same antigen. In a specific instance, the mouse expresses a single κ light chain from no more than one rearranged κ light chain sequence. In one instance, the serum titer is characterized as total immunoglobulin. In a specific instance, the serum titer is characterized as IgM specific titer. In a specific instance, the serum titer is characterized as IgG specific titer. In a more specific embodiment, the rearranged κ light chain sequence is selected from a Vκ1-39Jκ5 and Vκ3-20Jκ1 sequence. In one instance, the rearranged κ light chain sequence is a Vκ1-39Jκ5 sequence. In one instance, the rearranged κ light chain sequence is a Vκ3-20Jκ1 sequence.

Also referred to is a genetically modified mouse that expresses a population of antigen-specific antibodies, wherein all of the immunoglobulin light chains of the population of antigen-specific antibodies comprise a human light chain variable (V_{L}) region derived from the same single human V_{L} gene segment and the immunoglobulin heavy chains comprise a human heavy chain variable (V_{H}) region derived from one of a plurality of human V_{H} gene segments.

In various embodiments, the human V_{H} gene segments are selected from V_{H}1-2, V_{H}1-3, V_{H}1-8, V_{H}1-18, V_{H}1-24, V_{H}1-46, V_{H}1-58, V_{H}1-69, V_{H}2-5, V_{H}2-26, V_{H}2-70, V_{H}3-7, V_{H}3-9, V_{H}3-11, V_{H}3-13, V_{H}3-15, V_{H}3-20, V_{H}3-21, V_{H}3-23, V_{H}3-30, V_{H}3-33, V_{H}3-43, V_{H}3-48, V_{H}3-53, V_{H}3-64, V_{H}3-72, V_{H}3-73, V_{H}4-31, V_{H}4-34, V_{H}4-39, V_{H}4-59, V_{H}5-51, and V_{H}6-1.

In various embodiments, same single human V_{L} gene segment is selected from a human Vκ1-39 gene segment and a human Vκ3-20 gene segment. In various embodiments, all of the immunoglobulin light chains comprise a human light chain J (J_{L}) gene segment selected from a Jκ and a Jλ gene segment. In a specific embodiment, the human J_{L} gene segment is selected from a human Jκ1 and a Jκ5 gene segment. In various embodiments, the mouse lacks a sequence selected from a mouse immunoglobulin V_{L} gene segment, a mouse immunoglobulin J_{L} gene segment, and a combination thereof. In various embodiments, the human V_{L} region is operably linked to a human, mouse, or rat immunoglobulin light chain constant (C_{L}) region. In a specific embodiment, the human V_{L} region is operably linked to a mouse Cκ region. In a specific embodiment, the human V_{L} region is operably linked to a rat Cκ region.

In various embodiments, the human V_{L} region is expressed from an endogenous immunoglobulin light chain locus. In various embodiments, the human V_{H} region is operably linked to a human, mouse, or rat immunoglobulin heavy chain constant (C_{H}) region. In various embodiments the (C_{H}) region comprises a human sequence selected from a C_{H}1, a hinge, a C_{H}2, a C_{H}3, a C_{H}4, and/or a combination thereof. In various embodiments, the human V_{H} region is expressed from an endogenous immunoglobulin heavy chain locus.

Also referred to is a genetically modified mouse that expresses a plurality of immunoglobulin heavy chains associated with a single light chain. In one instance, the heavy chain comprises a human sequence. In various instances, the human sequence is selected from a variable sequence, a C_{H}1, a hinge, a C_{H}2, a C_{H}3, and a combination thereof. In one instance, the single light chain comprises a human sequence. In various instances, the human sequence is selected from a variable sequence, a constant sequence, and a combination thereof. In one instance, the mouse comprises a disabled endogenous immunoglobulin locus and expresses the heavy chain and/or the light chain from a transgene or extrachromosomal episome. In one instance, the mouse comprises a replacement at an endogenous mouse locus of some or all endogenous mouse heavy chain gene segments *(i.e.,* V, D, J), and/or some or all endogenous mouse heavy chain constant sequences (*e*.*g*., C_{H}1, hinge, C_{H}2, C_{H}3, or a combination thereof), and/or some or all endogenous mouse light chain sequences (*e*.*g*., V, J, constant, or a combination thereof), with one or more human immunoglobulin sequences.

In one aspect, a mouse suitable for making antibodies that have the same light chain is made by a method of the present invention , wherein all or substantially all antibodies made in the mouse are expressed with the same light chain. In one embodiment, the light chain is expressed from an endogenous light chain locus.

Also referred to is a method for making a light chain for a human antibody, comprising obtaining from a mouse as described herein a light chain sequence and a heavy chain sequence, and employing the light chain sequence and the heavy chain sequence in making a human antibody. In one instance, the human antibody is a bispecific antibody.

Also referred to is a method for identifying a human heavy chain variable domain that is capable of binding an antigen of interest with an engineered light chain as described herein, wherein the method comprises providing a heavy chain variable domain derived from a first antibody that is capable of binding the antigen, repairing the heavy chain variable domain with a germline light chain sequence and transfecting a cell so that each are expressed to form a second antibody, exposing the second antibody to the antigen, and measuring binding of the second antibody to the antigen.

In one instance, the light chain of the first antibody comprises a human Vκ1-39 sequence. In one instance, the light chain of the first antibody comprises a human Vκ3-20 sequence. In one instance, the germline light chain sequence comprises a human Vκ1-39 or Vκ3-20 sequence. In various instances, binding of the second antibody to the antigen is determined by comparison of binding of the first antibody to the antigen.

Any of the embodiments and aspects described herein can be used in conjunction with one another, unless otherwise indicated or apparent from the context. Other embodiments will become apparent to those skilled in the art from a review of the ensuing description.

### BRIEF DESCRIPTION OF FIGURES

**FIG. 1** illustrates a targeting strategy for replacing endogenous mouse immunoglobulin light chain variable region gene segments with a human Vκ1-39Jκ5 gene region.

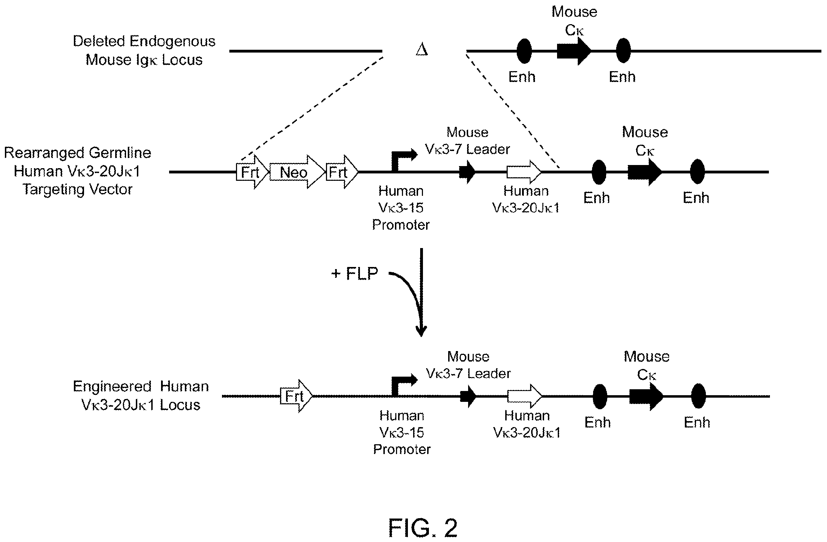

**FIG. 2** illustrates a targeting strategy for replacing endogenous mouse immunoglobulin light chain variable region gene segments with a human Vκ3-20Jκ1 gene region.

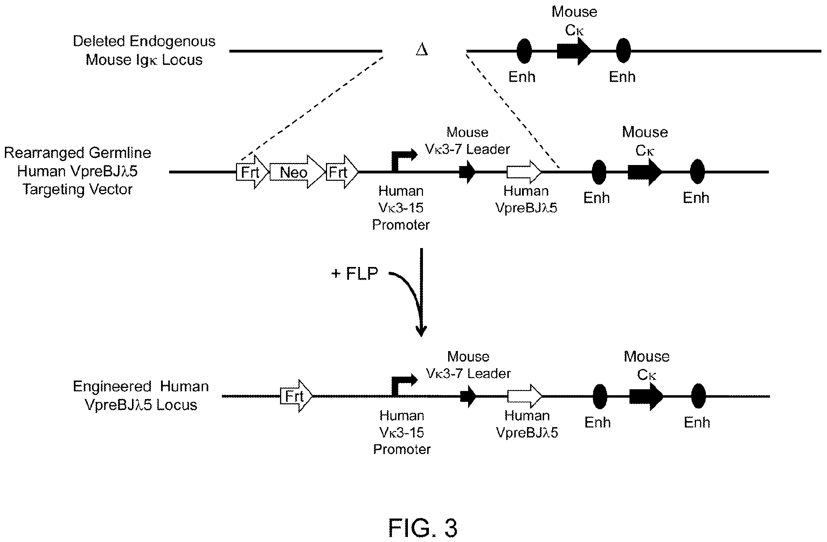

**FIG. 3** illustrates a targeting strategy for replacing endogenous mouse immunoglobulin light chain variable region gene segments with a human VpreB/Jλ5 gene region.

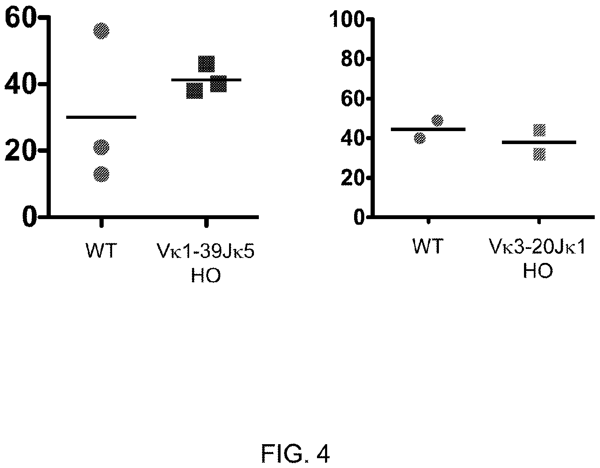

**FIG. 4** shows the percent of CD19⁺ B cells (y-axis) from peripheral blood for wild type mice (WT), mice homozygous for an engineered human rearranged Vκ1-39Jκ5 light chain region (Vκ1-39Jκ5 HO) and mice homozygous for an engineered human rearranged Vκ3-20Jκ1 light chain region (Vκ3-20Jκ1 HO).

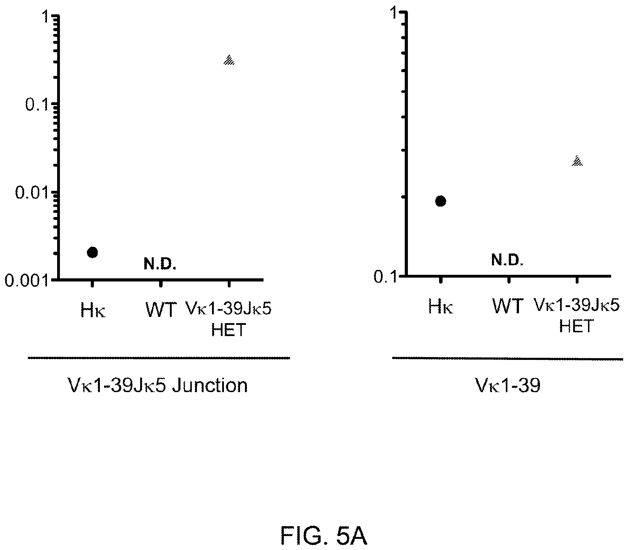

**FIG. 5A** shows the relative mRNA expression (y-axis) of a Vκ1-39-derived light chain in a quantitative PCR assay using probes specific for the junction of an engineered human rearranged Vκ1-39Jκ5 light chain region (Vκ1-39Jκ5 Junction Probe) and the human Vκ1-39 gene segment (Vκ1-39 Probe) in a mouse homozygous for a replacement of the endogenous Vκ and Jκ gene segments with human Vκ and Jκ gene segments (Hκ), a wild type mouse (WT), and a mouse heterozygous for an engineered human rearranged Vκ1-39Jκ5 light chain region (Vκ1-39Jκ5 HET). Signals are normalized to expression of mouse Cκ. N.D.: not detected.

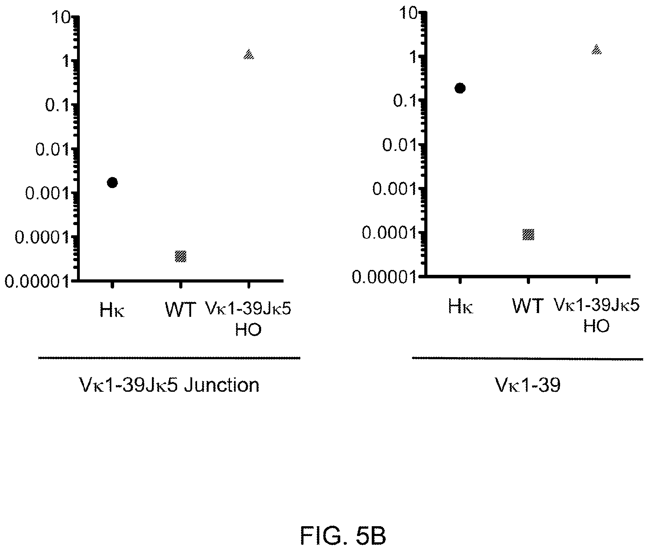

**FIG. 5B** shows the relative mRNA expression (y-axis) of a Vκ1-39-derived light chain in a quantitative PCR assay using probes specific for the junction of an engineered human rearranged Vκ1-39Jκ5 light chain region (Vκ1-39Jκ5 Junction Probe) and the human Vκ1-39 gene segment (Vκ1-39 Probe) in a mouse homozygous for a replacement of the endogenous Vκ and Jκ gene segments with human Vκ and Jκ gene segments (Hκ), a wild type mouse (WT), and a mouse homozygous for an engineered human rearranged Vκ1-39Jκ5 light chain region (Vκ1-39Jκ5 HO). Signals are normalized to expression of mouse Cκ.

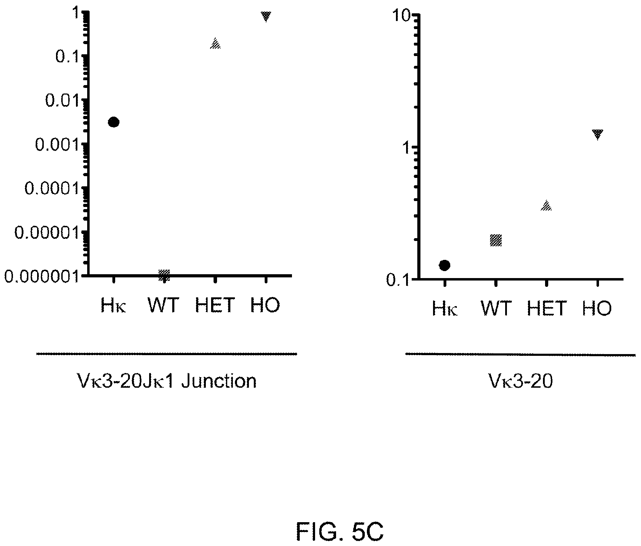

**FIG. 5C** shows the relative mRNA expression (y-axis) of a Vκ3-20-derived light chain in a quantitative PCR assay using probes specific for the junction of an engineered human rearranged Vκ3-20Jκ1 light chain region (Vκ3-20Jκ1 Junction Probe) and the human Vκ3-20 gene segment (Vκ3-20 Probe) in a mouse homozygous for a replacement of the endogenous Vκ and Jκ gene segments with human Vκ and Jκ gene segments (Hκ), a wild type mouse (WT), and a mouse heterozygous (HET) and homozygous (HO) for an engineered human rearranged Vκ3-20Jκ1 light chain region. Signals are normalized to expression of mouse Cκ.

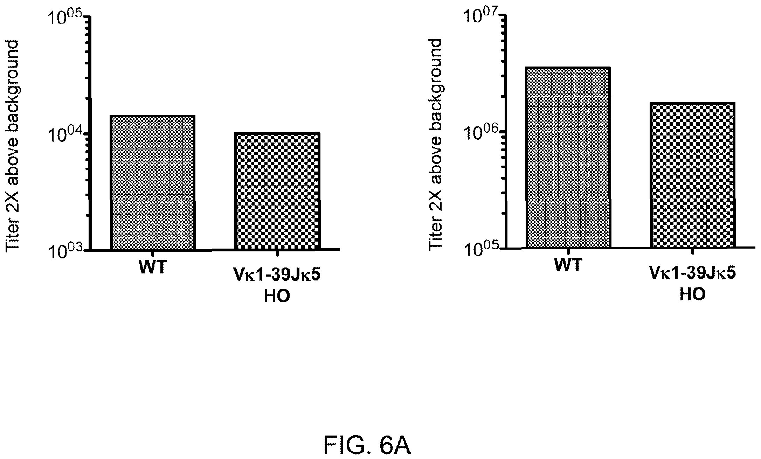

**FIG. 6A** shows IgM (left) and IgG (right) titer in wild type (WT; N=2) and mice homozygous for an engineered human rearranged Vκ1-39Jκ5 light chain region (Vκ1-39Jκ5 HO; N=2) immunized with β-galatosidase.

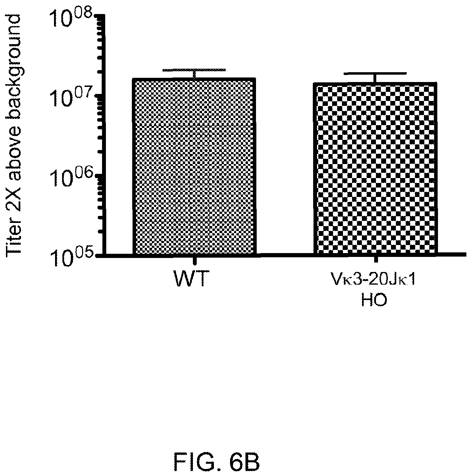

**FIG. 6B** shows total immunoglobulin (IgM, IgG, IgA) titer in wild type (WT; N=5) and mice homozygous for an engineered human rearranged Vκ3-20Jκ1 light chain region (Vκ3-20Jκ1 HO; N=5) immunized with β-galatosidase.

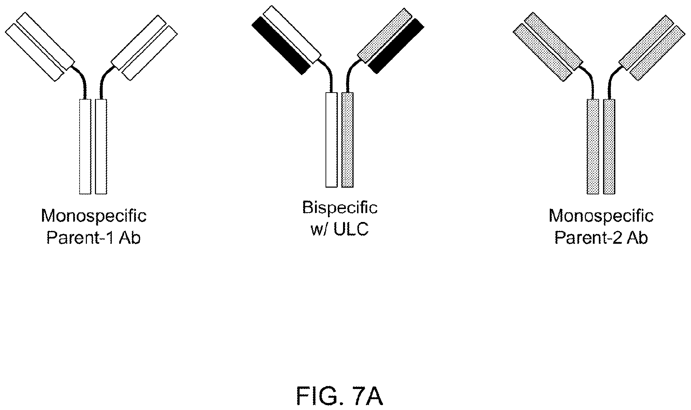

**FIG. 7A** shows a schematic of monospecific antibodies (Parent-1 and Parent-2) and a bispecific antibody (Bispecific) constructed from heavy chain variable regions from each parent monospecific antibody. A common light chain variable region (darkened) is indicated in the bispecific antibody.

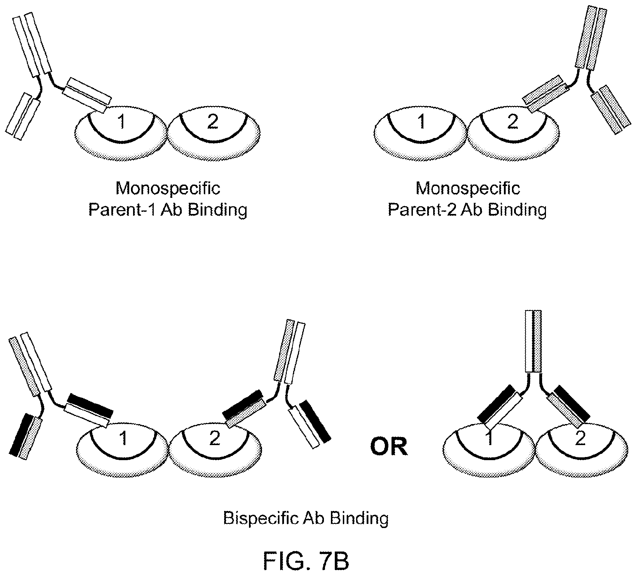

**FIG. 7B** shows a schematic for the binding characteristics of two parent monoclonal antibodies (Parent-1 and Parent-2) for an antigen of interest, as well as the binding characteristic of a bispecific antibody constructed from pairing the heavy chain variable regions from each monospecific parent antibody with a common light chain. The capability of the bispecific antibody to bind to two distinct epitopes of the antigen of interest either separately (bottom left) or simultaneously (bottom right) is indicated.

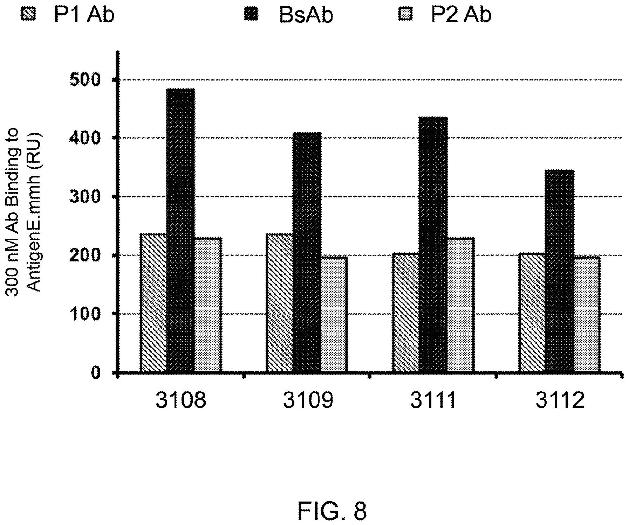

**FIG. 8** shows a bar graph of the binding of 300nM bispecific (darkened bars) and monospecific (striped and gray bars) antibodies to a captured monomeric Antigen E surface in BIACORE^{™} units (RU). Monoclonal parent-1 antibody (P1 Ab), monoclonal parent-2 (P2 Ab) and bispecific antibodies (BsAb) are indicated.

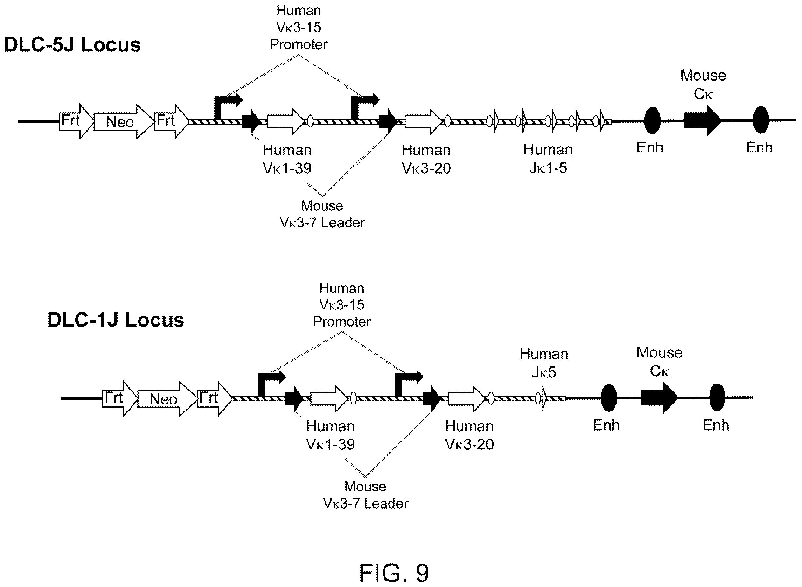

**FIG. 9** shows two genetically modified endogenous immunoglobulin light chain (*e*.*g*., κ light chain) loci. The locus on the top (DLC-5J) contains an engineered human DNA fragment (striped line) containing two human Vκ gene segments and five human Jκ gene segments. The locus on the bottom (DLC-1J) contains an engineered human DNA fragment (striped line) containing two human Vκ gene segments and one human Jκ gene segment. Each locus is capable of rearranging to form a human Vκ region operably linked to an endogenous light chain constant region (e.g., a Cκ). Immunoglobulin promoters (arrow above locus), leader exons (closed arrows), and the two human Vκ gene segments (open arrows), all flanked upstream (5') by a neomycin cassette containing Frt recombination sites are shown. Recombination signal sequences engineered with each of the human gene segments (Vκ and Jκ) are indicated by open ovals juxtaposed with each gene segment.

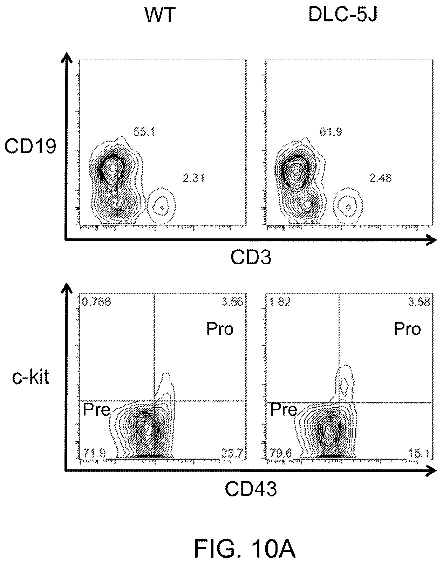

**FIG. 10A****,** in the top panel, shows representative contour plots of bone marrow stained for B and T cells (CD19⁺ and CD3⁺, respectively) from a wild type mouse (WT) and a mouse homozygous for two human Vκ and five human Jκ gene segments (DLC-5J). The bottom panel shows representative contour plots of bone marrow gated on CD19⁺ and stained for ckit⁺ and CD43⁺ from a wild type mouse (WT) and a mouse homozygous for two human Vκ and five human Jκ gene segments (DLC-5J). Pro and Pre B cells are noted on the contour plots of the bottom panel.

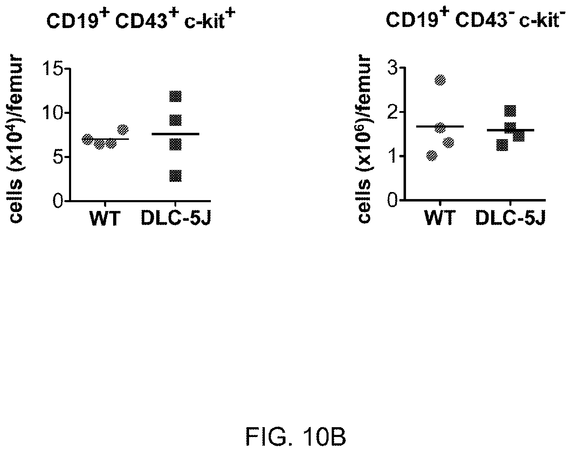

**FIG. 10B** shows the number of Pro (CD19⁺CD43⁺ckit⁺) and Pre (CD19⁺CD43⁻ckit⁻) B cells in bone marrow harvested from the femurs of wild type mice (WT) and mice homozygous for two human Vκ and five human Jκ gene segments (DLC-5J).

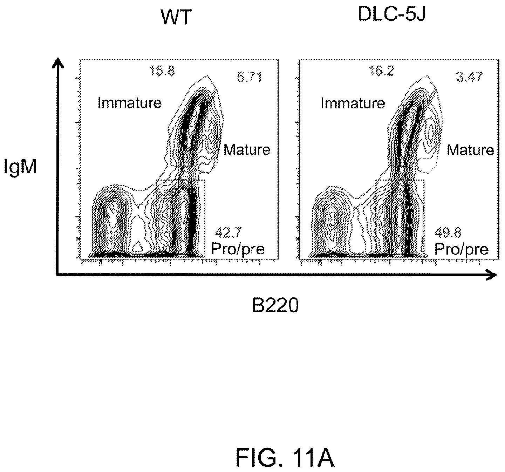

**FIG. 11A** shows representative contour plots of bone marrow gated on singlets stained for immunoglobulin M (IgM) and B220 from a wild type mouse (WT) and a mouse homozygous for two human Vκ and five human Jκ gene segments (DLC-5J). Immature, mature and pro/pre B cells are noted on each of the contour plots.

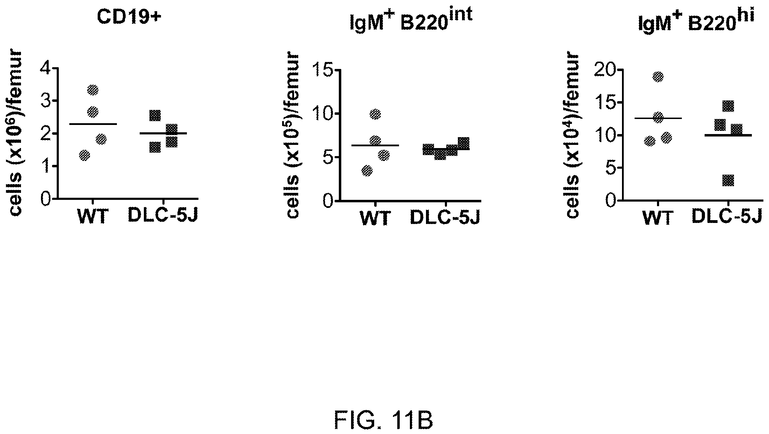

**FIG. 11B** shows the total number of B (CD19⁺), immature B (B220^{int}IgM⁺) and mature B (B220^{hi}IgM⁺) cells in bone marrow isolated from the femurs of wild type mice (WT) and mice homozygous for two human Vκ and five human Jκ gene segments (DLC-5J).

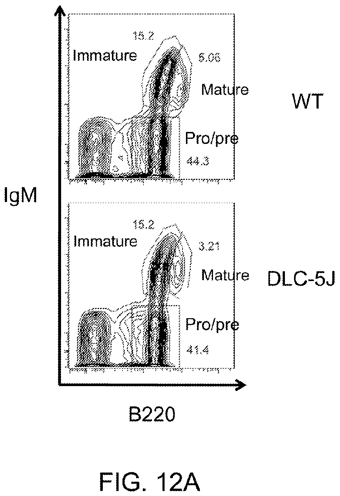

**FIG. 12A** shows representative contour plots of bone marrow gated on singlets stained for immunoglobulin M (IgM) and B220 from a wild type mouse (WT) and a mouse homozygous for two human Vκ and five human Jκ gene segments (DLC-5J). Immature, mature and pro/pre B cells are noted on each of the contour plots.

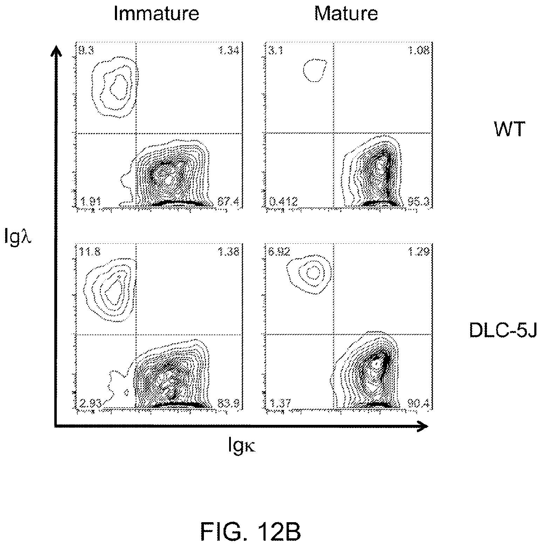

**FIG. 12B** shows representative contour plots of bone marrow gated on immature (B220^{int}IgM⁺) and mature (B220^{hi}IgM⁺) B cells stained for Igλ and Igκ expression isolated from the femurs of a wild type mouse (WT) and a mouse homozygous for two human Vκ and five human Jκ gene segments (DLC-5J).

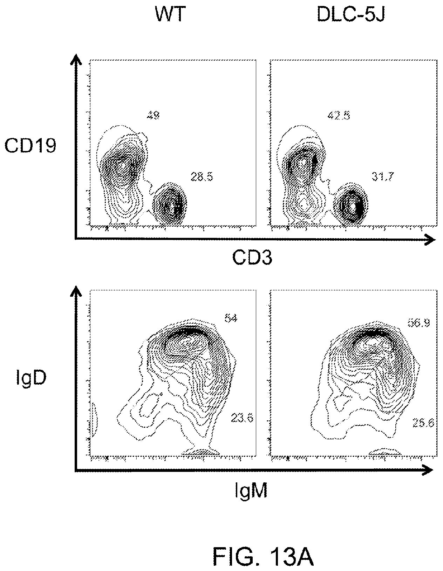

**FIG. 13A****,** in the top panel, shows representative contour plots of splenocytes gated on singlets and stained for B and T cells (CD19⁺ and CD3⁺, respectively) from a wild type mouse (WT) and a mouse homozygous for two human Vκ and five human Jκ gene segments (DLC-5J). The bottom panel shows representative contour plots of splenocytes gated on CD19⁺ and stained for immunoglobulin D (IgD) and immunoglobulin M (IgM) from a wild type mouse (WT) and a mouse homozygous for two human Vκ and five human Jκ gene segments (DLC-5J). Mature (54 for WT, 56.9 for DLC-5J) and transitional (23.6 for WT, 25.6 for DLC-5J) B cells are noted on each of the contour plots.

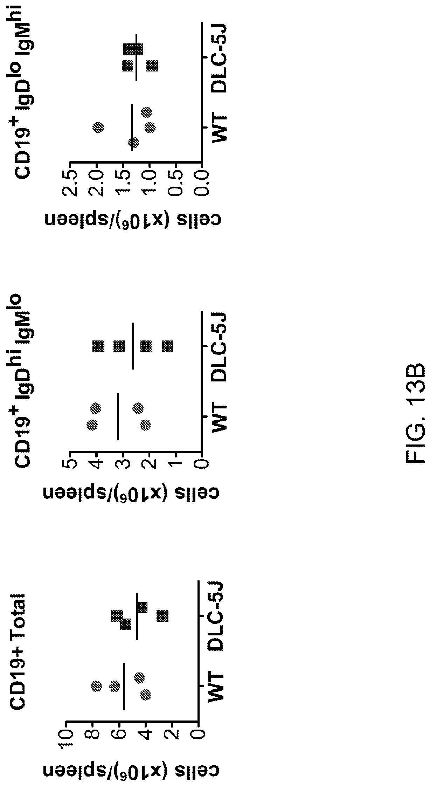

**FIG. 13B** shows the total number of CD19⁺ B cells, transitional B cells (CD19⁺IgM^{hi}IgD^{lo}) and mature B cells (CD19⁺lgM^{lo}lgD^{hi}) in harvested spleens from wild type mice (WT) and mice homozygous for two human Vκ and five human Jκ gene segments (DLC-5J).

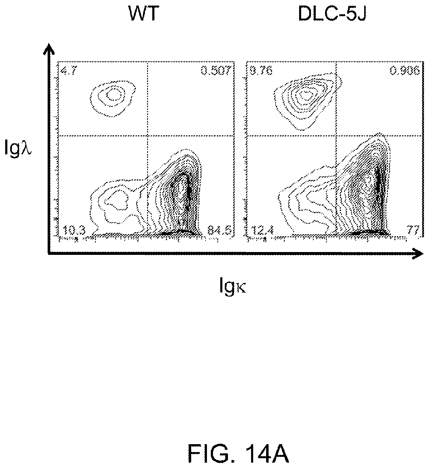

**FIG. 14A** shows representative contour plots of Igλ⁺ and Igκ⁺ splenocytes gated on CD19⁺ from a wild type mouse (WT) and a mouse homozygous for two human Vκ and five human Jκ gene segments (DLC-5J).

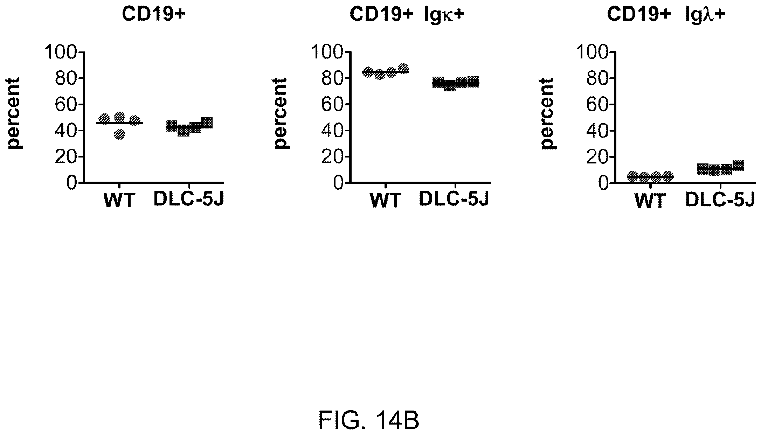

**FIG. 14B** shows the total number of B cells (CD19⁺), Igκ⁺ B cells (CD19⁺Igκ⁺) and Igλ⁺ B cells (CD19⁺lgλ⁺) in harvested spleens from wild type (WT) and mice homozygous for two human Vκ and five human Jκ gene segments (DLC-5J).

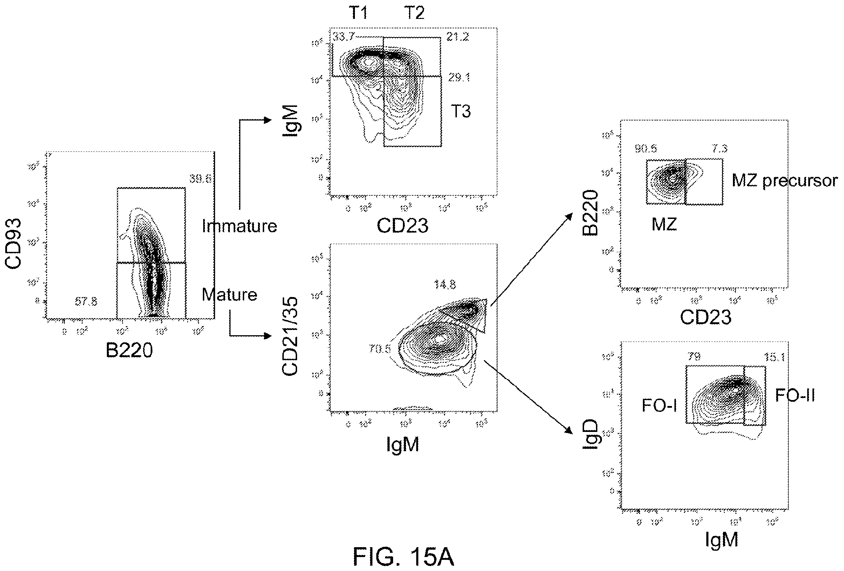

**FIG. 15A** shows the peripheral B cell development in mice homozygous for two human Vκ and five human Jκ gene segments. The first (far left) contour plot shows CD93⁺ and B220⁺ splenocytes gated on CD19⁺ indicating immature (39.6) and mature (57.8) B cells. The second (top middle) contour plot shows IgM⁺ and CD23⁺ expression in immature B cells indicating T1 (33.7; IgD⁻IgM⁺CD21^{lo}CD23⁻), T2 (21.2; IgD^{hi}IgM^{hi}CD21^{mid}CD23⁺) and T3 (29.1) B cell populations. The third (bottom middle) contour plot shows CD21⁺ (CD35⁺) and IgM⁺ expression of mature B cells indicating a small population (14.8) which give rise to marginal zone B cells and a second population (70.5) which gives rise to follicular (FO) B cells. The fourth (top right) contour plot shows B220⁺ and CD23⁺ expression in mature B cells indicating marginal zone (90.5; MZ) and marginal zone precursor (7.3; IgM^{hi}IgD^{hi}CD21^{hi}CD23⁺) B cell populations. The fifth (bottom right) contour plot shows IgD⁺ and IgM⁺ expression in mature B cells indicating FO-I (79.0; IgD^{hi}IgM^{lo}CD21^{mid}CD23⁺) and FO-II (15.1; IgD^{hi}IgM^{hi}CD21^{mid}CD23⁺) B cell populations. Percentage of cells within each gated region is shown.

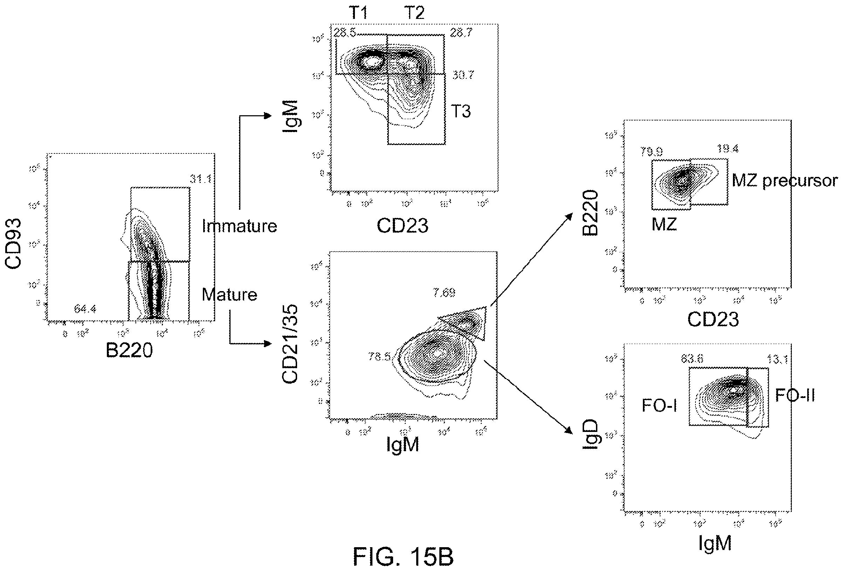

**FIG. 15B** shows the peripheral B cell development in wild type mice. The first (far left) contour plot shows CD93⁺ and B220⁺ splenocytes gated on CD19⁺ indicating immature (31.1) and mature (64.4) B cells. The second (top middle) contour plot shows IgM⁺ and CD23⁺ expression in immature B cells indicating T1 (28.5; IgD⁻IgM⁺CD21^{lo}CD23⁻), T2 (28.7; IgD^{hi}IgM^{hi}CD21^{mid}CD23⁺) and T3 (30.7) B cell populations. The third (bottom middle) contour plot shows CD21⁺ (CD35⁺) and IgM⁺ expression of mature B cells indicating a small population (7.69) which give rise to marginal zone B cells and a second population (78.5) which gives rise to follicular (FO) B cells. The fourth (top right) contour plot shows B220⁺ and CD23⁺ expression in mature B cells indicating marginal zone (79.9; MZ) and marginal zone precursor (19.4; IgM^{hi}IgD^{hi}CD21^{hi}CD23⁺) B cell populations. The fifth (bottom right) contour plot shows IgD⁺ and IgM⁺ expression in mature B cells indicating FO-I (83.6; IgD^{hi}IgM^{lo}CD21^{mid}CD23⁺) and FO-II (13.1; IgD^{hi}IgM^{hi}CD21^{mid}CD23⁺) B cell populations. Percentage of cells within each gated region is shown.

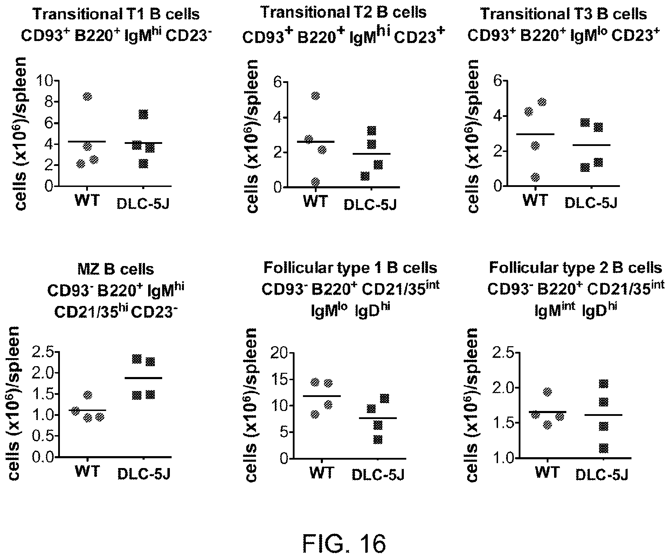

**FIG. 16** shows the total number of transitional, marginal zone and follicular B cell populations in harvested spleens of wild-type (WT) and mice homozygous for two human Vκ and five human Jκ gene segments (DLC-5J).

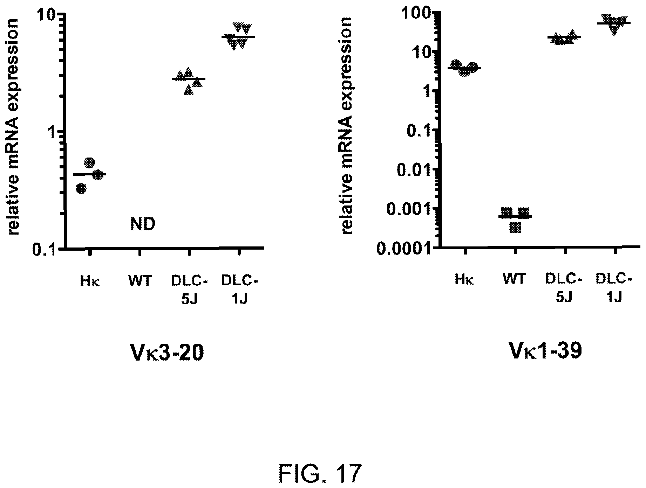

**FIG. 17** shows the relative mRNA expression in bone marrow (y-axis) of Vκ3-20-derived and Vκ1-39-derived light chains in a quantitative PCR assay using probes specific for Vκ3-20 or Vκ1-39 gene segments in mice homozygous for a replacement of the endogenous Vκ and Jκ gene segments with human Vκ and Jκ gene segments (Hκ), wild type mice (WT), mice homozygous for two human Vκ gene segments and five human Jκ gene segments (DLC-5J) and mice homozygous for two human Vκ gene segments and one

human Jκ gene segment (DLC-1J). Signals are normalized to expression of mouse Cκ. ND: not detected.

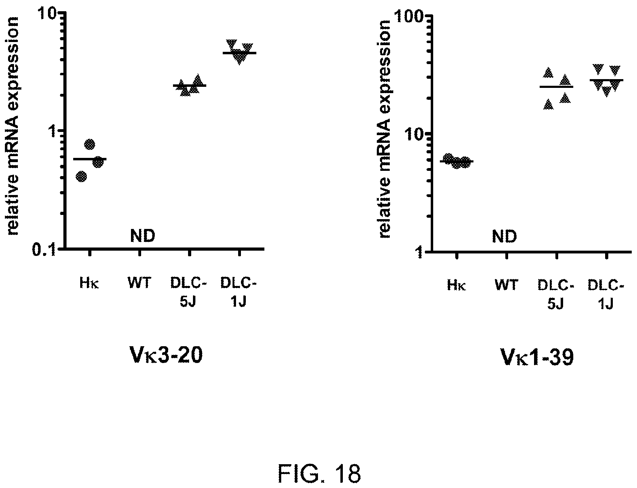

**FIG. 18** shows the relative mRNA expression in whole spleens (y-axis) of Vκ3-20-derived and Vκ1-39-derived light chains in a quantitative PCR assay using probes specific for Vκ3-20 or Vκ1-39 gene segments in mice homozygous for a replacement of the endogenous Vκ and Jκ gene segments with human Vκ and Jκ gene segments (Hκ), wild type mice (WT), mice homozygous for two human Vκ gene segments and five human Jκ gene segments (DLC-5J) and mice homozygous for two human Vκ gene segments and one human Jκ gene segment (DLC-1J). Signals are normalized to expression of mouse Cκ. ND: not detected.

**FIG. 19** shows a general illustration of recombination of a V and a J gene segment of an immunoglobulin κ light chain allele in a mouse and the structure of the light chain locus before rearrangement (top) and after rearrangement (bottom). Such a rearrangement as shown is only one of several possible rearrangement events.

### DETAILED DESCRIPTION

This invention is not limited to particular methods, and experimental conditions described, as such methods and conditions may vary. It is also to be understood that the terminology used herein is for the purpose of describing particular embodiments only, and is not intended to be limiting, since the scope of the present invention is defined by the claims.

Unless defined otherwise, all terms and phrases used herein include the meanings that the terms and phrases have attained in the art, unless the contrary is clearly indicated or clearly apparent from the context in which the term or phrase is used. Although any methods and materials similar or equivalent to those described herein can be used in the practice or testing of the present invention, particular methods and materials are now described.

The term "antibody", as used herein, includes immunoglobulin molecules comprising four polypeptide chains, two heavy (H) chains and two light (L) chains interconnected by disulfide bonds. Each heavy chain comprises a heavy chain variable (V_{H}) region and a heavy chain constant region (C_{H}). The heavy chain constant region comprises three domains, C_{H}1, C_{H}2 and C_{H}3. Each light chain comprises a light chain variable (V_{L}) region and a light chain constant region (C_{L}). The V_{H} and V_{L} regions can be further subdivided into regions of hypervariability, termed complementarity determining regions (CDR), interspersed with regions that are more conserved, termed framework regions (FR). Each V_{H} and V_{L} comprises three CDRs and four FRs, arranged from amino-terminus to carboxy-terminus in the following order: FR1, CDR1, FR2, CDR2, FR3, CDR3, FR4 (heavy chain CDRs may be abbreviated as HCDR1, HCDR2 and HCDR3; light chain CDRs may be abbreviated as LCDR1, LCDR2 and LCDR3. The term "high affinity" antibody refers to an antibody that has a K_{D} with respect to its target epitope about of 10⁻⁹ M or lower (*e*.*g*., about 1 x 10⁻⁹ M, 1 x 10⁻¹⁰ M, 1 x 10⁻¹¹ M, or about 1 x 10⁻¹² M). In one embodiment, K_{D} is measured by surface plasmon resonance, *e*.*g*., BIACORE^{™}; in another embodiment, K_{D} is measured by ELISA.

The phrase "bispecific antibody" refers to an antibody capable of selectively binding two or more epitopes. Bispecific antibodies include fragments of two different monoclonal antibodies (FIG. 7A) and generally comprise two nonidentical heavy chains derived from the two different monoclonal antibodies, with each heavy chain specifically binding a different epitope-either on two different molecules (*e*.*g*., different epitopes on two different immunogens; see FIG. 7B, bottom left) or on the same molecule (*e*.*g*., different epitopes on the same immunogen; see FIG. 7B, bottom right). If a bispecific antibody is capable of selectively binding two different epitopes (a first epitope and a second epitope), the affinity of the first heavy chain for the first epitope will generally be at least one to two or three or four or more orders of magnitude lower than the affinity of the first heavy chain for the second epitope, and vice versa. Epitopes specifically bound by the bispecific antibody can be on the same or a different target (*e*.*g*., on the same or a different protein; see FIG. 7B). Exemplary bispecific antibodies include those with a first heavy chain specific for a tumor antigen and a second heavy chain specific for a cytotoxic marker, *e*.*g*., an Fc receptor (*e.g.,* FcyRI, FcyRII, FcγRIII, etc.) or a T cell marker (*e.g.,* CD3, CD28, etc.). Further, the second heavy chain variable region can be substituted with a heavy chain variable region having a different desired specificity. For example, a bispecific antibody with a first heavy chain specific for a tumor antigen and a second heavy chain specific for a toxin can be paired so as to deliver a toxin (*e.g.,* saporin, vinca alkaloid, *etc.*) to a tumor cell. Other exemplary bispecific antibodies include those with a first heavy chain specific for an activating receptor (e.g., B cell receptor, FcyRl, FcyRIIA, FcγRIIIA, FcαRI, T cell receptor, *etc.*) and a second heavy chain specific for an inhibitory receptor (*e.g.,* FcyRIIB, CD5, CD22, CD72, CD300a, *etc.*)*.* Such bispecific antibodies can be constructed for therapeutic conditions associated with cell activation (*e*.*g*. allergy and asthma). Bispecific antibodies can be made, for example, by combining heavy chains that recognize different epitopes of the same or different immunogen (FIG. 7B). For example, nucleic acid sequences encoding heavy chain variable sequences that recognize different epitopes of the same or different immunogen can be fused to nucleic acid sequences encoding the same or different heavy chain constant regions, and such sequences can be expressed in a cell that expresses an immunoglobulin light chain. A typical bispecific antibody has two heavy chains each having three heavy chain CDRs, followed by (N-terminal to C-terminal) a C_{H}1 domain, a hinge, a C_{H}2 domain, and a C_{H}3 domain, and an immunoglobulin light chain that either does not confer epitope-binding specificity but that can associate with each heavy chain, or that can associate with each heavy chain and that can bind one or more of the epitopes bound by the heavy chain epitope-binding regions, or that can associate with each heavy chain and enable binding or one or both of the heavy chains to one or both epitopes.

The term "cell" includes any cell that is suitable for expressing a recombinant nucleic acid sequence. Cells include those of prokaryotes and eukaryotes (single-cell or multiple-cell), bacterial cells (*e.g.,* strains of *E. coli, Bacillus spp., Streptomyces spp., etc.),* mycobacteria cells, fungal cells, yeast cells (*e.g., S. cerevisiae, S. pombe, P. pastoris, P. methanolica, etc.*), plant cells, insect cells (*e.g.,* SF-9, SF-21, baculovirus-infected insect cells, *Trichoplusia ni, etc.*), non-human animal cells, human cells, or cell fusions such as, for example, hybridomas or quadromas. In some instances, the cell is a human, monkey, ape, hamster, rat, or mouse cell. In some instances, the cell is eukaryotic and is selected from the following cells: CHO (*e.g.,* CHO K1, DXB-11 CHO, Veggie-CHO), COS (*e.g.,* COS-7), retinal cell, Vero, CV1, kidney (*e*.*g*., HEK293, 293 EBNA, MSR 293, MDCK, HaK, BHK), HeLa, HepG2, WI38, MRC 5, Colo205, HB 8065, HL-60, (e.g., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some embodiments, the cell comprises one or more viral genes, *e.g.,* a retinal cell that expresses a viral gene (*e.g.,* a PER.C6^{™} cell).

The phrase "complementarity determining region," or the term "CDR," includes an amino acid sequence encoded by a nucleic acid sequence of an organism's immunoglobulin genes that normally *(i.e.,* in a wild type animal) appears between two framework regions in a variable region of a light or a heavy chain of an immunoglobulin molecule (*e*.*g*., an antibody or a T cell receptor). A CDR can be encoded by, for example, a germline sequence or a rearranged or unrearranged sequence, and, for example, by a naive or a mature B cell or a T cell. A CDR can be somatically mutated (*e*.*g*., vary from a sequence encoded in an animal's germline), humanized, and/or modified with amino acid substitutions, additions, or deletions. In some circumstances (*e*.*g*., for a CDR3), CDRs can be encoded by two or more sequences *(e.g.,* germline sequences) that are not contiguous (*e.g.,* in an unrearranged nucleic acid sequence) but are contiguous in a B cell nucleic acid sequence, *e*.*g*., as the result of splicing or connecting the sequences (*e*.*g*., V-D-J recombination to form a heavy chain CDR3).

The term "conservative," when used to describe a conservative amino acid substitution, includes substitution of an amino acid residue by another amino acid residue having a side chain R group with similar chemical properties (*e*.*g*., charge or hydrophobicity). In general, a conservative amino acid substitution will not substantially change the functional properties of interest of a protein, for example, the ability of a variable region to specifically bind a target epitope with a desired affinity. Examples of groups of amino acids that have side chains with similar chemical properties include aliphatic side chains such as glycine, alanine, valine, leucine, and isoleucine; aliphatic-hydroxyl side chains such as serine and threonine; amide-containing side chains such as asparagine and glutamine; aromatic side chains such as phenylalanine, tyrosine, and tryptophan; basic side chains such as lysine, arginine, and histidine; acidic side chains such as aspartic acid and glutamic acid; and, sulfur-containing side chains such as cysteine and methionine. Conservative amino acids substitution groups include, for example, valine/leucine/isoleucine, phenylalanine/tyrosine, lysine/arginine, alanine/valine, glutamate/aspartate, and asparagine/glutamine. In some embodiments, a conservative amino acid substitution can be substitution of any native residue in a protein with alanine, as used in, for example, alanine scanning mutagenesis. In some embodiments, a conservative substitution is made that has a positive value in the PAM250 log-likelihood matrix disclosed in Gonnet et al. (1992) Exhaustive Matching of the Entire Protein Sequence Database, Science 256:1443-45. In some embodiments, the substitution is a moderately conservative substitution wherein the substitution has a nonnegative value in the PAM250 log-likelihood matrix.

In some embodiments, residue positions in an immunoglobulin light chain or heavy chain differ by one or more conservative amino acid substitutions. In some embodiments, residue positions in an immunoglobulin light chain or functional fragment thereof *(e.g.,* a fragment that allows expression and secretion from, *e.g.,* a B cell) are not identical to a light chain whose amino acid sequence is listed herein, but differs by one or more conservative amino acid substitutions.

The phrase "epitope-binding protein" includes a protein having at least one CDR and that is capable of selectively recognizing an epitope, *e*.*g*., is capable of binding an epitope with a K_{D} that is at about one micromolar or lower (e.g., a K_{D} that is about 1 x 10⁻⁶ M, 1 × 10⁻⁷ M, 1 x 10⁻⁹ M, 1 x 10⁻⁹ M, 1 x 10⁻¹⁰ M, 1 x 10⁻¹¹ M, or about 1 x 10⁻¹² M). Therapeutic epitope-binding proteins (e.g., therapeutic antibodies) frequently require a K_{D} that is in the nanomolar or the picomolar range.

The phrase "functional fragment" includes fragments of epitope-binding proteins that can be expressed, secreted, and specifically bind to an epitope with a K_{D} in the micromolar, nanomolar, or picomolar range. Specific recognition includes having a K_{D} that is at least in the micromolar range, the nanomolar range, or the picomolar range.

The term "germline" includes reference to an immunoglobulin nucleic acid sequence in a non-somatically mutated cell, *e*.*g*., a non-somatically mutated B cell or pre-B cell or hematopoietic cell.

The phrase "heavy chain," or "immunoglobulin heavy chain" includes an immunoglobulin heavy chain constant region sequence from any organism. Heavy chain variable domains include three heavy chain CDRs and four FR regions, unless otherwise specified. Fragments of heavy chains include CDRs, CDRs and FRs, and combinations thereof. A typical heavy chain has, following the variable domain (from N-terminal to C-terminal), a C_{H}1 domain, a hinge, a C_{H}2 domain, and a C_{H}3 domain. A functional fragment of a heavy chain includes a fragment that is capable of specifically recognizing an epitope (*e*.*g*., recognizing the epitope with a K_{D} in the micromolar, nanomolar, or picomolar range), that is capable of expressing and secreting from a cell, and that comprises at least one CDR.

The term "identity" when used in connection with sequence includes identity as determined by a number of different algorithms known in the art that can be used to measure nucleotide and/or amino acid sequence identity. In some embodiments described herein, identities are determined using a ClustalW v. 1.83 (slow) alignment employing an open gap penalty of 10.0, an extend gap penalty of 0.1, and using a Gonnet similarity matrix (MACVECTOR^{™} 10.0.2, MacVector Inc., 2008). The length of the sequences compared with respect to identity of sequences will depend upon the particular sequences, but in the case of a light chain constant domain, the length should contain sequence of sufficient length to fold into a light chain constant domain that is capable of self-association to form a canonical light chain constant domain, e.g., capable of forming two beta sheets comprising beta strands and capable of interacting with at least one C_{H}1 domain of a human or a mouse. In the case of a C_{H}1 domain, the length of sequence should contain sequence of sufficient length to fold into a C_{H}1 domain that is capable of forming two beta sheets comprising beta strands and capable of interacting with at least one light chain constant domain of a mouse or a human.

The phrase "immunoglobulin molecule" includes two immunoglobulin heavy chains and two immunoglobulin light chains. The heavy chains may be identical or different, and the light chains may be identical or different.

The phrase "light chain" includes an immunoglobulin light chain sequence from any organism, and unless otherwise specified includes human κ and λ light chains and a VpreB, as well as surrogate light chains. Light chain variable (V_{L}) domains typically include three light chain CDRs and four framework (FR) regions, unless otherwise specified. Generally, a full-length light chain includes, from amino terminus to carboxyl terminus, a V_{L} domain that includes FR1-CDR1-FR2-CDR2-FR3-CDR3-FR4, and a light chain constant domain. Light chains include those, *e*.*g*., that do not selectively bind either a first or a second epitope selectively bound by the epitope-binding protein in which they appear. Light chains also include those that bind and recognize, or assist the heavy chain with binding and recognizing, one or more epitopes selectively bound by the epitope-binding protein in which they appear. Common light chains are those derived from a rearranged human Vκ1-39Jκ5 sequence or a rearranged human Vκ3-20Jκ1 sequence, and include somatically mutated (e.g., affinity matured) versions.

The phrase "micromolar range" is intended to mean 1-999 micromolar; the phrase "nanomolar range" is intended to mean 1-999 nanomolar; the phrase "picomolar range" is intended to mean 1-999 picomolar.

The phrase "somatically mutated" includes reference to a nucleic acid sequence from a B cell that has undergone class-switching, wherein the nucleic acid sequence of an immunoglobulin variable region (*e*.*g*., a heavy chain variable domain or including a heavy chain CDR or FR sequence) in the class-switched B cell is not identical to the nucleic acid sequence in the B cell prior to class-switching, such as, for example, a difference in a CDR or framework nucleic acid sequence between a B cell that has not undergone class-switching and a B cell that has undergone class-switching. "Somatically mutated" includes reference to nucleic acid sequences from affinity-matured B cells that are not identical to corresponding immunoglobulin variable region sequences in B cells that are not affinity-matured (*i.e.,* sequences in the genome of germline cells). The phrase "somatically mutated" also includes reference to an immunoglobulin variable region nucleic acid sequence from a B cell after exposure of the B cell to an epitope of interest, wherein the nucleic acid sequence differs from the corresponding nucleic acid sequence prior to exposure of the B cell to the epitope of interest. The phrase "somatically mutated" refers to sequences from antibodies that have been generated in an animal, *e*.*g*., a mouse having human immunoglobulin variable region nucleic acid sequences, in response to an immunogen challenge, and that result from the selection processes inherently operative in such an animal.

The term "unrearranged," with reference to a nucleic acid sequence, includes nucleic acid sequences that exist in the germline of an animal cell.

The phrase "variable domain" includes an amino acid sequence of an immunoglobulin light or heavy chain (modified as desired) that comprises the following amino acid regions, in sequence from N-terminal to C-terminal (unless otherwise indicated): FR1, CDR1, FR2, CDR2, FR3, CDR3, FR4.

### Universal Light Chain

Prior efforts to make useful multispecific epitope-binding proteins, *e*.*g*., bispecific antibodies, have been hindered by variety of problems that frequently share a common paradigm: *in vitro* selection or manipulation of sequences to rationally engineer, or to engineer through trial-and-error, a suitable format for pairing a heterodimeric bispecific human immunoglobulin. Unfortunately, most if not all of the *in vitro* engineering approaches provide largely *ad hoc* fixes that are suitable, if at all, for individual molecules. On the other hand, *in vivo* methods for employing complex organisms to select appropriate pairings that are capable of leading to human therapeutics have not been realized.

Mice containing human immunoglobulin loci, variable and constant regions randomly inserted into the mouse genome, are known in the art. Initial strains of such mice contained a limited number of human immunoglobulin gene segments. Specifically, a handful of strains containing human immunoglobulin light chain gene segments contained either one, three or four human immunoglobulin V_{L} gene segments and five human immunoglobulin J_{L} gene segments (Taylor et al. 1992, Nucleic Acids Research 20(23): 6287-6295; Fishwild et al. 1996, Nature Biotechnology 14: 845-851; Lonberg et al. 1994, Nature 368: 856-859; Green et al. 1994, Nature Genetics 7:13-21; Green and Jakobovits 1998, J. Exp. Med. 188(3): 483-495; Green 1999, J. Immunol. Methods 231: 11-23). These mice that contained only a few human immunoglobulin V_{L} gene segments as part of fully human transgenes randomly inserted into the mouse genome demonstrated compromised B cell numbers, impaired B cell development and other immune deficiencies. Expression of the human immunoglobulin V_{L} genes, as detected by surface expression of human Cκ on B cells, was lower than the endogenous κ light chain as compared to wild type. Surprisingly, the present invention is concerned with mice whose B cell numbers and development is nearly wild-type in respects when mice are engineered at the endogenous immunoglobulin κ light chain loci to contain either one or two human immunoglobulin Vκ gene segments (e.g., Examples 2 and 14, Tables 3, 25 and 26, and FIGs. 4, 10A-18). Further, in some embodiments, mice are able to generate several high-affinity reverse chimeric antibodies containing human V_{H} and V_{L} domains in response to antigen, wherein the V_{L} domains each contain one of two possible human V_{L} gene segments and one of five possible human J_{L} gene segments (*e*.*g*., see Examples 5 - 10, 12, and 14). Thus, in contrast to preliminary strains of mice engineered with human immunoglobulin light chain miniloci *(i.e.,* a limited number of human immunoglobulin gene segments), presently engineered mice that contain a limited number of human immunoglobulin V_{L} gene segments (two) and 5 human immunoglobulin J_{L} gene segments, surprisingly exhibit normal B cell numbers, normal immunoglobulin light chain expression, and normal B cell development. Further, such mice also show no reduced or impaired ability to mount robust immune responses to multiple antigens as a result of a limited immunoglobulin light chain repertoire. Accordingly, mouse ES cells, and mouse embryos are provided that comprise a humanized V_{L} locus comprising two unrearranged human immunoglobulin V_{L} gene segments and 5 human immunoglobulin J_{L} gene segments and which can give rise to mice that exhibit wild-type B cell populations in number, and exhibit wild-type B cell development.

Generally, native mouse sequences are frequently not a good source for human therapeutic sequences. For at least that reason, generating mouse heavy chain immunoglobulin variable regions that pair with a common human light chain is of limited practical utility. More *in vitro* engineering efforts would be expended in a trial-and-error process to try to humanize the mouse heavy chain variable sequences while hoping to retain epitope specificity and affinity while maintaining the ability to couple with the common human light chain, with uncertain outcome. At the end of such a process, the final product may maintain some of the specificity and affinity, and associate with the common light chain, but ultimately immunogenicity in a human would likely remain a profound risk.

Therefore, a suitable mouse for making human therapeutics would include a suitably large repertoire of human heavy chain variable region gene segments in place of endogenous mouse heavy chain variable region gene segments. The human heavy chain variable region gene segments should be able to rearrange and recombine with an endogenous mouse heavy chain constant domain to form a reverse chimeric heavy chain *(i.e.,* a heavy chain comprising a human variable domain and a mouse constant region). The heavy chain should be capable of class switching and somatic hypermutation so that a suitably large repertoire of heavy chain variable domains are available for the mouse to select one that can associate with the limited repertoire of human light chain variable regions.

A mouse that selects a common light chain for a plurality of heavy chains has a practical utility. In various embodiments, antibodies that express in a mouse that can only express a common light chain will have heavy chains that can associate and express with an identical or substantially identical light chain. This is particularly useful in making bispecific antibodies. For example, such a mouse can be immunized with a first immunogen to generate a B cell that expresses an antibody that specifically binds a first epitope. The mouse (or a mouse genetically the same) can be immunized with a second immunogen to generate a B cell that expresses an antibody that specifically binds the second epitope. Variable heavy chain regions can be cloned from the B cells and expressed with the same heavy chain constant region, and the same variable light chain region (e.g., a common light chain) in a cell to make a bispecific antibody, wherein the variable heavy chain component of the bispecific antibody has been selected by a mouse to associate and express with the variable light chain (or common light chain) component.

The inventors have engineered a mouse for generating immunoglobulin light chains that will suitably pair with a rather diverse family of heavy chains, including heavy chains whose variable regions depart from germline sequences, *e*.*g*., affinity matured or somatically mutated variable regions. In various embodiments, the mouse is devised to pair human light chain variable domains with human heavy chain variable domains that comprise somatic mutations, thus enabling a route to high affinity binding proteins suitable for use as human therapeutics.

The genetically engineered mouse, through the long and complex process of antibody selection within an organism, makes biologically appropriate choices in pairing a diverse collection of human heavy chain variable domains with a limited number of human light chain options. In order to achieve this, the mouse is engineered to present a limited number of human light chain variable domain options in conjunction with a wide diversity of human heavy chain variable domain options. Upon challenge with an immunogen, the mouse maximizes the number of solutions in its repertoire to develop an antibody to the immunogen, limited largely or solely by the number or light chain options in its repertoire. In various embodiments, this includes allowing the mouse to achieve suitable and compatible somatic mutations of the light chain variable domain that will nonetheless be compatible with a relatively large variety of human heavy chain variable domains, including in particular somatically mutated human heavy chain variable domains.

To achieve a limited repertoire of light chain options, the mouse is engineered to render nonfunctional or substantially nonfunctional its ability to make, or rearrange, a native mouse light chain variable domain. This can be achieved, e.g., by deleting the mouse's light chain variable region gene segments. The endogenous mouse locus can then be modified by an exogenous suitable human light chain variable region gene segment of choice, operably linked to the endogenous mouse light chain constant domain, in a manner such that the exogenous human variable region gene segments can combine with the endogenous mouse light chain constant region gene and form a rearranged reverse chimeric light chain gene (human variable, mouse constant). In various embodiments, the light chain variable region is capable of being somatically mutated. In various embodiments, to maximize ability of the light chain variable region to acquire somatic mutations, the appropriate enhancer(s) is retained in the mouse. For example, in modifying a mouse κ light chain locus to replace endogenous mouse κ light chain gene segments with human κ light chain gene segments, the mouse κ intronic enhancer and mouse κ 3' enhancer are functionally maintained, or undisrupted.

Hence, the present invention provides a mouse embryonic stem (ES) cell that has genetically modified so that it comprises in its genome:
exactly two unrearranged human immunoglobulin Vκ gene segments and five unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment. The present invention also provides a mouse embryo derived from such a genetically modified ES cell.

The present invention also provides a method of making a genetically modified mouse ES cell, the method comprising genetically modifying an ES cell so that it comprises in its genome exactly two unrearranged human immunoglobulin Vκ gene segments and five unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment.

The present invention also provides a method of making a genetically modified mouse comprising genetically modifying the germline genome of the mouse so that it includes exactly two unrearranged human immunoglobulin Vκ gene segments and five unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment.

Also referred to is a genetically engineered mouse that expresses a limited repertoire of reverse chimeric (human variable, mouse constant) light chains associated with a diversity of reverse chimeric (human variable, mouse constant) heavy chains.

In various embodiments, the endogenous mouse κ light chain gene segments are deleted and replaced with two rearranged human light chain region, operably linked to the endogenous mouse Cκ gene. In embodiments for maximizing somatic hypermutation of the rearranged human light chain region, the mouse κ intronic enhancer and the mouse κ 3' enhancer are maintained. In various embodiments, the mouse also comprises a nonfunctional λ light chain locus, or a deletion thereof or a deletion that renders the locus unable to make a λ light chain.

Also referred to is a genetically engineered mouse that, in various instances, comprises a light chain variable region locus lacking endogenous mouse light chain V_{L} and J_{L} gene segments and comprising a rearranged human light chain variable region, in one instance a rearranged human V_{L}/J_{L} sequence, operably linked to a mouse constant region, wherein the locus is capable of undergoing somatic hypermutation, and wherein the locus expresses a light chain comprising the human V_{L}/J_{L} sequence linked to a mouse constant region. Thus, in various instances, the locus comprises a mouse κ 3' enhancer, which is correlated with a normal, or wild type, level of somatic hypermutation.

The genetically engineered mouse in various embodiments when immunized with an antigen of interest generates B cells that exhibit a diversity of rearrangements of human immunoglobulin heavy chain variable regions that express and function with two rearranged light chains, including embodiments where the two light chains comprise human light chain variable regions that comprise, *e*.*g*., 1 to 5 somatic mutations. In various embodiments, the human light chains so expressed are capable of associating and expressing with any human immunoglobulin heavy chain variable region expressed in the mouse.

In addition to genetically engineered mice comprising restricted immunoglobulin light chain repertoire (two human V_{L} gene segments and, five human J_{L} gene segments) as described herein, also described herein are other genetically modified non-human animals that comprise a single human V_{L} gene segment or no more than two human V_{L} gene segments. In some instances, such non-human animals comprise a single rearranged human V_{L} region composed of a rearranged human V_{L}J_{L} sequence. In some instances, such non-human animals comprise no more than two human V_{L} gene segments and 5 human J_{L} gene segments. In various instances, human gene segments are operably linked to a non-human light chain constant region, e.g., a mouse a rat light chain constant region.

The non-human animal ES cells and embryos provided, are mouse ES cells and mouse embryos and the non-human animal made by a method of the present invention is a a mouse. For non-human animals where suitable genetically modifiable ES cells are not readily available, other methods are employed to make a non-human animal comprising genetic modifications as described herein. Such methods include, e.g., modifying a non-ES cell genome (e.g., a fibroblast or an induced pluripotent cell) and employing nuclear transfer to transfer the modified genome to a suitable cell, e.g., an oocyte, and gestating the modified cell (e.g., the modified oocyte) in a non-human animal under suitable conditions to form an embryo.

In some embodiments, the mouse is of a C57BL strain selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, and C57BL/Ola. In some certain embodiments, a mouse is a 129 strain selected from the group consisting of a strain that is 129P1, 129P2, 129P3,129X1, 129S1 (e.g., 129S1/SV, 129S1/Svlm), 129S2, 129S4, 129S5, 129S9/SvEvH, 129S6 (129/SvEvTac), 129S7, 129S8, 129T1, 129T2 (see, e.g., Festing et al., 1999, Mammalian Genome 10:836; Auerbach et al., 2000, Biotechniques 29(5):1024-1028, 1030, 1032). In some certain embodiments, a genetically modified mouse is a mix of an aforementioned 129 strain and an aforementioned C57BL/6 strain. In some certain embodiments, a mouse is a mix of aforementioned 129 strains, or a mix of aforementioned BL/6 strains. In some certain embodiments, a 129 strain of the mix as described herein is a 129S6 (129/SvEvTac) strain. In some embodiment, a mouse is a BALB strain, e.g., BALB/c strain. In some embodiments, a mouse is a mix of a BALB strain and another aforementioned strain.

### Epitope-binding Proteins Binding More Than One Epitope

Compositions and methods described herein can be used to make binding proteins that bind more than one epitope with high affinity, *e*.*g*., bispecific antibodies. Advantages include the ability to select suitably high binding (*e*.*g*., affinity matured) heavy chain immunoglobulin chains each of which will associate with a single light chain.

Several techniques for making bispecific antibody fragments from recombinant cell culture have been reported. However, synthesis and expression of bispecific binding proteins has been problematic, in part due to issues associated with identifying a suitable light chain that can associate and express with two different heavy chains, and in part due to isolation issues. In various embodiments, compositions and methods described herein provide the advantage of full length bispecific antibodies that do not require special modification(s) to maintain traditional immunoglobulin structure by increasing stability/interaction of the components (FIG. 7A). In various embodiments, such modification(s) has proven cumbersome and served as an obstacle to development of bispecific antibody technology and their potential use in treating for human disease. Thus, in various embodiments, through providing a natural immunoglobulin structure (*i.e.,* full length) having the added property of multiple specificities, full length bispecific antibodies maintain their critical effector functions that previous bispecific fragments lack, and further provide therapeutics that demonstrate the important pharmacokinetic parameter of a longer half-life.

Methods and compositions described herein allow for a genetically modified mouse to select, through otherwise natural processes, a suitable light chain that can associate and express with more than one heavy chain, including heavy chains that are somatically mutated (*e*.*g*., affinity matured). Human V_{L} and V_{H} sequences from suitable B cells of immunized mice as described herein that express affinity matured antibodies having reverse chimeric heavy chains (i.e., human variable and mouse constant) can be identified and cloned in frame in an expression vector with a suitable human constant region gene sequence (*e*.*g*., a human IgG1). Two such constructs can be prepared, wherein each construct encodes a human heavy chain variable domain that binds a different epitope. One of the human V_{L}s (*e.g.,* human Vκ1-39Jκ5 or human Vκ3-20Jκ1), in germline sequence or from a B cell wherein the sequence has been somatically mutated, can be fused in frame to a suitable human constant region gene (*e*.*g*., a human κ constant gene). These three fully human heavy and light constructs can be placed in a suitable cell for expression. The cell will express two major species: a homodimeric heavy chain with the identical light chain, and a heterodimeric heavy chain with the identical light chain. To allow for a facile separation of these major species, one of the heavy chains is modified to omit a Protein A-binding determinant, resulting in a differential affinity of a homodimeric binding protein from a heterodimeric binding protein. Compositions and methods that address this issue are described in USSN 12/832,838, filed 25 June 2010, entitled "Readily Isolated Bispecific Antibodies with Native Immunoglobulin Format," published as US 2010/0331527A1.

Also referred to is an epitope-binding protein, wherein human V_{L} and V_{H} sequences are derived from mice described herein that have been immunized with an antigen comprising an epitope of interest.

Also referred to is an epitope-binding protein that comprises a first and a second polypeptide, the first polypeptide comprising, from N-terminal to C-terminal, a first epitope-binding region that selectively binds a first epitope, followed by a constant region that comprises a first C_{H}3 region of a human IgG selected from IgG1, IgG2, IgG4, and a combination thereof; and, a second polypeptide comprising, from N-terminal to C-terminal, a second epitope-binding region that selectively binds a second epitope, followed by a constant region that comprises a second C_{H}3 region of a human IgG selected from IgG1, IgG2, IgG4, and a combination thereof, wherein the second C_{H}3 region comprises a modification that reduces or eliminates binding of the second C_{H}3 domain to protein A.

In one instance, the second C_{H}3 region comprises an H95R modification (by IMGT exon numbering; H435R by EU numbering). In another embodiment, the second C_{H}3 region further comprises a Y96F modification (IMGT; Y436F by EU).

In one instance, the second C_{H}3 region is from a modified human IgG1, and further comprises a modification selected from the group consisting of D16E, L18M, N44S, K52N, V57M, and V82I (IMGT; D356E, L358M, N384S, K392N, V397M, and V422I by EU).

In one instance, the second C_{H}3 region is from a modified human IgG2, and further comprises a modification selected from the group consisting of N44S, K52N, and V82I (IMGT; N384S, K392N, and V422I by EU).

In one instance, the second C_{H}3 region is from a modified human IgG4, and further comprises a modification selected from the group consisting of Q15R, N44S, K52N, V57M, R69K, E79Q, and V82I (IMGT; Q355R, N384S, K392N, V397M, R409K, E419Q, and V422I by EU).

One method for making an epitope-binding protein that binds more than one epitope is to immunize a first mouse with an antigen that comprises a first epitope of interest, wherein the mouse comprises an endogenous immunoglobulin light chain variable region locus that does not contain an endogenous mouse V_{L} that is capable of rearranging and forming a light chain, wherein at the endogenous mouse immunoglobulin light chain variable region locus is a single rearranged human V_{L} region operably linked to the mouse endogenous light chain constant region gene, and the rearranged human V_{L} region is selected from a human Vκ1-39Jκ5 and a human Vκ3-20Jκ1, and the endogenous mouse V_{H} gene segments have been replaced in whole or in part with human V_{H} gene segments, such that immunoglobulin heavy chains made by the mouse are solely or substantially heavy chains that comprise human variable domains and mouse constant domains. When immunized, such a mouse will make a reverse chimeric antibody, comprising only one of two human light chain variable domains (*e*.*g*., one of human Vκ1-39Jκ5 or human Vκ3-20Jκ1). Once a B cell is identified that encodes a V_{H} that binds the epitope of interest, the nucleotide sequence of the V_{H} (and, optionally, the V_{L}) can be retrieved (*e*.*g*., by PCR) and cloned into an expression construct in frame with a suitable human immunoglobulin constant domain. This process can be repeated to identify a second V_{H} domain that binds a second epitope, and a second V_{H} gene sequence can be retrieved and cloned into an expression vector in frame to a second suitable immunoglobulin constant domain. The first and the second immunoglobulin constant domains can the same or different isotype, and one of the immunoglobulin constant domains (but not the other) can be modified as described herein or in US 2010/0331527A1, and epitope-binding protein can be expressed in a suitable cell and isolated based on its differential affinity for Protein A as compared to a homodimeric epitope-binding protein, *e*.*g*., as described in US 2010/0331527A1.

Also disclosed is a method for making a bispecific epitope-binding protein, comprising identifying a first affinity-matured (*e*.*g*., comprising one or more somatic hypermutations) human V_{H} nucleotide sequence (V_{H}1) from a mouse as described herein, identifying a second affinity-matured (*e*.*g*., comprising one or more somatic hypermutations) human V_{H} nucleotide sequence (V_{H}2) from a mouse as described herein, cloning V_{H}1 in frame with a human heavy chain lacking a Protein A-determinant modification as described in US 2010/0331527A1 for form heavy chain 1 (HC1), cloning V_{H}2 in frame with a human heavy chain comprising a Protein A-determinant as described in US 2010/0331527A1 to form heavy chain 2 (HC2), introducing an expression vector comprising HC1 and the same or a different expression vector comprising HC2 into a cell, wherein the cell also expresses a human immunoglobulin light chain that comprises a human Vκ1-39/human Jκ5 or a human Vκ3-20/human Jκ1 fused to a human light chain constant domain, allowing the cell to express a bispecific epitope-binding protein comprising a V_{H} domain encoded by V_{H}1 and a V_{H} domain encoded by V_{H}2, and isolating the bispecific epitope-binding protein based on its differential ability to bind Protein A as compared with a monospecific homodimeric epitope-binding protein. In a specific instance, HC1 is an IgG1, and HC2 is an IgG1 that comprises the modification H95R (IMGT; H435R by EU) and further comprises the modification Y96F (IMGT; Y436F by EU). In one instance, the VH domain encoded by V_{H}1, the V_{H} domain encoded by V_{H}2, or both, are somatically mutated.

### Human V_{H} Genes That Express with a Common Human V_{L}

A variety of human variable regions from affinity-matured antibodies raised against four different antigens were expressed with either their cognate light chain, or at least one of a human light chain selected from human Vκ1-39/Jκ5, human Vκ3-20/Jκ1, or human VpreB/Jλ5 (see Example 1). For antibodies to each of the antigens, somatically mutated high affinity heavy chains from different gene families paired successfully with rearranged human germline Vκ1-39Jκ5 and Vκ3-20Jκ1 regions and were secreted from cells expressing the heavy and light chains. For Vκ1-39Jκ5 and Vκ3-20Jκ1, V_{H} domains derived from the following human V_{H} gene families expressed favorably: 1-2, 1-8, 1-24, 2-5, 3-7, 3-9, 3-11, 3-13, 3-15, 3-20, 3-23, 3-30, 3-33, 3-48, 4-31, 4-39, 4-59, 5-51, and 6-1. Thus, a mouse that is engineered to express a limited repertoire of human V_{L} domains from both of Vκ1-39Jκ5 and Vκ3-20Jκ1 will generate a diverse population of somatically mutated human V_{H} domains from a V_{H} locus modified to replace mouse V_{H} gene segments with human V_{H} gene segments.

Mice genetically engineered to express reverse chimeric (human variable, mouse constant) immunoglobulin heavy chains associated with a single rearranged light chain (*e*.*g*., a Vκ1-39/J or a Vκ3-20/J), when immunized with an antigen of interest, generated B cells that comprised a diversity of human V_{H} rearrangements and expressed a diversity of high-affinity antigen-specific antibodies with diverse properties with respect to their ability to block binding of the antigen to its ligand, and with respect to their ability to bind variants of the antigen (see Examples 5 through 10).

Thus, the mice and methods described herein are useful in making and selecting human immunoglobulin heavy chain variable domains, including somatically mutated human heavy chain variable domains, that result from a diversity of rearrangements, that exhibit a wide variety of affinities (including exhibiting a K_{D} of about a nanomolar or less), a wide variety of specificities (including binding to different epitopes of the same antigen), and that associate and express with the same or substantially the same human immunoglobulin light chain variable region.

### Fully Human Bispecific Antibodies Having a Common Light Chain

As a first step in various instances, the first and second nucleic acid sequences that each encode human heavy chain variable domains (and any additional nucleic acid sequences forming the bispecific antibody) are selected from parent monoclonal antibodies having desired characteristics such as, for example, capable of binding different epitopes (see FIGs. 7A and 7B), having different affinities, *etc.* Normally, the nucleic acid sequences encoding the human heavy chain variable domains are isolated from immunized mice, as described herein, to allow for fusing with human heavy chain constant regions to be suitable for human administration. Further modifications to the sequence(s) can be made by introducing mutations that add additional functionality to the bispecific antibody can be achieved, which include, for example, increasing serum half-life (*e*.*g*., see U.S. 7,217,797) and/or increasing antibody-dependent cell-mediated cytotoxicity (*e*.*g*., see U.S. 6,737,056). Introducing mutations into the constant regions of antibodies is known in the art. Additionally, part of the bispecific antibody can be made recombinantly in cell culture and other part(s) of the molecule can be made by those techniques mentioned above.

Several techniques for the producing antibodies have been described. For example, in various instances chimeric antibodies are produced in mice as described herein. Antibodies can be isolated directly from B cells of an immunized mouse (*e*.*g*., see U.S. 2007/0280945A1) and/or the B cells of the immunized mouse can be used to make hybridomas (Kohler and Milstein, 1975, Nature 256:495-497). DNA encoding the antibodies (human heavy and/or light chains) from mice as described herein is readily isolated and sequenced using conventional techniques. Hybridoma and/or B cells of derived from mice as described herein serve as a preferred source of such DNA. Once isolated, the DNA may be placed into expression vectors, which are then transfected into host cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the murine sequences.

In various instances, following isolation of the DNA and selection of the first and second nucleic acid sequences that encode the first and second human heavy chain variable domains having the desired specificities/affinities, and a third nucleic acid sequence that encodes a human light chain domain (a germline rearranged sequence or a light chain sequence isolated from a mouse as described herein), the three nucleic acids sequences encoding the molecules are expressed to form the bispecific antibody using recombinant techniques which are widely available in the art. Often, the expression system of choice will involve a mammalian cell expression vector and host so that the bispecific antibody is appropriately glycosylated (*e*.*g*., in the case of bispecific antibodies comprising antibody domains which are glycosylated). However, the molecules can also be produced in the prokaryotic expression systems. Normally, the host cell will be transformed with DNA encoding both the first human heavy chain variable domain, the second human heavy chain variable domain, the human light chain domain on a single vector or independent vectors. However, it is possible to express the first human heavy chain variable domain, second human heavy chain variable domain, and human light chain domain (the bispecific antibody components) in independent expression systems and couple the expressed polypeptides in vitro. In various instances, the human light chain domain comprises a germline sequence. In various instances, the human light chain domain comprises no more than one, no more than two, no more than three, no more than four, or no more than five somatic hypermutations with the light chain variable sequence of the light chain domain.

In various instances embodiments, the nucleic acid(s) (*e*.*g*., cDNA or genomic DNA) encoding the two heavy chains and single human light chain is inserted into a replicable vector for further cloning (amplification of the DNA) and/or for expression. Many vectors are available, and generally include, but are not limited to, one or more of the following: a signal sequence, an origin of replication, one or more marker genes, an enhancer element, a promoter, and a transcription termination sequence. Each component may be selected individually or based on a host cell choice or other criteria determined experimentally. Several examples of each component are known in the art.

Expression and cloning vectors usually contain a promoter that is recognized by the host organism and is operably linked to the nucleic acid sequences that encode each or all the components of the bispecific antibody. A large number of promoters recognized by a variety of potential host cells are well known. These promoters are operably linked to bispecific antibody-encoding DNA by removing the promoter from the source DNA by restriction enzyme digestion and inserting the isolated promoter sequence into the vector.

Expression vectors used in eukaryotic host cells (yeast, fungi, insect, plant, animal, human, or nucleated cells from other multicellular organisms) may also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding the bispecific antibody components. Suitable expression vectors for various instances include those that provide for the transient expression in mammalian cells of DNA encoding the bispecific antibody. In general, transient expression involves the use of an expression vector that is able to replicate efficiently in a host cell, such that the host cell accumulates many copies of the expression vector and, in turn, synthesizes high levels of a desired polypeptide encoded by the expression vector. Transient expression systems, comprising a suitable expression vector and a host cell, allow for the convenient positive identification of polypeptides encoded by cloned DNAs, as well as for the rapid screening of bispecific antibodies having desired binding specificities/affinities or the desired gel migration characteristics relative to the parental antibodies having homodimers of the first or second human heavy chain variable domains.

In various instances, once the DNA encoding the components of the bispecific antibody are assembled into the desired vector(s) as described above, they are introduced into a suitable host cell for expression and recovery. Transfecting host cells can be accomplished using standard techniques known in the art appropriate to the host cell selected (*e*.*g*., electroporation, nuclear microinjection, bacterial protoplast fusion with intact cells, or polycations, *e.g.,* polybrene, polyornithine, *etc.).*

A host cell is chosen, in various instances, that best suits the expression vector containing the components and allows for the most efficient and favorable production of the bispecific antibody species. Exemplary host cells for expression include those of prokaryotes and eukaryotes (single-cell or multiple-cell), bacterial cells (*e*.*g*., strains of E. coli, *Bacillus spp., Streptomyces spp., etc.*), mycobacteria cells, fungal cells, yeast cells (*e.g., S. cerevisiae, S. pombe, P. pastoris, P. methanolica, etc.*), plant cells, insect cells (*e.g.,* SF-9, SF-21, baculovirus-infected insect cells, *Trichoplusia ni, etc.*), non-human animal cells, human cells, or cell fusions such as, for example, hybridomas or quadromas. In various embodiments, the cell is a human, monkey, ape, hamster, rat, or mouse cell. In various embodiments, the cell is eukaryotic cell selected from CHO (*e*.*g*., CHO K1, DXB-11 CHO, Veggie-CHO), COS *(e.g.,* COS-7), retinal cell, Vero, CV1, kidney *(e.g.,* HEK293, 293 EBNA, MSR 293, MDCK, HaK, BHK), HeLa, HepG2, WI38, MRC 5, Colo205, HB 8065, HL-60, (*e*.*g*., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In various instances, the cell comprises one or more viral genes, *e.g.* a retinal cell that expresses a viral gene (*e.g.,* a PER.C6^{™} cell).

Mammalian host cells used to produce the bispecific antibody may be cultured in a variety of media. Commercially available media such as Ham's F10 (Sigma), Minimal Essential Medium ((MEM), Sigma), RPMI-1640 (Sigma), and Dulbeccols Modified Eagle's Medium ((DMEM), Sigma) are suitable for culturing the host cells. Media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as GENTAMYCIN^{™}), trace elements (defined as inorganic compounds usually present at final concentrations in the micromolar range), and glucose or an equivalent energy source. Any other supplements may also be included at appropriate concentrations as known to those skilled in the art. The culture conditions, such as temperature, pH, and the like, are, in various embodiments, those previously used with the host cell selected for expression, and will be apparent to those skilled in the art.

The bispecific antibody is in various instances recovered from the culture medium as a secreted polypeptide, although it also may be recovered from host cell lysate when directly produced without a secretory signal. If the bispecific antibody is membrane-bound, it can be released from the membrane using a suitable detergent solution (e.g., Triton-X 100). Preferably, the bispecific antibodies described herein involves the use of a first immunoglobulin C_{H}3 domain and a second immunoglobulin C_{H}3 domain, wherein the first and second immunoglobulin C_{H}3 domains differ from one another by at least one amino acid, and wherein at least one amino acid difference reduces binding of the bispecific antibody to Protein A as compared to a bi-specific antibody lacking the amino acid difference (see U.S. 2010/0331527A1). In one instancet, the first immunoglobulin C_{H}3 domain binds Protein A and the second immunoglobulin C_{H}3 domain contains a mutation that reduces or abolishes Protein A binding such as an H95R modification (by IMGT exon numbering; H435R by EU numbering). The second C_{H}3 may further comprise a Y96F modification (by IMGT; Y436F by EU). Further modifications that may be found within the second C_{H}3 include: D16E, L18M, N44S, K52N, V57M, and V82I (by IMGT; D356E, L358M, N384S, K392N, V397M, and V422I by EU) in the case of IgG1 antibodies; N44S, K52N, and V82I (IMGT; N384S, K392N, and V422I by EU) in the case of IgG2 antibodies; and Q15R, N44S, K52N, V57M, R69K, E79Q, and V82I (by IMGT; Q355R, N384S, K392N, V397M, R409K, E419Q, and V422I by EU) in the case of IgG4 antibodies. Variations on the bi-specific antibody format described above are contemplated.

Because of the dual nature of bispecific antibodies *(i.e.,* may be specific for different epitopes of one polypeptide or may contain antigen-binding domains specific for more than one target polypeptide, see FIG. 7B; see also, *e.g.,* Tutt et al., 1991, J. Immunol. 147:60-69; Kufer et al., 2004, Trends Biotechnol. 22:238-244), they offer many useful advantages for therapeutic application. For example, the bispecific antibodies can be used for redirected cytotoxicity (*e*.*g*., to kill tumor cells), as a vaccine adjuvant, for delivering thrombolytic agents to clots, for converting enzyme activated prodrugs at a target site (*e*.*g*., a tumor), for treating infectious diseases, targeting immune complexes to cell surface receptors, or for delivering immunotoxins to tumor cells.

The bispecific antibodies described herein can also be used in several therapeutic and non-therapeutic and/or diagnostic assay methods, such as, enzyme immunoassays, two-site immunoassays, *in vitro* or *in vivo* immunodiagnosis of various diseases (*e*.*g*., cancer), competitive binding assays, direct and indirect sandwich assays, and immunoprecipitation assays. Other uses for the bispecific antibodies will be apparent to those skilled in the art.

The following examples are provided so as to describe to those of ordinary skill in the art how to make and use methods and compositions of the invention, and are not intended to limit the scope of what the inventors regard as their invention. Efforts have been made to ensure accuracy with respect to numbers used (*e.g.,* amounts, temperature, *etc.*) but some experimental errors and deviations should be accounted for. Unless indicated otherwise, parts are parts by weight, molecular weight is average molecular weight, temperature is indicated in Celsius, and pressure is at or near atmospheric.

### EXAMPLES

The following examples are provided so as to describe to those of ordinary skill in the art how to make and use methods and compositions of the invention, and are not intended to limit the scope of what the inventors regard as their invention. Efforts have been made to ensure accuracy with respect to numbers used (*e*.*g*., amounts, temperature, etc.) but some experimental errors and deviations should be accounted for. Unless indicated otherwise, temperature is indicated in Celsius, pressure is at or near atmospheric, parts are by parts by weight, and molecular weight is average molecular weight.

### Example 1. Identification of Human V_{H} Regions That Associate with Selected Human V_{L} Regions

An *in vitro* expression system was constructed to determine if a single rearranged human germline light chain could be co-expressed with human heavy chains from antigen specific human antibodies.

Methods for generating human antibodies in genetically modified mice are known (see *e*.*g*., US 6,596,541, Regeneron Pharmaceuticals, VELOCIMMUNE^{®}). The VELOCIMMUNE^{®} technology involves generation of a genetically modified mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibodies produced from a VELOCIMMUNE^{®} mouse are fully human. Initially, high affinity chimeric antibodies are isolated having a human variable region and a mouse constant region. As described below, the antibodies are characterized and selected for desirable characteristics, including affinity, selectivity, epitope, *etc.* The mouse constant regions are replaced with a desired human constant region to generate a fully human antibody containing a non-IgM isotype, for example, wild type or modified IgG1, IgG2, IgG3 or IgG4. While the constant region selected may vary according to specific use, high affinity antigen-binding and target specificity characteristics reside in the variable region.

A VELOCIMMUNE^{®} mouse was immunized with a growth factor that promotes angiogenesis (Antigen C) and antigen-specific human antibodies were isolated and sequenced for V gene usage using standard techniques recognized in the art. Selected antibodies were cloned onto human heavy and light chain constant regions and 69 heavy chains were selected for pairing with one of three human light chains: (1) the cognate κ light chain linked to a human κ constant region, (2) a rearranged human germline Vκ1-39Jκ5 linked to a human κ constant region, or (3) a rearranged human germline Vκ3-20Jκ1 linked to a human κ constant region. Each heavy chain and light chain pair was co-transfected in CHO-K1 cells using standard techniques. Presence of antibody in the supernatant was detected by anti-human IgG in an ELISA assay. Antibody titer (ng/ml) was determined for each heavy chain/light chain pair and titers with the different rearranged germline light chains were compared to the titers obtained with the parental antibody molecule (*i.e.,* heavy chain paired with cognate light chain) and percent of native titer was calculated (Table 1). V_{H}: Heavy chain variable gene. ND: no expression detected under current experimental conditions.

**TABLE 1**

| V_{H} | Antibody Titer (ng/mL) | | | | Percent of Native Titer | |
|---|---|---|---|---|---|---|
| | Cognate LC | Vκ1-39Jκ5 | Vκ3-20Jκ1 | | Vκ1-39Jκ5 | Vκ3-20Jκ1 |
| 3-15 | 63 | 23 | 11 | | 36.2 | 17.5 |
| 1-2 | 103 | 53 | ND | | 51.1 | - |
| 3-23 | 83 | 60 | 23 | | 72.0 | 27.5 |
| 3-33 | 15 | 77 | ND | | 499.4 | - |
| 4-31 | 22 | 69 | 17 | | 309.4 | 76.7 |
| 3-7 | 53 | 35 | 28 | | 65.2 | 53.1 |
| - | 22 | 32 | 19 | | 148.8 | 89.3 |
| 1-24 | 3 | 13 | ND | | 455.2 | - |
| 3-33 | 1 | 47 | ND | | 5266.7 | - |
| 3-33 | 58 | 37 | ND | | 63.1 | - |
| - | 110 | 67 | 18 | | 60.6 | 16.5 |
| 3-23 | 127 | 123 | 21 | | 96.5 | 16.3 |
| 3-33 | 28 | 16 | 2 | | 57.7 | 7.1 |
| 3-23 | 32 | 50 | 38 | | 157.1 | 119.4 |
| - | 18 | 45 | 18 | | 254.3 | 101.7 |
| 3-9 | 1 | 30 | 23 | | 2508.3 | 1900.0 |
| 3-11 | 12 | 26 | 6 | | 225.9 | 48.3 |
| 1-8 | 16 | ND | 13 | | - | 81.8 |
| 3-33 | 54 | 81 | 10 | | 150.7 | 19.1 |
| - | 34 | 9 | ND | | 25.9 | - |
| 3-20 | 7 | 14 | 54 | | 203.0 | 809.0 |
| 3-33 | 19 | 38 | ND | | 200.5 | - |
| 3-11 | 48 | ND | 203 | | - | 423.6 |
| - | 11 | 23 | 8 | | 212.7 | 74.5 |
| 3-33 | 168 | 138 | 182 | | 82.0 | 108.2 |
| 3-20 | 117 | 67 | 100 | | 57.5 | 86.1 |
| 3-23 | 86 | 61 | 132 | | 70.7 | 154.1 |
| 3-33 | 20 | 12 | 33 | | 60.9 | 165.3 |
| 4-31 | 69 | 92 | 52 | | 133.8 | 75.0 |
| 3-23 | 87 | 78 | 62 | | 89.5 | 71.2 |
| 1-2 | 31 | 82 | 51 | | 263.0 | 164.6 |
| 3-23 | 53 | 93 | 151 | | 175.4 | 285.4 |
| - | 11 | 8 | 17 | | 75.7 | 151.4 |
| 3-33 | 114 | 36 | 27 | | 31.6 | 23.4 |
| 3-15 | 73 | 39 | 44 | | 53.7 | 59.6 |
| 3-33 | 1 | 34 | 16 | | 5600.0 | 2683.3 |
| 3-9 | 58 | 112 | 57 | | 192.9 | 97.6 |
| 3-33 | 67 | 20 | 105 | | 30.1 | 157.0 |
| 3-33 | 34 | 21 | 24 | | 62.7 | 70.4 |
| 3-20 | 10 | 49 | 91 | | 478.4 | 888.2 |
| 3-33 | 66 | 32 | 25 | | 48.6 | 38.2 |
| 3-23 | 17 | 59 | 56 | | 342.7 | 329.8 |
| - | 58 | 108 | 19 | | 184.4 | 32.9 |
| - | 68 | 54 | 20 | | 79.4 | 29.9 |
| 3-33 | 42 | 35 | 32 | | 83.3 | 75.4 |
| - | 29 | 19 | 13 | | 67.1 | 43.9 |
| 3-9 | 24 | 34 | 29 | | 137.3 | 118.4 |
| 3-30/33 | 17 | 33 | 7 | | 195.2 | 43.1 |
| 3-7 | 25 | 70 | 74 | | 284.6 | 301.6 |
| 3-33 | 87 | 127 | ND | | 145.1 | - |
| 6-1 | 28 | 56 | ND | | 201.8 | - |
| 3-33 | 56 | 39 | 20 | | 69.9 | 36.1 |
| 3-33 | 10 | 53 | 1 | | 520.6 | 6.9 |
| 3-33 | 20 | 67 | 10 | | 337.2 | 52.3 |
| 3-33 | 11 | 36 | 18 | | 316.8 | 158.4 |
| 3-23 | 12 | 42 | 32 | | 356.8 | 272.9 |
| 3-33 | 66 | 95 | 15 | | 143.6 | 22.5 |
| 3-15 | 55 | 68 | ND | | 123.1 | - |
| - | 32 | 68 | 3 | | 210.9 | 10.6 |
| 1-8 | 28 | 48 | ND | | 170.9 | - |
| 3-33 | 124 | 192 | 21 | | 154.3 | 17.0 |
| 3-33 | 0 | 113 | ND | | 56550.0 | - |
| 3-33 | 10 | 157 | 1 | | 1505.8 | 12.5 |
| 3-33 | 6 | 86 | 15 | | 1385.5 | 243.5 |
| 3-23 | 70 | 115 | 22 | | 163.5 | 31.0 |
| 3-7 | 71 | 117 | 21 | | 164.6 | 29.6 |
| 3-33 | 82 | 100 | 47 | | 122.7 | 57.1 |
| 3-7 | 124 | 161 | 41 | | 130.0 | 33.5 |

In a similar experiment, VELOCIMMUNE^{®} mice were immunized with several different antigens and selected heavy chains of antigen specific human antibodies were tested for their ability to pair with different rearranged human germline light chains (as described above). The antigens used in this experiment included an enzyme involved in cholesterol homeostasis (Antigen A), a serum hormone involved in regulating glucose homeostasis (Antigen B), a growth factor that promotes angiogenesis (Antigen C) and a cell-surface receptor (Antigen D). Antigen specific antibodies were isolated from mice of each immunization group and the heavy chain and light chain variable regions were cloned and sequenced. From the sequence of the heavy and light chains, V gene usage was determined and selected heavy chains were paired with either their cognate light chain or a rearranged human germline Vκ1-39Jκ5 region. Each heavy/light chain pair was co-transfected in CHO-K1 cells and the presence of antibody in the supernatant was detected by anti-human IgG in an ELISA assay. Antibody titer (µg/ml) was determined for each heavy chain/light chain pairing and titers with the different rearranged human germline light chains were compared to the titers obtained with the parental antibody molecule (*i.e.,* heavy chain paired with cognate light chain) and percent of native titer was calculated (Table 2). V_{H}: Heavy chain variable gene. Vκ: κ light chain variable gene. ND: no expression detected under current experimental conditions.

**TABLE 2**

| Antigen | Antibody | V_{H} | Vκ | Titer (µg/ml) | | | Percent of Native Titer |
|---|---|---|---|---|---|---|---|
| | | | | V_{H} Alone | V_{H} + Vκ | V_{H} + Vκ1-39Jκ5 | |
| A | 320 | 1-18 | 2-30 | 0.3 | 3.1 | 2.0 | 66 |
| | 321 | 2-5 | 2-28 | 0.4 | 0.4 | 1.9 | 448 |
| | 334 | 2-5 | 2-28 | 0.4 | 2.7 | 2.0 | 73 |
| | 313 | 3-13 | 3-15 | 0.5 | 0.7 | 4.5 | 670 |
| | 316 | 3-23 | 4-1 | 0.3 | 0.2 | 4.1 | 2174 |
| | 315 | 3-30 | 4-1 | 0.3 | 0.2 | 3.2 | 1327 |
| | 318 | 4-59 | 1-17 | 0.3 | 4.6 | 4.0 | 86 |
| | 257 | 3-13 | 1-5 | 0.4 | 3.1 | 3.2 | 104 |
| | 283 | 3-13 | 1-5 | 0.4 | 5.4 | 3.7 | 69 |
| | 637 | 3-13 | 1-5 | 0.4 | 4.3 | 3.0 | 70 |
| B | 638 | 3-13 | 1-5 | 0.4 | 4.1 | 3.3 | 82 |
| | 624 | 3-23 | 1-17 | 0.3 | 5.0 | 3.9 | 79 |
| | 284 | 3-30 | 1-17 | 0.3 | 4.6 | 3.4 | 75 |
| | 653 | 3-33 | 1-17 | 0.3 | 4.3 | 0.3 | 7 |
| | 268 | 4-34 | 1-27 | 0.3 | 5.5 | 3.8 | 69 |
| | 633 | 4-34 | 1-27 | 0.6 | 6.9 | 3.0 | 44 |
| | 730 | 3-7 | 1-5 | 0.3 | 1.1 | 2.8 | 249 |
| | 728 | 3-7 | 1-5 | 0.3 | 2.0 | 3.2 | 157 |
| | 691 | 3-9 | 3-20 | 0.3 | 2.8 | 3.1 | 109 |
| | 749 | 3-33 | 3-15 | 0.3 | 3.8 | 2.3 | 62 |
| | 750 | 3-33 | 1-16 | 0.3 | 3.0 | 2.8 | 92 |
| | 724 | 3-33 | 1-17 | 0.3 | 2.3 | 3.4 | 151 |
| | 706 | 3-33 | 1-16 | 0.3 | 3.6 | 3.0 | 84 |
| C | 744 | 1-18 | 1-12 | 0.4 | 5.1 | 3.0 | 59 |
| | 696 | 3-11 | 1-16 | 0.4 | 3.0 | 2.9 | 97 |
| | 685 | 3-13 | 3-20 | 0.3 | 0.5 | 3.4 | 734 |
| | 732 | 3-15 | 1-17 | 0.3 | 4.5 | 3.2 | 72 |
| | 694 | 3-15 | 1-5 | 0.4 | 5.2 | 2.9 | 55 |
| | 743 | 3-23 | 1-12 | 0.3 | 3.2 | 0.3 | 10 |
| | 742 | 3-23 | 2-28 | 0.4 | 4.2 | 3.1 | 74 |
| | 693 | 3-23 | 1-12 | 0.5 | 4.2 | 4.0 | 94 |
| | 136 | 3-23 | 2-28 | 0.4 | 5.0 | 2.7 | 55 |
| | 155 | 3-30 | 1-16 | 0.4 | 1.0 | 2.2 | 221 |
| | 163 | 3-30 | 1-16 | 0.3 | 0.6 | 3.0 | 506 |
| | 171 | 3-30 | 1-16 | 0.3 | 1.0 | 2.8 | 295 |
| | 145 | 3-43 | 1-5 | 0.4 | 4.4 | 2.9 | 65 |
| D | 49 | 3-48 | 3-11 | 0.3 | 1.7 | 2.6 | 155 |
| | 51 | 3-48 | 1-39 | 0.1 | 1.9 | 0.1 | 4 |
| | 159 | 3-7 | 6-21 | 0.4 | 3.9 | 3.6 | 92 |
| | 169 | 3-7 | 6-21 | 0.3 | 1.3 | 3.1 | 235 |
| | 134 | 3-9 | 1-5 | 0.4 | 5.0 | 2.9 | 58 |
| | 141 | 4-31 | 1-33 | 2.4 | 4.2 | 2.6 | 63 |
| | 142 | 4-31 | 1-33 | 0.4 | 4.2 | 2.8 | 67 |

The results obtained from these experiments demonstrate that somatically mutated, high affinity heavy chains from different gene families are able to pair with rearranged human germline Vκ1-39Jκ5 and Vκ3-20Jκ1 regions and be secreted from the cell as a normal antibody molecule. As shown in Table 1, antibody titer was increased for about 61% (42 of 69) heavy chains when paired with the rearranged human Vκ1-39Jκ5 light chain and about 29% (20 of 69) heavy chains when paired with the rearranged human Vκ3-20Jκ1 light chain as compared to the cognate light chain of the parental antibody. For about 20% (14 of 69) of the heavy chains, both rearranged human germline light chains conferred an increase in expression as compared to the cognate light chain of the parental antibody. As shown in Table 2, the rearranged human germline Vκ1-39Jκ5 region conferred an increase in expression of several heavy chains specific for a range of different classes of antigens as compared to the cognate light chain for the parental antibodies. Antibody titer was increased by more than two-fold for about 35% (15/43) of the heavy chains as compared to the cognate light chain of the parental antibodies. For two heavy chains (315 and 316), the increase was greater than ten-fold as compared to the parental antibody. Within all the heavy chains that showed increase expression relative to the cognate light chain of the parental antibody, family three (V_{H}3) heavy chains are over represented in comparison to other heavy chain variable region gene families. This demonstrates a favorable relationship of human V_{H}3 heavy chains to pair with rearranged human germline Vκ1-39Jκ5 and Vκ3-20Jκ1 light chains.

### Example 2. Generation of a Rearranged Human Germline Light Chain Locus

Various rearranged human germline light chain targeting vectors were made using VELOCIGENE^{®} technology (see, *e.g.,* US Pat. No. 6,586,251 and Valenzuela *et al.* (2003) High-throughput engineering of the mouse genome coupled with high-resolution expression analysis, Nature Biotech. 21(6): 652-659) to modify mouse genomic Bacterial Artificial Chromosome (BAC) clones 302g12 and 254m04 (Invitrogen). Using these two BAC clones, genomic constructs were engineered to contain a single rearranged human germline light chain region and inserted into an endogenous κ light chain locus that was previously modified to delete the endogenous κ variable and joining gene segments.

**Construction of Rearranged Human Germline Light Chain Targeting** Vectors. Three different rearranged human germline light chain regions were made using standard molecular biology techniques recognized in the art. The human variable gene segments used for constructing these three regions included rearranged human Vκ1-39Jκ5 sequence, a rearranged human Vκ3-20Jκ1 sequence and a rearranged human VpreBJλ5 sequence.

A DNA segment containing exon 1 (encoding the leader peptide) and intron 1 of the mouse Vκ3-7 gene was made by de novo DNA synthesis (Integrated DNA Technologies). Part of the 5' untranslated region up to a naturally occurring Blpl restriction enzyme site was included. Exons of human Vκ1-39 and Vκ3-20 genes were PCR amplified from human genomic BAC libraries. The forward primers had a 5' extension containing the splice acceptor site of intron 1 of the mouse Vκ3-7 gene. The reverse primer used for PCR of the human Vκ1-39 sequence included an extension encoding human Jκ5, whereas the reverse primer used for PCR of the human Vκ3-20 sequence included an extension encoding human Jκ1. The human VpreBJλ5 sequence was made by de novo DNA synthesis (Integrated DNA Technologies). A portion of the human Jκ-Cκ intron including the splice donor site was PCR amplified from plasmid pBS-296-HA18-PIScel. The forward PCR primer included an extension encoding part of either a human Jκ5, Jκ1, or Jλ5 sequence. The reverse primer included a PI-Scel site, which was previously engineered into the intron.

The mouse Vκ3-7 exon1/intron 1, human variable light chain exons, and human Jκ-Cκ intron fragments were sewn together by overlap extension PCR, digested with Blpl and PI-Scel, and ligated into plasmid pBS-296-HA18-PIScel, which contained the promoter from the human Vκ3-15 variable gene segment. A loxed hygromycin cassette within plasmid pBS-296-HA18-PIScel was replaced with a FRTed hygromycin cassette flanked by Notl and Ascl sites. The Notl/PI-Scel fragment of this plasmid was ligated into modified mouse BAC 254m04, which contained part of the mouse Jκ-Cκ intron, the mouse Cκ exon, and about 75 kb of genomic sequence downstream of the mouse κ locus, which provided a 3' homology arm for homologous recombination in mouse ES cells. The Notl/Ascl fragment of this BAC was then ligated into modified mouse BAC 302g12, which contained a FRTed neomycin cassette and about 23 kb of genomic sequence upstream of the endogenous κ locus for homologous recombination in mouse ES cells.

**Rearranged Human Germline Vκ1-39Jκ5 Targeting Vector (****FIG. 1****).** Restriction enzyme sites were introduced at the 5' and 3' ends of an engineered light chain insert for cloning into a targeting vector: an Ascl site at the 5' end and a PI-Scel site at the 3' end. Within the 5' Ascl site and the 3' PI-Scel site the targeting construct from 5' to 3' included a 5' homology arm containing sequence 5' to the endogenous mouse κ light chain locus obtained from mouse BAC clone 302g12, a FRTed neomycin resistance gene, an genomic sequence including the human Vκ3-15 promoter, a leader sequence of the mouse Vκ3-7 variable gene segment, a intron sequence of the mouse Vκ3-7 variable gene segment, an open reading frame of a rearranged human germline Vκ1-39Jκ5 region, a genomic sequence containing a portion of the human Jκ-Cκ intron, and a 3' homology arm containing sequence 3' of the endogenous mouse Jκ5 gene segment obtained from mouse BAC clone 254m04 (Figure 1, middle). Genes and/or sequences upstream of the endogenous mouse κ light chain locus and downstream of the most 3' Jκ gene segment (e.g., the endogenous 3' enhancer) were unmodified by the targeting construct (see Figure 1). The sequence of the engineered human Vκ1-39Jκ5 locus is shown in SEQ ID NO: 1.

Targeted insertion of the rearranged human germline Vκ1-39Jκ5 region into BAC DNA was confirmed by polymerase chain reaction (PCR) using primers located at sequences within the rearranged human germline light chain region. Briefly, the intron sequence 3' to the mouse Vκ3-7 leader sequence was confirmed with primers ULC-m1F (AGGTGAGGGT ACAGATAAGT GTTATGAG; SEQ ID NO: 2) and ULC-m1R (TGACAAATGC CCTAATTATA GTGATCA; SEQ ID NO: 3). The open reading frame of the rearranged human germline Vκ1-39Jκ5 region was confirmed with primers 1633-h2F (GGGCAAGTCA GAGCATTAGC A; SEQ ID NO: 4) and 1633-h2R (TGCAAACTGG ATGCAGCATA G; SEQ ID NO: 5). The neomycin cassette was confirmed with primers neoF (GGTGGAGAGG CTATTCGGC; SEQ ID NO: 6) and neoR (GAACACGGCG GCATCAG; SEQ ID NO: 7). Targeted BAC DNA was then used to electroporate mouse ES cells to created modified ES cells for generating chimeric mice that express a rearranged human germline Vκ1-39Jκ5 region.

Positive ES cell clones were confirmed by TAQMAN^{™} screening and karyotyping using probes specific for the engineered Vκ1-39Jκ5 light chain region inserted into the endogenous locus. Briefly, probe neoP (TGGGCACAAC AGACAATCGG CTG; SEQ ID NO: 8) which binds within the neomycin marker gene, probe ULC-m1P (CCATTATGAT GCTCCATGCC TCTCTGTTC; SEQ ID NO: 9) which binds within the intron sequence 3' to the mouse Vκ3-7 leader sequence, and probe 1633h2P (ATCAGCAGAA ACCAGGGAAA GCCCCT; SEQ ID NO: 10) which binds within the rearranged human germline Vκ1-39Jκ5 open reading frame. Positive ES cell clones were then used to implant female mice to give rise to a litter of pups expressing the germline Vκ1-39Jκ5 light chain region.

Alternatively, ES cells bearing the rearranged human germline Vκ1-39Jκ5 light chain region are transfected with a construct that expresses FLP in order to remove the FRTed neomycin cassette introduced by the targeting construct. Optionally, the neomycin cassette is removed by breeding to mice that express FLP recombinase (*e*.*g*., US 6,774,279). Optionally, the neomycin cassette is retained in the mice.

**Rearranged Human Germline Vκ3-20Jκ1 Targeting Vector (****FIG. 2****).** In a similar fashion, an engineered light chain locus expressing a rearranged human germline Vκ3-20Jκ1 region was made using a targeting construct including, from 5' to 3', a 5' homology arm containing sequence 5' to the endogenous mouse κ light chain locus obtained from mouse BAC clone 302g12, a FRTed neomycin resistance gene, a genomic sequence including the human Vκ3-15 promoter, a leader sequence of the mouse Vκ3-7 variable gene segment, an intron sequence of the mouse Vκ3-7 variable gene segment, an open reading frame of a rearranged human germline Vκ3-20Jκ1 region, a genomic sequence containing a portion of the human Jκ-Cκ intron, and a 3' homology arm containing sequence 3' of the endogenous mouse Jκ5 gene segment obtained from mouse BAC clone 254m04 (Figure 2, middle). The sequence of the engineered human Vκ3-20Jκ1 locus is shown in SEQ ID NO: 11.

Targeted insertion of the rearranged human germline Vκ3-20Jκ1 region into BAC DNA was confirmed by polymerase chain reaction (PCR) using primers located at sequences within the rearranged human germline Vκ3-20Jκ1 light chain region. Briefly, the intron sequence 3' to the mouse Vκ3-7 leader sequence was confirmed with primers ULC-m1F (SEQ ID NO: 2) and ULC-m1R (SEQ ID NO: 3). The open reading frame of the rearranged human germline Vκ3-20Jκ1 region was confirmed with primers 1635-h2F (TCCAGGCACC CTGTCTTTG; SEQ ID NO: 12) and 1635-h2R (AAGTAGCTGC TGCTAACACT CTGACT; SEQ ID NO: 13). The neomycin cassette was confirmed with primers neoF (SEQ ID NO: 6) and neoR (SEQ ID NO: 7). Targeted BAC DNA was then used to electroporate mouse ES cells to created modified ES cells for generating chimeric mice that express the rearranged human germline Vκ3-20Jκ1 light chain.

Positive ES cell clones were confirmed by TAQMAN^{™} screening and karyotyping using probes specific for the engineered Vκ3-20Jκ1 light chain region inserted into the endogenous κ light chain locus. Briefly, probe neoP (SEQ ID NO: 8) which binds within the neomycin marker gene, probe ULC-m1P (SEQ ID NO: 9) which binds within the mouse Vκ3-7 leader sequence, and probe 1635h2P (AAAGAGCCAC CCTCTCCTGC AGGG; SEQ ID NO: 14) which binds within the human Vκ3-20Jκ1 open reading frame. Positive ES cell clones were then used to implant female mice. A litter of pups expressing the human germline Vκ3-20Jκ1 light chain region.

Alternatively, ES cells bearing human germline Vκ3-20Jκ1 light chain region can be transfected with a construct that expresses FLP in order to remove the FRTed neomycin cassette introduced by the targeting construct. Optionally, the neomycin cassette may be removed by breeding to mice that express FLP recombinase (e.g., US 6,774,279). Optionally, the neomycin cassette is retained in the mice.

**Rearranged Human Germline VpreBJλ5 Targeting Vector (****FIG. 3****).** In a similar fashion, an engineered light chain locus expressing a rearranged human germline VpreBJλ5 region was made using a targeting construct including, from 5' to 3', a 5' homology arm containing sequence 5' to the endogenous mouse κ light chain locus obtained from mouse BAC clone 302g12, a FRTed neomycin resistance gene, an genomic sequence including the human Vκ3-15 promoter, a leader sequence of the mouse Vκ3-7 variable gene segment, an intron sequence of the mouse Vκ3-7 variable gene segment, an open reading frame of a rearranged human germline VpreBJλ5 region, a genomic sequence containing a portion of the human Jκ-Cκ intron, and a 3' homology arm containing sequence 3' of the endogenous mouse Jκ5 gene segment obtained from mouse BAC clone 254m04 (Figure 3, middle). The sequence of the engineered human VpreBJλ5 locus is shown in SEQ ID NO: 15.

Targeted insertion of the rearranged human germline VpreBJλ5 region into BAC DNA was confirmed by polymerase chain reaction (PCR) using primers located at sequences within the rearranged human germline VpreBJλ5 region light chain region. Briefly, the intron sequence 3' to the mouse Vκ3-7 leader sequence was confirmed with primers ULC-m1F (SEQ ID NO: 2) and ULC-m1R (SEQ ID NO: 3). The open reading frame of the rearranged human germline VpreBJλ5 region was confirmed with primers 1616-h1F (TGTCCTCGGC CCTTGGA; SEQ ID NO: 16) and 1616-h1R (CCGATGTCAT GGTCGTTCCT; SEQ ID NO: 17). The neomycin cassette was confirmed with primers neoF (SEQ ID NO: 6) and neoR (SEQ ID NO: 7). Targeted BAC DNA was then used to electroporate mouse ES cells to created modified ES cells for generating chimeric mice that express the rearranged human germline VpreBJλ5 light chain.

Positive ES cell clones are confirmed by TAQMAN^{™} screening and karyotyping using probes specific for the engineered VpreBJλ5 light chain region inserted into the endogenous κ light chain locus. Briefly, probe neoP (SEQ ID NO:8), which binds within the neomycin marker gene, probe ULC-m1P (SEQ ID NO: 9), which binds within the mouse IgVκ3-7 leader sequence, and probe 1616h1P (ACAATCCGCC TCACCTGCAC CCT; SEQ ID NO: 18) which binds within the human VpreBJλ5 open reading frame. Positive ES cell clones are then used to implant female mice to give rise to a litter of pups expressing a germline light chain region.

Alternatively, ES cells bearing the rearranged human germline VpreBJλ5 light chain region are transfected with a construct that expresses FLP in order to remove the FRTed neomycin cassette introduced by the targeting construct. Optionally, the neomycin cassette is removed by breeding to mice that express FLP recombinase (*e*.*g*., US 6,774,279). Optionally, the neomycin cassette is retained in the mice.

### Example 3. Generation of Mice Expressing a Single Rearranged Human Light Chain

Targeted ES cells described above were used as donor ES cells and introduced into an 8-cell stage mouse embryo by the VELOCIMOUSE^{®} method (see, *e*.*g*., US Pat. No. 7,294,754 and Poueymirou *et al.* (2007) F0 generation mice that are essentially fully derived from the donor gene-targeted ES cells allowing immediate phenotypic analyses Nature Biotech. 25(1): 91-99. VELOCIMICE^{®} independently bearing an engineered human germline Vκ1-39Jκ5 light chain region, a Vκ3-20Jκ1 light chain region or a VpreBJλ5 light chain region are identified by genotyping using a modification of allele assay (Valenzuela *et al., supra*) that detects the presence of the unique rearranged human germline light chain region.

Pups are genotyped and a pup heterozygous or homozygous for the unique rearranged human germline light chain region are selected for characterizing expression of the rearranged human germline light chain region.

**Flow Cytometry.** Expression of the rearranged human light chain region in the normal antibody repertoire of common light chain mice was validated by analysis of immunoglobulin κ and λ expression in splenocytes and peripheral blood of common light chain mice. Cell suspensions from harvested spleens and peripheral blood of wild type (n=5), Vκ1-39Jκ5 common light chain heterozygote (n=3), Vκ1-39Jκ5 common light chain homozygote (n=3), Vκ3-20Jκ1 common light chain heterozygote (n=2), and Vκ3-20Jκ1 common light chain homozygote (n=2) mice were made using standard methods and stained with CD19⁺, Igλ⁺ and Igκ⁺ using fluorescently labeled antibodies (BD Pharmigen).

Briefly, 1×10⁶ cells were incubated with anti-mouse CD16/CD32 (clone 2.4G2, BD Pharmigen) on ice for 10 minutes, followed by labeling with the following antibody cocktail for 30 minutes on ice: APC conjugated anti-mouse CD19 (clone 1D3, BD Pharmigen), PerCP-Cy5.5 conjugated anti-mouse CD3 (clone 17A2, BioLegend), FITC conjugated anti-mouse Igκ (clone 187.1, BD Pharmigen), PE conjugated anti-mouse Igλ (clone RML-42, BioLegend). Following staining, cells were washed and fixed in 2% formaldehyde. Data acquisition was performed on an LSRII flow cytometer and analyzed with FlowJo. Gating: total B cells (CD19⁺CD3⁻), Igκ⁺ B cells (Igκ⁺Igλ⁻CD19⁺CD3⁻), Igλ⁺ B cells (Igκ⁻Igλ⁺CD19⁺CD3⁻). Data gathered from blood and splenocyte samples demonstrated similar results. Table 3 sets forth the percent positive CD19⁺ B cells from peripheral blood of one representative mouse from each group that are Igλ⁺, Igκ⁺, or Igλ⁺Igκ⁺. Percent of CD19⁺ B cells in peripheral blood from wild type (WT) and mice homozygous for either the Vκ1-39Jκ5 or Vκ3-20Jκ1 common light chain are shown in FIG. 4.

**TABLE 3**

| Mouse | CD19⁺ B cells | | |
|---|---|---|---|
| | Igλ⁺ | Igκ⁺ | Igλ⁺Igκ⁺ |
| Wild type | 4.8 | 93 | 0.53 |
| Vκ1-39Jκ5 | 1.4 | 93 | 2.6 |
| Vκ3-20Jκ1 | 4.2 | 88 | 6 |

**Common Light Chain Expression.** Expression of each common light chain (Vκ1-39Jκ5 and Vκ3-20Jκ1) was analyzed in heterozygous and homozygous mice using a quantitative PCR assay (e.g. TAQMAN^{™}).

Briefly, CD19⁺ B cells were purified from the spleens of wild type, mice homozygous for a replacement of the mouse heavy chain and κ light chain variable region loci with corresponding human heavy chain and κ light chain variable region loci (Hκ), as well as mice homozygous and heterozygous for each rearranged human light chain region (Vκ1-39Jκ5 or Vκ3-20Jκ1) using mouse CD19 Microbeads (Miltenyi Biotec) according to manufacturer's specifications. Total RNA was purified from CD19⁺ B cells using RNeasy Mini kit (Qiagen) according to manufacturer's specifications and genomic RNA was removed using an RNase-free DNase on-column treatment (Qiagen). 200 ng mRNA was reverse-transcribed into cDNA using the First Stand cDNA Synthesis kit (Invitrogen) and the resulting cDNA was amplified with the Taqman Universal PCR Master Mix (Applied Biosystems). All reactions were performed using the ABI 7900 Sequence Detection System (Applied Biosystems) using primers and Taqman MGB probes spanning (1) the Vκ-Jκ junction for both common light chains, (2) the Vκ gene alone (*i.e.* Vκ1-39 and Vκ3-20), and (3) the mouse Cκ region. Table 4 sets forth the sequences of the primers and probes employed for this assay. Relative expression was normalized to expression of the mouse Cκ region. Results are shown in FIG. 5A, 5B and 5C.

**TABLE 4**

| Region | Primer/Probe Description (5'-3') | SEQ ID NOs: |
|---|---|---|
| Vκ1-39Jκ5 Junction | (Sense) AGCAGTCTGC AACCTGAAGA TTT | 19 |
| | (Anti-sense) GTTTAATCTC CAGTCGTGTC CCTT | 20 |
| | (Probe) CCTCCGATCA CCTTC | 21 |
| Vκ1-39 | (Sense) AAACCAGGGA AAGCCCCTAA | 22 |
| | (Anti-sense) ATGGGACCCC ACTTTGCA | 23 |
| | (Probe) CTCCTGATCT ATGCTGCAT | 24 |
| Vκ3-20Jκ1 Junction | (Sense) CAGCAGACTG GAGCCTGAAG A | 25 |
| | (Anti-sense) TGATTTCCAC CTTGGTCCCT T | 26 |
| | (Probe) TAGCTCACCT TGGACGTT | 27 |
| Vκ3-20 | (Sense) CTCCTCATCT ATGGTGCATC CA | 28 |
| | (Anti-sense) GACCCACTGC CACTGAACCT | 29 |
| | (Probe) CCACTGGCAT CCC | 30 |
| Mouse Cκ | (Sense) TGAGCAGCAC CCTCACGTT | 31 |
| | (Anti-sense) GTGGCCTCAC AGGTATAGCT GTT | 32 |
| | (Probe) ACCAAGGACG AGTATGAA | 33 |

**Antigen Specific Common Light Chain Antibodies.** Common light chain mice bearing either a Vκ1-39Jκ5 or Vκ3-20Jκ1 common light chain at the endogenous mouse κ light chain locus were immunized with β-galactosidase and antibody titer was measured.

Briefly, β-galactosidase (Sigma) was emulsified in titermax adjuvant (Sigma), as per manufacturer's directions. Wild type (n=7), Vκ1-39Jκ5 common light chain homozygotes (n=2) and Vκ3-20Jκ1 common light chain homozygotes (n=5) were immunized by subcutaneous injection with 100 µg β-galactosidase/Titermax. Mice were boosted by subcutaneous injection two times, 3 weeks apart, with 50 µg β-galactosidase/Titermax. After the second boost, blood was collected from anaesthetized mice using a retro-orbital bleed into serum separator tubes (BD Biosciences) as per manufacturer's directions. To measure anti-β-galactosidase IgM or IgG antibodies, ELISA plates (Nunc) were coated with 1 µg/mL β-galactosidase overnight at 4°C. Excess antigen was washed off before blocking with PBS with 1% BSA for one hour at room temperature. Serial dilutions of serum were added to the plates and incubated for one hour at room temperature before washing. Plates were then incubated with HRP conjugated anti-IgM (Southern Biotech) or anti-IgG (Southern Biotech) for one hour at room temperature. Following another wash, plates were developed with TMB substrate (BD Biosciences). Reactions were stopped with 1N sulfuric acid and OD₄₅₀ was read using a Victor X5 Plate Reader (Perkin Elmer). Data was analyzed with GraphPad Prism and signal was calculated as the dilution of serum that is two times above background. Results are shown in FIG. 6A and 6B.

As shown in this Example, the ratio of κ/λ B cells in both the splenic and peripheral compartments of Vκ1-39Jκ5 and Vκ3-20Jκ1 common light chain mice demonstrated a near wild type pattern (Table 3 and FIG. 4). VpreBJλ5 common light chain mice, however, demonstrated fewer peripheral B cells, of which about 1-2% express the engineered human light chain region (data not shown). The expression levels of the Vκ1-39Jκ5 and Vκ3-20Jκ1 rearranged human light chain regions from the endogenous κ light chain locus were elevated in comparison to an endogenous κ light chain locus containing a complete replacement of mouse Vκ and Jκ gene segments with human Vκ and Jκ gene segments (FIG. 5A, 5B and 5C). The expression levels of the VpreBJλ5 rearranged human light chain region demonstrated similar high expression from the endogenous κ light chain locus in both heterozygous and homozygous mice (data not shown). This demonstrates that in direct competition with the mouse λ, κ, or both endogenous light chain loci, a single rearranged human V_{L}/J_{L} sequence can yield better than wild type level expression from the endogenous κ light chain locus and give rise to normal splenic and blood B cell frequency. Further, the presence of an engineered κ light chain locus having either a human Vκ1-39Jκ5 or human Vκ3-20Jκ1 sequence was well tolerated by the mice and appear to function in wild type fashion by representing a substantial portion of the light chain repertoire in the humoral component of the immune response (FIG 6A and 6B).

### Example 4. Breeding of Mice Expressing a Single Rearranged Human Germline Light Chain

This Example describes several other genetically modified mouse strains that can be bred to any one of the common light chain mice described herein to create multiple genetically modified mouse strains harboring multiple genetically modified immunoglobulin loci.

Endogenous Igλ Knockout (KO). To optimize the usage of the engineered light chain locus, mice bearing one of the rearranged human germline light chain regions are bred to another mouse containing a deletion in the endogenous λ light chain locus. In this manner, the progeny obtained will express, as their only light chain, the rearranged human germline light chain region as described in Example 2. Breeding is performed by standard techniques recognized in the art and, alternatively, by a commercial breeder (e.g., The Jackson Laboratory). Mouse strains bearing an engineered light chain locus and a deletion of the endogenous λ light chain locus are screened for presence of the unique light chain region and absence of endogenous mouse λ light chains.

**Humanized Endogenous Heavy Chain Locus.** Mice bearing an engineered human germline light chain locus are bred with mice that contain a replacement of the endogenous mouse heavy chain variable gene locus with the human heavy chain variable gene locus (see US 6,596,541; the VELOCIMMUNE^{®} mouse, Regeneron Pharmaceuticals, Inc.). The VELOCIMMUNE^{®} mouse comprises a genome comprising human heavy chain variable regions operably linked to endogenous mouse constant region loci such that the mouse produces antibodies comprising a human heavy chain variable region and a mouse heavy chain constant region in response to antigenic stimulation. The DNA encoding the variable regions of the heavy chains of the antibodies is isolated and operably linked to DNA encoding the human heavy chain constant regions. The DNA is then expressed in a cell capable of expressing the fully human heavy chain of the antibody.

Mice bearing a replacement of the endogenous mouse V_{H} locus with the human VH locus and a single rearranged human germline V_{L} region at the endogenous κ light chain locus are obtained. Reverse chimeric antibodies containing somatically mutated heavy chains (human V_{H} and mouse C_{H}) with a single human light chain (human V_{L} and mouse C_{L}) are obtained upon immunization with an antigen of interest. V_{H} and V_{L} nucleotide sequences of B cells expressing the antibodies are identified and fully human antibodies are made by fusion the V_{H} and V_{L} nucleotide sequences to human C_{H} and C_{L} nucleotide sequences in a suitable expression system.

### Example 5. Generation of Antibodies from Mice Expressing Human Heavy Chains and a Rearranged Human Germline Light Chain Region

After breeding mice that contain the engineered human light chain region to various desired strains containing modifications and deletions of other endogenous Ig loci (as described in Example 4), selected mice can be immunized with an antigen of interest.

Generally, a VELOCIMMUNE^{®} mouse containing one of the single rearranged human germline light chain regions is challenged with an antigen, and lymphatic cells (such as B-cells) are recovered from serum of the animals. The lymphatic cells are fused with a myeloma cell line to prepare immortal hybridoma cell lines, and such hybridoma cell lines are screened and selected to identify hybridoma cell lines that produce antibodies containing human heavy chain variables and a rearranged human germline light chains which are specific to the antigen used for immunization. DNA encoding the variable regions of the heavy chains and the light chain are isolated and linked to desirable isotypic constant regions of the heavy chain and light chain. Due to the presence of the endogenous mouse sequences and any additional cis-acting elements present in the endogenous locus, the single light chain of each antibody may be somatically mutated. This adds additional diversity to the antigen-specific repertoire comprising a single light chain and diverse heavy chain sequences. The resulting cloned antibody sequences are subsequently expressed in a cell, such as a CHO cell. Alternatively, DNA encoding the antigen-specific chimeric antibodies or the variable domains of the light and heavy chains is identified directly from antigen-specific lymphocytes.

Initially, high affinity chimeric antibodies are isolated having a human variable region and a mouse constant region. As described above, the antibodies are characterized and selected for desirable characteristics, including affinity, selectivity, epitope, *etc.* The mouse constant regions are replaced with a desired human constant region to generate the fully human antibody containing a somatically mutated human heavy chain and a single light chain derived from a rearranged human germline light chain region of the invention. Suitable human constant regions include, for example wild type or modified IgG1 or IgG4.

Separate cohorts of VELOCIMMUNE^{®} mice containing a replacement of the endogenous mouse heavy chain locus with human V_{H}, D_{H}, and J_{H} gene segments and a replacement of the endogenous mouse κ light chain locus with either the engineered germline V 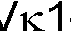 1-39J 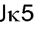 5 human light chain region or the engineered germline Vκ3-20Jκ1 human light chain region (described above) were immunized with a human cell-surface receptor protein (Antigen E). Antigen E is administered directly onto the hind footpad of mice with six consecutive injections every 3-4 days. Two to three micrograms of Antigen E are mixed with 10 µg of CpG oligonucleotide (Cat # tlrl-modn - ODN1826 oligonucleotide; InVivogen, San Diego, CA) and 25 µg of Adju-Phos (Aluminum phosphate gel adjuvant, Cat# H-71639-250; Brenntag Biosector, Frederikssund, Denmark) prior to injection. A total of six injections are given prior to the final antigen recall, which is given 3-5 days prior to sacrifice. Bleeds after the 4th and 6th injection are collected and the antibody immune response is monitored by a standard antigen-specific immunoassay.

When a desired immune response is achieved splenocytes are harvested and fused with mouse myeloma cells to preserve their viability and form hybridoma cell lines. The hybridoma cell lines are screened and selected to identify cell lines that produce Antigen E-specific common light chain antibodies. Using this technique several anti-Antigen E-specific common light chain antibodies *(i.e.,* antibodies possessing human heavy chain variable domains, the same human light chain variable domain, and mouse constant domains) are obtained.

Alternatively, anti-Antigen E common light chain antibodies are isolated directly from antigen-positive B cells without fusion to myeloma cells, as described in U.S. 2007/0280945A1. Using this method, several fully human anti-Antigen E common light chain antibodies *(i.e.,* antibodies possessing human heavy chain variable domains, either an engineered human Vκ1-39Jκ5 light chain or an engineered human Vκ3-20Jκ1 light chain region, and human constant domains) were obtained.

The biological properties of the exemplary anti-Antigen E common light chain antibodies generated in accordance with the methods of this Example are described in detail in the sections set forth below.

### Example 6. Heavy Chain Gene Segment Usage in Antigen-Specific Common Light Chain Antibodies

To analyze the structure of the human anti-Antigen E common light chain antibodies produced, nucleic acids encoding heavy chain antibody variable regions were cloned and sequenced. From the nucleic acid sequences and predicted amino acid sequences of the antibodies, gene usage was identified for the heavy chain variable region (HCVR) of selected common light chain antibodies obtained from immunized VELOCIMMUNE^{®} mice containing either the engineered human Vκ1-39Jκ5 light chain or engineered human Vκ3-20Jκ1 light chain region. Results are shown in Tables 5 and 6, which demonstrate that mice according to the invention generate antigen-specific common light chain antibodies from a variety of human heavy chain gene segments, due to a variety of rearrangements, when employing either a mouse that expresses a light chain from only a human Vκ1-39- or a human Vκ3-20-derived light chain. Human V_{H} gene segments of the 2, 3, 4, and 5 families rearranged with a variety of human D_{H} segments and human J_{H} segments to yield antigen-specific antibodies.

**TABLE 5**

| Vκ1-39Jκ5 Common Light Chain Antibodies | | | | | | | | |
|---|---|---|---|---|---|---|---|---|
| Antibody | HCVR | | | | Antibody | HCVR | | |
| | V_{H} | D_{H} | J_{H} | | | V_{H} | D_{H} | J_{H} |
| 2952 | 2-5 | 6-6 | 1 | | 6030 | 3-30 | 6-6 | 5 |
| 5978 | 2-5 | 6-6 | 1 | | 6032 | 3-30 | 6-6 | 5 |
| 5981 | 2-5 | 3-22 | 1 | | 2985 | 3-30 | 6-13 | 4 |
| 6027 | 3-13 | 6-6 | 5 | | 2997 | 3-30 | 6-13 | 4 |
| 3022 | 3-23 | 3-10 | 4 | | 3011 | 3-30 | 6-13 | 4 |
| 3028 | 3-23 | 3-3 | 4 | | 3047 | 3-30 | 6-13 | 4 |
| 5999 | 3-23 | 6-6 | 4 | | 5982 | 3-30 | 6-13 | 4 |
| 6009 | 3-23 | 2-8 | 4 | | 6002 | 3-30 | 6-13 | 4 |
| 6011 | 3-23 | 7-27 | 4 | | 6003 | 3-30 | 6-13 | 4 |
| 5980 | 3-30 | 1-1 | 4 | | 6012 | 3-30 | 6-13 | 4 |
| 3014 | 3-30 | 1-7 | 4 | | 6013 | 3-30 | 6-13 | 4 |
| 3015 | 3-30 | 1-7 | 4 | | 6014 | 3-30 | 6-13 | 4 |
| 3023 | 3-30 | 1-7 | 4 | | 6015 | 3-30 | 6-13 | 4 |
| 3024 | 3-30 | 1-7 | 4 | | 6016 | 3-30 | 6-13 | 4 |
| 3032 | 3-30 | 1-7 | 4 | | 6017 | 3-30 | 6-13 | 4 |
| 6024 | 3-30 | 1-7 | 4 | | 6020 | 3-30 | 6-13 | 4 |
| 6025 | 3-30 | 1-7 | 4 | | 6034 | 3-30 | 6-13 | 4 |
| 6031 | 3-30 | 1-7 | 4 | | 2948 | 3-30 | 7-27 | 4 |
| 6007 | 3-30 | 3-3 | 4 | | 2987 | 3-30 | 7-27 | 4 |
| 2982 | 3-30 | 3-22 | 5 | | 2996 | 3-30 | 7-27 | 4 |
| 6001 | 3-30 | 3-22 | 5 | | 3005 | 3-30 | 7-27 | 4 |
| 6005 | 3-30 | 3-22 | 5 | | 3012 | 3-30 | 7-27 | 4 |
| 6035 | 3-30 | 5-5 | 2 | | 3020 | 3-30 | 7-27 | 4 |
| 3013 | 3-30 | 5-12 | 4 | | 3021 | 3-30 | 7-27 | 4 |
| 3042 | 3-30 | 5-12 | 4 | | 3025 | 3-30 | 7-27 | 4 |
| 2955 | 3-30 | 6-6 | 1 | | 3030 | 3-30 | 7-27 | 4 |
| 3043 | 3-30 | 6-6 | 3 | | 3036 | 3-30 | 7-27 | 4 |
| 3018 | 3-30 | 6-6 | 4 | | 5997 | 3-30 | 7-27 | 4 |
| 2949 | 3-30 | 6-6 | 5 | | 6033 | 3-30 | 7-27 | 4 |
| 2950 | 3-30 | 6-6 | 5 | | 3004 | 3-30 | 7-27 | 5 |
| 2954 | 3-30 | 6-6 | 5 | | 6028 | 3-30 | 7-27 | 6 |
| 2978 | 3-30 | 6-6 | 5 | | 3010 | 4-59 | 3-16 | 3 |
| 3016 | 3-30 | 6-6 | 5 | | 3019 | 4-59 | 3-16 | 3 |
| 3017 | 3-30 | 6-6 | 5 | | 6018 | 4-59 | 3-16 | 3 |
| 3033 | 3-30 | 6-6 | 5 | | 6026 | 4-59 | 3-16 | 3 |
| 3041 | 3-30 | 6-6 | 5 | | 6029 | 4-59 | 3-16 | 3 |
| 5979 | 3-30 | 6-6 | 5 | | 6036 | 4-59 | 3-16 | 3 |
| 5998 | 3-30 | 6-6 | 5 | | 6037 | 4-59 | 3-16 | 3 |
| 6004 | 3-30 | 6-6 | 5 | | 2964 | 4-59 | 3-22 | 3 |
| 6010 | 3-30 | 6-6 | 5 | | 3027 | 4-59 | 3-16 | 4 |
| 6019 | 3-30 | 6-6 | 5 | | 3046 | 5-51 | 5-5 | 3 |
| 6021 | 3-30 | 6-6 | 5 | | 6000 | 1-69 | 6-13 | 4 |
| 6022 | 3-30 | 6-6 | 5 | | 6006 | 1-69 | 6-6 | 5 |
| 6023 | 3-30 | 6-6 | 5 | | 6008 | 1-69 | 6-13 | 4 |

**TABLE 6**

| Vκ3-20Jκ1 Common Light Chain Antibodies | | | | | | | | |
|---|---|---|---|---|---|---|---|---|
| Antibody | HCVR | | | | Antibody | HCVR | | |
| | V_{H} | D_{H} | J_{H} | | | V_{H} | D_{H} | J_{H} |
| 5989 | 3-30 | 3-3 | 3 | | 5992 | 4-39 | 1-26 | 3 |
| 5994 | 3-33 | 1-7 | 4 | | 2975 | 5-51 | 6-13 | 5 |
| 5985 | 3-33 | 2-15 | 4 | | 2972 | 5-51 | 3-16 | 6 |
| 5987 | 3-33 | 2-15 | 4 | | 5986 | 5-51 | 3-16 | 6 |
| 5995 | 3-33 | 2-15 | 4 | | 5993 | 5-51 | 3-16 | 6 |
| 2968 | 4-39 | 1-26 | 3 | | 5996 | 5-51 | 3-16 | 6 |
| 5988 | 4-39 | 1-26 | 3 | | 5984 | 3-53 | 1-1 | 4 |
| 5990 | 4-39 | 1-26 | 3 | | | | | |

### Example 7. Determination of Blocking Ability of Antigen-Specific Common Light Chain Antibodies by LUMINEX^{™} Assay

Ninety-eight human common light chain antibodies raised against Antigen E were tested for their ability to block binding of Antigen E's natural ligand (Ligand Y) to Antigen E in a bead-based assay.

The extracellular domain (ECD) of Antigen E was conjugated to two myc epitope tags and a 6X histidine tag (Antigen E-mmH) and amine-coupled to carboxylated microspheres at a concentration of 20 µg/mL in MES buffer. The mixture was incubated for two hours at room temperature followed by bead deactivation with 1M Tris pH 8.0 followed by washing in PBS with 0.05% (v/v) Tween-20. The beads were then blocked with PBS (Irvine Scientific, Santa Ana, CA) containing 2% (w/v) BSA (Sigma-Aldrich Corp., St. Louis, MO). In a 96-well filter plate, supernatants containing Antigen E-specific common light chain antibodies were diluted 1:15 in buffer. A negative control containing a mock supernatant with the same media components as for the antibody supernatant was prepared. Antigen E-labeled beads were added to the supernatants and incubated overnight at 4°C. Biotinylated-Ligand Y protein was added to a final concentration of 0.06 nM and incubated for two hours at room temperature. Detection of biotinylated-Ligand Y bound to Antigen E-myc-myc-6His labeled beads was determined with R-Phycoerythrin conjugated to Streptavidin (Moss Inc, Pasadena, MD) followed by measurement in a LUMINEX^{™} flow cytometry-based analyzer. Background Mean Fluorescence Intensity (MFI) of a sample without Ligand Y was subtracted from all samples. Percent blocking was calculated by division of the background-subtracted MFI of each sample by the adjusted negative control value, multiplying by 100 and subtracting the resulting value from 100.

In a similar experiment, the same 98 human common light chain antibodies raised against Antigen E were tested for their ability to block binding of Antigen E to Ligand Y-labeled beads.

Briefly, Ligand Y was amine-coupled to carboxylated microspheres at a concentration of 20 µg/mL diluted in MES buffer. The mixture and incubated two hours at room temperature followed by deactivation of beads with 1M Tris pH 8 then washing in PBS with 0.05% (v/v) Tween-20. The beads were then blocked with PBS (Irvine Scientific, Santa Ana, CA) containing 2% (w/v) BSA (Sigma-Aldrich Corp., St. Louis, MO). In a 96-well filter plate, supernatants containing Antigen E-specific common light chain antibodies were diluted 1:15 in buffer. A negative control containing a mock supernatant with the same media components as for the antibody supernatant was prepared. A biotinylated-Antigen E-mmH was added to a final concentration of 0.42 nM and incubated overnight at 4°C. Ligand Y-labeled beads were then added to the antibody/Antigen E mixture and incubated for two hours at room temperature. Detection of biotinylated-Antigen E-mmH bound to Ligand Y-beads was determined with R-Phycoerythrin conjugated to Streptavidin (Moss Inc, Pasadena, MD) followed by measurement in a LUMINEX^{™} flow cytometry-based analyzer. Background Mean Fluorescence Intensity (MFI) of a sample without Antigen E was subtracted from all samples. Percent blocking was calculated by division of the background-subtracted MFI of each sample by the adjusted negative control value, multiplying by 100 and subtracting the resulting value from 100.

Tables 7 and 8 show the percent blocking for all 98 anti-Antigen E common light chain antibodies tested in both LUMINEX^{™} assays. ND: not determined under current experimental conditions.

**TABLE 7**

| V 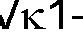 1-39J 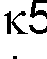 5 | | |
|---|---|---|
| Common Light Chain Antibodies | | |
| Antibody | % Blocking of Antigen E-Labeled Beads | % Blocking of Antigen E In Solution |
| 2948 | 81.1 | 47.8 |
| 2948G | 38.6 | ND |
| 2949 | 97.6 | 78.8 |
| 2949G | 97.1 | 73.7 |
| 2950 | 96.2 | 81.9 |
| 2950G | 89.8 | 31.4 |
| 2952 | 96.1 | 74.3 |
| 2952G | 93.5 | 39.9 |
| 2954 | 93.7 | 70.1 |
| 2954G | 91.7 | 30.1 |
| 2955 | 75.8 | 30.0 |
| 2955G | 71.8 | ND |
| 2964 | 92.1 | 31.4 |
| 2964G | 94.6 | 43.0 |
| 2978 | 98.0 | 95.1 |
| 2978G | 13.9 | 94.1 |
| 2982 | 92.8 | 78.5 |
| 2982G | 41.9 | 52.4 |
| 2985 | 39.5 | 31.2 |
| 2985G | 2.0 | 5.0 |
| 2987 | 81.7 | 67.8 |
| 2987G | 26.6 | 29.3 |
| 2996 | 87.3 | 55.3 |
| 2996G | 95.9 | 38.4 |
| 2997 | 93.4 | 70.6 |
| 2997G | 9.7 | 7.5 |
| 3004 | 79.0 | 48.4 |
| 3004G | 60.3 | 40.7 |
| 3005 | 97.4 | 93.5 |
| 3005G | 77.5 | 75.6 |
| 3010 | 98.0 | 82.6 |
| 3010G | 97.9 | 81.0 |
| 3011 | 87.4 | 42.8 |
| 3011G | 83.5 | 41.7 |
| 3012 | 91.0 | 60.8 |
| 3012G | 52.4 | 16.8 |
| 3013 | 80.3 | 65.8 |
| 3013G | 17.5 | 15.4 |
| 3014 | 63.4 | 20.7 |
| 3014G | 74.4 | 28.5 |
| 3015 | 89.1 | 55.7 |
| 3015G | 58.8 | 17.3 |
| 3016 | 97.1 | 81.6 |
| 3016G | 93.1 | 66.4 |
| 3017 | 94.8 | 70.2 |
| 3017G | 87.9 | 40.8 |
| 3018 | 85.4 | 54.0 |
| 3018G | 26.1 | 12.7 |
| 3019 | 99.3 | 92.4 |
| 3019G | 99.3 | 88.1 |
| 3020 | 96.7 | 90.3 |
| 3020G | 85.2 | 41.5 |
| 3021 | 74.5 | 26.1 |
| 3021G | 81.1 | 27.4 |
| 3022 | 65.2 | 17.6 |
| 3022G | 67.2 | 9.1 |
| 3023 | 71.4 | 28.5 |
| 3023G | 73.8 | 29.7 |
| 3024 | 73.9 | 32.6 |
| 3024G | 89.0 | 10.0 |
| 3025 | 70.7 | 15.6 |
| 3025G | 76.7 | 24.3 |
| 3027 | 96.2 | 61.6 |
| 3027G | 98.6 | 75.3 |
| 3028 | 92.4 | 29.0 |
| 3028G | 87.3 | 28.8 |
| 3030 | 6.0 | 10.6 |
| 3030G | 41.3 | 14.2 |
| 3032 | 76.5 | 31.4 |
| 3032G | 17.7 | 11.0 |
| 3033 | 98.2 | 86.1 |
| 3033G | 93.6 | 64.0 |
| 3036 | 74.7 | 32.7 |
| 3036G | 90.1 | 51.2 |
| 3041 | 95.3 | 75.9 |
| 3041G | 92.4 | 51.6 |
| 3042 | 88.1 | 73.3 |
| 3042G | 60.9 | 25.2 |
| 3043 | 90.8 | 65.8 |
| 3043G | 92.8 | 60.3 |

**TABLE 8**

| Vκ3-20Jκ1 | | |
|---|---|---|
| Common Light Chain Antibodies | | |
| Antibody | % Blocking of Antigen E-Labeled Beads | % Blocking of Antigen E In Solution |
| 2968 | 97.1 | 73.3 |
| 2968G | 67.1 | 14.6 |
| 2969 | 51.7 | 20.3 |
| 2969G | 37.2 | 16.5 |
| 2970 | 92.2 | 34.2 |
| 2970G | 92.7 | 27.2 |
| 2971 | 23.4 | 11.6 |
| 2971G | 18.8 | 18.9 |
| 2972 | 67.1 | 38.8 |
| 2972G | 64.5 | 39.2 |
| 2973 | 77.7 | 27.0 |
| 2973G | 51.1 | 20.7 |
| 2974 | 57.8 | 12.4 |
| 2974G | 69.9 | 17.6 |
| 2975 | 49.4 | 18.2 |
| 2975G | 32.0 | 19.5 |
| 2976 | 1.0 | 1.0 |
| 2976G | 50.4 | 20.4 |

In the first LUMINEX^{™} experiment described above, 80 common light chain antibodies containing the Vκ1-39Jκ5 engineered light chain were tested for their ability to block Ligand Y binding to Antigen E-labeled beads. Of these 80 common light chain antibodies, 68 demonstrated >50% blocking, while 12 demonstrated <50% blocking (6 at 25-50% blocking and 6 at <25% blocking). For the 18 common light chain antibodies containing the Vκ3-20Jκ1 engineered light chain, 12 demonstrated >50% blocking, while 6 demonstrated <50% blocking (3 at 25-50% blocking and 3 at <25% blocking) of Ligand Y binding to Antigen E-labeled beads.

In the second LUMINEX^{™} experiment described above, the same 80 common light chain antibodies containing the Vκ1-39Jκ5 engineered light chain were tested for their ability to block binding of Antigen E to Ligand Y-labeled beads. Of these 80 common light chain antibodies, 36 demonstrated >50% blocking, while 44 demonstrated <50% blocking (27 at 25-50% blocking and 17 at <25% blocking). For the 18 common light chain antibodies containing the Vκ3-20Jκ1 engineered light chain, 1 demonstrated >50% blocking, while 17 demonstrated <50% blocking (5 at 25-50% blocking and 12 at <25% blocking) of Antigen E binding to Ligand Y-labeled beads.

The data of Tables 7 and 8 establish that the rearrangements described in Tables 5 and 6 generated anti-Antigen E-specific common light chain antibodies that blocked binding of Ligand Y to its cognate receptor Antigen E with varying degrees of efficacy, which is consistent with the anti-Antigen E common light chain antibodies of Tables 5 and 6 comprising antibodies with overlapping and non-overlapping epitope specificity with respect to Antigen E.

### Example 8. Determination of Blocking Ability of Antigen-Specific Common Light Chain Antibodies by ELISA

Human common light chain antibodies raised against Antigen E were tested for their ability to block Antigen E binding to a Ligand Y-coated surface in an ELISA assay.

Ligand Y was coated onto 96-well plates at a concentration of 2 µg/mL diluted in PBS and incubated overnight followed by washing four times in PBS with 0.05% Tween-20. The plate was then blocked with PBS (Irvine Scientific, Santa Ana, CA) containing 0.5% (w/v) BSA (Sigma-Aldrich Corp., St. Louis, MO) for one hour at room temperature. In a separate plate, supernatants containing anti-Antigen E common light chain antibodies were diluted 1:10 in buffer. A mock supernatant with the same components of the antibodies was used as a negative control. Antigen E-mmH (described above) was added to a final concentration of 0.150 nM and incubated for one hour at room temperature. The antibody/Antigen E-mmH mixture was then added to the plate containing Ligand Y and incubated for one hour at room temperature. Detection of Antigen E-mmH bound to Ligand Y was determined with Horse-Radish Peroxidase (HRP) conjugated to anti-Penta-His antibody (Qiagen, Valencia, CA) and developed by standard colorimetric response using tetramethylbenzidine (TMB) substrate (BD Biosciences, San Jose, CA) neutralized by sulfuric acid. Absorbance was read at OD450 for 0.1 sec. Background absorbance of a sample without Antigen E was subtracted from all samples. Percent blocking was calculated by division of the background-subtracted MFI of each sample by the adjusted negative control value, multiplying by 100 and subtracting the resulting value from 100.

Tables 9 and 10 show the percent blocking for all 98 anti-Antigen E common light chain antibodies tested in the ELISA assay. ND: not determined under current experimental conditions.

**TABLE 9**

| Vκ1-39Jκ5 | | | | |
|---|---|---|---|---|
| Common Light Chain Antibodies | | | | |
| Antibody | % Blocking of Antigen E In Solution | | Antibody | % Blocking of Antigen E In Solution |
| 2948 | 21.8 | | 3015 | 23.7 |
| 2948G | 22.9 | | 3015G | 10.2 |
| 2949 | 79.5 | | 3016 | 78.1 |
| 2949G | 71.5 | | 3016G | 37.4 |
| 2950 | 80.4 | | 3017 | 61.6 |
| 2950G | 30.9 | | 3017G | 25.2 |
| 2952 | 66.9 | | 3018 | 40.6 |
| 2952G | 47.3 | | 3018G | 14.5 |
| 2954 | 55.9 | | 3019 | 94.6 |
| 2954G | 44.7 | | 3019G | 92.3 |
| 2955 | 12.1 | | 3020 | 80.8 |
| 2955G | 25.6 | | 3020G | ND |
| 2964 | 34.8 | | 3021 | 7.6 |
| 2964G | 47.7 | | 3021G | 20.7 |
| 2978 | 90.0 | | 3022 | 2.4 |
| 2978G | 90.2 | | 3022G | 15.0 |
| 2982 | 59.0 | | 3023 | 9.1 |
| 2982G | 20.4 | | 3023G | 19.2 |
| 2985 | 10.5 | | 3024 | 7.5 |
| 2985G | ND | | 3024G | 15.2 |
| 2987 | 31.4 | | 3025 | ND |
| 2987G | ND | | 3025G | 13.9 |
| 2996 | 29.3 | | 3027 | 61.4 |
| 2996G | ND | | 3027G | 82.7 |
| 2997 | 48.7 | | 3028 | 40.3 |
| 2997G | ND | | 3028G | 12.3 |
| 3004 | 16.7 | | 3030 | ND |
| 3004G | 3.5 | | 3030G | 9.5 |
| 3005 | 87.2 | | 3032 | ND |
| 3005G | 54.3 | | 3032G | 13.1 |
| 3010 | 74.5 | | 3033 | 77.1 |
| 3010G | 84.6 | | 3033G | 32.9 |
| 3011 | 19.4 | | 3036 | 17.6 |
| 3011G | ND | | 3036G | 24.6 |
| 3012 | 45.0 | | 3041 | 59.3 |
| 3012G | 12.6 | | 3041G | 30.7 |
| 3013 | 39.0 | | 3042 | 39.9 |
| 3013G | 9.6 | | 3042G | 16.1 |
| 3014 | 5.2 | | 3043 | 57.4 |
| 3014G | 17.1 | | 3043G | 46.1 |

**TABLE 10**

| Vκ3-20Jκ1 | | | | |
|---|---|---|---|---|
| Common Light Chain Antibodies | | | | |
| Antibody | % Blocking of Antigen E In Solution | | Antibody | % Blocking of Antigen E In Solution |
| 2968 | 68.9 | | 2972G | 35.7 |
| 2968G | 15.2 | | 2973 | 20.7 |
| 2969 | 10.1 | | 2973G | 23.1 |
| 2969G | 23.6 | | 2974 | ND |
| 2970 | 34.3 | | 2974G | 22.0 |
| 2970G | 41.3 | | 2975 | 8.7 |
| 2971 | 6.3 | | 2975G | 19.2 |
| 2971G | 27.1 | | 2976 | 4.6 |
| 2972 | 9.6 | | 2976G | 26.7 |

As described in this Example, of the 80 common light chain antibodies containing the Vκ1-39Jκ5 engineered light chain tested for their ability to block Antigen E binding to a Ligand Y-coated surface, 22 demonstrated >50% blocking, while 58 demonstrated <50% blocking (20 at 25-50% blocking and 38 at <25% blocking). For the 18 common light chain antibodies containing the Vκ3-20Jκ1 engineered light chain, one demonstrated >50% blocking, while 17 demonstrated <50% blocking (5 at 25-50% blocking and 12 at <25% blocking) of Antigen E binding to a Ligand Y-coated surface.

These results are also consistent with the Antigen E-specific common light chain antibody pool comprising antibodies with overlapping and non-overlapping epitope specificity with respect to Antigen E.

### Example 9. BIACORE^{™} Affinity Determination for Antigen-Specific Common Light Chain Antibodies

Equilibrium dissociation constants (K_{D}) for selected antibody supernatants were determined by SPR (Surface Plasmon Resonance) using a BIACORE^{™} T100 instrument (GE Healthcare). All data was obtained using HBS-EP (10mM Hepes, 150mM NaCl, 0.3mM EDTA, 0.05% Surfactant P20, pH 7.4) as both the running and sample buffers, at 25°C. Antibodies were captured from crude supernatant samples on a CM5 sensor chip surface previously derivatized with a high density of anti-human Fc antibodies using standard amine coupling chemistry. During the capture step, supernatants were injected across the anti-human Fc surface at a flow rate of 3 µL/min, for a total of 3 minutes. The capture step was followed by an injection of either running buffer or analyte at a concentration of 100 nM for 2 minutes at a flow rate of 35 µL/min. Dissociation of antigen from the captured antibody was monitored for 6 minutes. The captured antibody was removed by a brief injection of 10 mM glycine, pH 1.5. All sensorgrams were double referenced by subtracting sensorgrams from buffer injections from the analyte sensorgrams, thereby removing artifacts caused by dissociation of the antibody from the capture surface. Binding data for each antibody was fit to a 1:1 binding model with mass transport using BIAcore T100 Evaluation software v2.1. Results are shown in Tables 11 and 12.

**TABLE 11**

| Vκ1-39Jκ5 Common Light Chain Antibodies | | | | | | |
|---|---|---|---|---|---|---|
| Antibody | 100 nM Antigen E | | | Antibody | 100 nM Antigen E | |
| | K_{D} (nM) | T_{1/2} (min) | | | K_{D} (nM) | T_{1/2} (min) |
| 2948 | 8.83 | 28 | | 3015 | 29.1 | 11 |
| 2948G | 95.0 | 1 | | 3015G | 65.9 | 0 |
| 2949 | 3.57 | 18 | | 3016 | 4.99 | 17 |
| 2949G | 6.37 | 9 | | 3016G | 18.9 | 4 |
| 2950 | 4.91 | 17 | | 3017 | 9.83 | 8 |
| 2950G | 13.6 | 5 | | 3017G | 55.4 | 2 |
| 2952 | 6.25 | 7 | | 3018 | 11.3 | 36 |
| 2952G | 7.16 | 4 | | 3018G | 32.5 | 3 |
| 2954 | 2.37 | 24 | | 3019 | 1.54 | 59 |
| 2954G | 5.30 | 9 | | 3019G | 2.29 | 42 |
| 2955 | 14.4 | 6 | | 3020 | 5.41 | 39 |
| 2955G | 12.0 | 4 | | 3020G | 41.9 | 6 |
| 2964 | 14.8 | 6 | | 3021 | 50.1 | 6 |
| 2964G | 13.0 | 9 | | 3021G | 26.8 | 4 |
| 2978 | 1.91 | 49 | | 3022 | 25.7 | 17 |
| 2978G | 1.80 | 58 | | 3022G | 20.8 | 12 |
| 2982 | 6.41 | 19 | | 3023 | 263 | 9 |
| 2982G | 16.3 | 9 | | 3023G | 103 | 5 |
| 2985 | 64.4 | 9 | | 3024 | 58.8 | 7 |
| 2985G | 2.44 | 8 | | 3024G | 7.09 | 10 |
| 2987 | 21.0 | 11 | | 3025 | 35.2 | 6 |
| 2987G | 37.6 | 4 | | 3025G | 42.5 | 8 |
| 2996 | 10.8 | 9 | | 3027 | 7.15 | 6 |
| 2996G | 24.0 | 2 | | 3027G | 4.24 | 18 |
| 2997 | 7.75 | 19 | | 3028 | 6.89 | 37 |
| 2997G | 151 | 1 | | 3028G | 7.23 | 22 |
| 3004 | 46.5 | 14 | | 3030 | 46.2 | 7 |
| 3004G | 1.93 | 91 | | 3030G | 128 | 3 |
| 3005 | 2.35 | 108 | | 3032 | 53.2 | 9 |
| 3005G | 6.96 | 27 | | 3032G | 13.0 | 1 |
| 3010 | 4.13 | 26 | | 3033 | 4.61 | 17 |
| 3010G | 2.10 | 49 | | 3033G | 12.0 | 5 |
| 3011 | 59.1 | 5 | | 3036 | 284 | 12 |
| 3011G | 41.7 | 5 | | 3036G | 18.2 | 10 |
| 3012 | 9.71 | 20 | | 3041 | 6.90 | 12 |
| 3012G | 89.9 | 2 | | 3041G | 22.9 | 2 |
| 3013 | 20.2 | 20 | | 3042 | 9.46 | 34 |
| 3013G | 13.2 | 4 | | 3042G | 85.5 | 3 |
| 3014 | 213 | 4 | | 3043 | 9.26 | 29 |
| 3014G | 36.8 | 3 | | 3043G | 13.1 | 22 |

**TABLE 12**

| Vκ3-20Jκ1 Common Light Chain Antibodies | | | | | | |
|---|---|---|---|---|---|---|
| Antibody | 100 nM Antigen E | | | Antibody | 100 nM Antigen E | |
| | K_{D} (nM) | T_{1/2} (min) | | | K_{D} (nM) | T_{1/2} (min) |
| 2968 | 5.50 | 8 | | 2973 | 5.35 | 39 |
| 2968G | 305 | 0 | | 2973G | 11.0 | 44 |
| 2969 | 34.9 | 2 | | 2974 | 256 | 0 |
| 2969G | 181 | 1 | | 2974G | 138 | 0 |
| 2970G | 12.3 | 3 | | 2975 | 38.0 | 2 |
| 2971G | 32.8 | 22 | | 2975G | 134 | 1 |
| 2972 | 6.02 | 13 | | 2976 | 6.73 | 10 |
| 2972G | 74.6 | 26 | | 2976G | 656 | 8 |

The binding affinities of common light chain antibodies comprising the rearrangements shown in Tables 5 and 6 vary, with nearly all exhibiting a K_{D} in the nanomolar range. The affinity data is consistent with the common light chain antibodies resulting from the combinatorial association of rearranged variable domains described in Tables 5 and 6 being high-affinity, clonally selected, and somatically mutated. Coupled with data previously shown, the common light chain antibodies described in Tables 5 and 6 comprise a collection of diverse, high-affinity antibodies that exhibit specificity for one or more epitopes on Antigen E.

### Example 10. Determination of Binding Specificities of Antigen-Specific Common Light Chain Antibodies by LUMINEX^{™} Assay

Selected anti-Antigen E common light chain antibodies were tested for their ability to bind to the ECD of Antigen E and Antigen E ECD variants, including the cynomolgous monkey ortholog (Mf Antigen E), which differs from the human protein in approximately 10% of its amino acid residues; a deletion mutant of Antigen E lacking the last 10 amino acids from the C-terminal end of the ECD (Antigen E-ΔCT); and two mutants containing an alanine substitution at suspected locations of interaction with Ligand Y (Antigen E-Ala1 and AntigenE-Ala2). The Antigen E proteins were produced in CHO cells and each contained a myc-myc-His C-terminal tag.

For the binding studies, Antigen E ECD protein or variant protein (described above) from 1 mL of culture medium was captured by incubation for 2 hr at room temperature with 1 x 10⁶ microsphere (LUMINEX^{™}) beads covalently coated with an anti-myc monoclonal antibody (MAb 9E10, hybridoma cell line CRL-1729^{™}; ATCC, Manassas, VA). The beads were then washed with PBS before use. Supernatants containing anti-Antigen E common light chain antibodies were diluted 1:4 in buffer and added to 96-well filter plates. A mock supernatant with no antibody was used as negative control. The beads containing the captured Antigen E proteins were then added to the antibody samples (3000 beads per well) and incubated overnight at 4°C. The following day, the sample beads were washed and the bound common light chain antibody was detected with a R-phycoerythrin-conjugated anti-human IgG antibody. The fluorescence intensity of the beads (approximately 100 beads counted for each antibody sample binding to each Antigen E protein) was measured with a LUMINEX^{™} flow cytometry-based analyzer, and the median fluorescence intensity (MFI) for at least 100 counted beads per bead/antibody interaction was recorded. Results are shown in Tables 13 and 14.

**TABLE 13**

| Vκ1-39Jκ5 Common Light Chain Antibodies | | | | | |
|---|---|---|---|---|---|
| Antibody | Mean Fluorescence Intensity (MFI) | | | | |
| | Antigen E-ECD | Antigen E-ΔCT | Antigen E-Ala1 | Antigen E-Ala2 | *Mf* Antigen E |
| 2948 | 1503 | 2746 | 4953 | 3579 | 1648 |
| 2948G | 537 | 662 | 2581 | 2150 | 863 |
| 2949 | 3706 | 4345 | 8169 | 5678 | 5142 |
| 2949G | 3403 | 3318 | 7918 | 5826 | 5514 |
| 2950 | 3296 | 4292 | 7756 | 5171 | 4749 |
| 2950G | 2521 | 2408 | 7532 | 5079 | 3455 |
| 2952 | 3384 | 1619 | 1269 | 168 | 911 |
| 2952G | 3358 | 1001 | 108 | 55 | 244 |
| 2954 | 2808 | 3815 | 7114 | 5039 | 3396 |
| 2954G | 2643 | 2711 | 7620 | 5406 | 3499 |
| 2955 | 1310 | 2472 | 4738 | 3765 | 1637 |
| 2955G | 1324 | 1802 | 4910 | 3755 | 1623 |
| 2964 | 5108 | 1125 | 4185 | 346 | 44 |
| 2964G | 4999 | 729 | 4646 | 534 | 91 |
| 2978 | 6986 | 2800 | 14542 | 10674 | 8049 |
| 2978G | 5464 | 3295 | 11652 | 8026 | 6452 |
| 2982 | 4955 | 2388 | 13200 | 9490 | 6772 |
| 2982G | 3222 | 2013 | 8672 | 6509 | 4949 |
| 2985 | 1358 | 832 | 4986 | 3892 | 1669 |
| 2985G | 43 | 43 | 128 | 244 | 116 |
| 2987 | 3117 | 1674 | 7646 | 5944 | 2546 |
| 2987G | 3068 | 1537 | 9202 | 6004 | 4744 |
| 2996 | 4666 | 1917 | 12875 | 9046 | 6459 |
| 2996G | 2752 | 1736 | 8742 | 6150 | 4873 |
| 2997 | 5164 | 2159 | 12167 | 8361 | 5922 |
| 2997G | 658 | 356 | 3392 | 2325 | 1020 |
| 3004 | 2794 | 1397 | 8542 | 6268 | 3083 |
| 3004G | 2753 | 1508 | 8267 | 5808 | 4345 |
| 3005 | 5683 | 2221 | 12900 | 9864 | 5868 |
| 3005G | 4344 | 2732 | 10669 | 7125 | 5880 |
| 3010 | 4829 | 1617 | 2642 | 3887 | 44 |
| 3010G | 3685 | 1097 | 2540 | 3022 | 51 |
| 3011 | 2859 | 2015 | 7855 | 5513 | 3863 |
| 3011G | 2005 | 1072 | 6194 | 4041 | 3181 |
| 3012 | 3233 | 2221 | 8543 | 5637 | 3307 |
| 3012G | 968 | 378 | 3115 | 2261 | 1198 |
| 3013 | 2343 | 1791 | 6715 | 4810 | 2528 |
| 3013G | 327 | 144 | 1333 | 1225 | 370 |
| 3014 | 1225 | 1089 | 5436 | 3621 | 1718 |
| 3014G | 1585 | 851 | 5178 | 3705 | 2411 |
| 3015 | 3202 | 2068 | 8262 | 5554 | 3796 |
| 3015G | 1243 | 531 | 4246 | 2643 | 1611 |
| 3016 | 4220 | 2543 | 8920 | 5999 | 5666 |
| 3016G | 2519 | 1277 | 6344 | 4288 | 4091 |
| 3017 | 3545 | 2553 | 8700 | 5547 | 5098 |
| 3017G | 1972 | 1081 | 5763 | 3825 | 3038 |
| 3018 | 2339 | 1971 | 6140 | 4515 | 2293 |
| 3018G | 254 | 118 | 978 | 1020 | 345 |
| 3019 | 5235 | 1882 | 7108 | 4249 | 54 |
| 3019G | 4090 | 1270 | 4769 | 3474 | 214 |
| 3020 | 3883 | 3107 | 8591 | 6602 | 4420 |
| 3020G | 2165 | 1209 | 6489 | 4295 | 2912 |
| 3021 | 1961 | 1472 | 6872 | 4641 | 2742 |
| 3021G | 2091 | 1005 | 6430 | 3988 | 2935 |
| 3022 | 2418 | 793 | 7523 | 2679 | 36 |
| 3022G | 2189 | 831 | 6182 | 3051 | 132 |
| 3023 | 1692 | 1411 | 5788 | 3898 | 2054 |
| 3023G | 1770 | 825 | 5702 | 3677 | 2648 |
| 3024 | 1819 | 1467 | 6179 | 4557 | 2450 |
| 3024G | 100 | 87 | 268 | 433 | 131 |
| 3025 | 1853 | 1233 | 6413 | 4337 | 2581 |
| 3025G | 1782 | 791 | 5773 | 3871 | 2717 |
| 3027 | 4131 | 1018 | 582 | 2510 | 22 |
| 3027G | 3492 | 814 | 1933 | 2596 | 42 |
| 3028 | 4361 | 2545 | 9884 | 5639 | 975 |
| 3028G | 2835 | 1398 | 7124 | 3885 | 597 |
| 3030 | 463 | 277 | 1266 | 1130 | 391 |
| 3030G | 943 | 302 | 3420 | 2570 | 1186 |
| 3032 | 2083 | 1496 | 6594 | 4402 | 2405 |
| 3032G | 295 | 106 | 814 | 902 | 292 |
| 3033 | 4409 | 2774 | 8971 | 6331 | 5825 |
| 3033G | 2499 | 1234 | 6745 | 4174 | 4210 |
| 3036 | 1755 | 1362 | 6137 | 4041 | 1987 |
| 3036G | 2313 | 1073 | 6387 | 4243 | 3173 |
| 3041 | 3674 | 2655 | 8629 | 5837 | 4082 |
| 3041G | 2519 | 1265 | 6468 | 4274 | 3320 |
| 3042 | 2653 | 2137 | 7277 | 5124 | 3325 |
| 3042G | 1117 | 463 | 4205 | 2762 | 1519 |
| 3043 | 3036 | 2128 | 7607 | 5532 | 3366 |
| 3043G | 2293 | 1319 | 6573 | 4403 | 3228 |

**TABLE 14**

| Vκ3-20Jκ1 Common Light Chain Antibodies | | | | | |
|---|---|---|---|---|---|
| Antibody | Mean Fluorescence Intensity (MFI) | | | | |
| | Antigen E-ECD | Antigen E-ΔCT | Antigen E-Ala1 | Antigen E-Ala2 | *Mf* Antigen E |
| 2968 | 6559 | 3454 | 14662 | 3388 | 29 |
| 2968G | 2149 | 375 | 9109 | 129 | 22 |
| 2969 | 2014 | 1857 | 7509 | 5671 | 3021 |
| 2969G | 1347 | 610 | 6133 | 4942 | 2513 |
| 2970 | 5518 | 1324 | 14214 | 607 | 32 |
| 2970G | 4683 | 599 | 12321 | 506 | 31 |
| 2971 | 501 | 490 | 2506 | 2017 | 754 |
| 2971G | 578 | 265 | 2457 | 2062 | 724 |
| 2972 | 2164 | 2158 | 8408 | 6409 | 3166 |
| 2972G | 1730 | 992 | 6364 | 4602 | 2146 |
| 2973 | 3527 | 1148 | 3967 | 44 | 84 |
| 2973G | 1294 | 276 | 1603 | 28 | 44 |
| 2974 | 1766 | 722 | 8821 | 241 | 19 |
| 2974G | 2036 | 228 | 8172 | 135 | 26 |
| 2975 | 1990 | 1476 | 8669 | 6134 | 2468 |
| 2975G | 890 | 315 | 4194 | 3987 | 1376 |
| 2976 | 147 | 140 | 996 | 1079 | 181 |
| 2976G | 1365 | 460 | 6024 | 3929 | 1625 |

The anti-Antigen E common light chain antibody supernatants exhibited high specific binding to the beads linked to Antigen E-ECD. For these beads, the negative control mock supernatant resulted in negligible signal (<10 MFI) when combined with the Antigen E-ECD bead sample, whereas the supernatants containing anti-Antigen E common light chain antibodies exhibited strong binding signal (average MFI of 2627 for 98 antibody supernatants; MFI > 500 for 91/98 antibody samples).

As a measure of the ability of the selected anti-Antigen E common light chain antibodies to identify different epitopes on the ECD of Antigen E, the relative binding of the antibodies to the variants were determined. All four Antigen E variants were captured to the anti-myc LUMINEX^{™} beads as described above for the native Antigen E-ECD binding studies, and the relative binding ratios (MFIlᵥₐᵣᵢₐₙₜ/MFI_{Antigen E-ECD}) were determined. For 98 tested common light chain antibody supernatants shown in Tables 12 and 13, the average ratios (MFIᵥₐᵣᵢₐₙₜ/MFI_{antigen E-ECD}) differed for each variant, likely reflecting different capture amounts of proteins on the beads (average ratios of 0.61, 2.9, 2.0, and 1.0 for Antigen E-ΔCT, Antigen E-Ala1, Antigen E-Ala2, and *Mf* Antigen E, respectively). For each protein variant, the binding for a subset of the 98 tested common light chain antibodies showed greatly reduced binding, indicating sensitivity to the mutation that characterized a given variant. For example, 19 of the common light chain antibody samples bound to the Mf Antigen E with MFIᵥₐᵣᵢₐₙₜ/MFI_{Antigen E-ECD} of <8%. Since many in this group include high or moderately high affinity antibodies (5 with K_{D} < 5nM, 15 with K_{D} < 50 nM), it is likely that the lower signal for this group results from sensitivity to the sequence (epitope) differences between native Antigen E-ECD and a given variant rather than from lower affinities.

These data establish that the common light chain antibodies described in Tables 5 and 6 represent a diverse group of Antigen-E-specific common light chain antibodies that specifically recognize more than one epitope on Antigen E.

### Example 11. Light Chain Shuffling in Common Light Chain Antibodies

Heavy chains of selected antigen-specific common light chain antibodies were tested for binding to Antigen E after repairing the heavy chains with either a germline Vκ1-39Jκ5 or a germline Vκ3-20Jκ1 engineered light chain (as described in Example 1).

Briefly, 247 heavy chains of Antigen E-specific common light chain antibodies (Vκ1-39Jκ5 and Vκ3-20Jκ1) were transfected with either a germline Vκ1-39 or a germline Vκ3-20 engineered light chain and rescreened for binding to Antigen E by a LUMINEX^{™} assay (as described in Example 7 and Example 10). Binding to Antigen E was confirmed by BIACORE^{™} (as described in Example 9). The results are shown in Table 15.

As shown in this Example, twenty-eight common light chain antibodies specific for Antigen E were capable of binding to Antigen E when repaired with a germline form of the light chain.

**TABLE 15**

| Original Light Chain | Repaired Light Chain | No. Tested | No. Confirmed Binders |
|---|---|---|---|
| 1-39 | 1-39 | 198 | 23 |
| 3-20 | 3-20 | 49 | 5 |

### Example 12. Heavy Chain Gene Usage and Somatic Hypermutation Frequency in Common Light Chain Antibodies

Heavy and light chain sequences (>6000) of antibodies raised in VELCOlMMUNE^{®} mice (*e.g.,* US 6,596,541 and US 7,105,348) were compiled with heavy and light chain sequences (>600) of common light chain antibodies obtained by a multi-antigen immunization scheme employing the engineered light chain mice (described above) to compare heavy chain gene segment usage and somatic hypermutation frequencies of the antibody chains.

**Heavy Chain Gene Usage.** Heavy and light chain sequences obtained from VELOCIMMUNE^{®} mice containing a replacement of the endogenous mouse heavy chain locus with human V_{H}, D_{H}, and J_{H} gene segments and a replacement of the endogenous mouse κ light chain locus with either the engineered germline Vκ1-39Jκ5 human light chain region or the engineered germline Vκ3-20Jκ1 human light chain region (as described in Example 2) immunized with a human cell-surface receptor (Antigen E), a heterodimer of two human cell-surface glycoproteins (Antigen F), a human cytokine receptor (Antigen G) and a human tumor differentiation antigen (Antigen H) were analyzed for heavy chain gene segment usage and V_{H} and J_{H} gene segments were recorded. Results are shown in Tables 16 - 18. Percentages in Tables 16 - 18 represent rounded values and in some cases may not equal 100% when added together.

Table 16 sets forth the percent heavy chain family usage for antibodies from VELCOlMMUNE^{®} mice (VI), antibodies from VELCOlMMUNE^{®} mice having a cognate Vκ1-39 light chain (VI - Vκ1-39), antibodies from Vκ1-39 engineered light chain mice (Vκ1-39), antibodies from VELCOlMMUNE^{®} mice having a cognate Vκ3-20 light chain (VI - Vκ3-20), and antibodies from Vκ3-20 engineered light chain mice (Vκ3-20). Table 17 sets forth the percent V_{H} and J_{H} gene usage for antibodies from VELCOIMMUNE^{®} mice (VI), antibodies from VELCOIMMUNE^{®} mice having a cognate Vκ1-39 light chain (VI - Vκ1-39), antibodies from Vκ1-39 engineered light chain mice (Vκ1-39), antibodies from VELCOIMMUNE^{®} mice having a cognate Vκ3-20 light chain (VI - Vκ3-20), and antibodies from Vκ3-20 engineered light chain mice (Vκ3-20). Table 18 sets forth the percent V_{H} gene usage for antibodies from Vκ1-39 engineered light chain mice (Vκ1-39 Mice) from each immunization group (Antigens E, F, G and H) and the percent V_{H} gene usage for antibodies from Vκ3-20 engineered light chain mice (Vκ3-20 Mice) from selected immunization groups (Antigens E and G).

As shown in this Example, heavy chain gene usage for antigens tested in Vκ1-39Jκ5-engineered light chain mice was characterized by a preponderance of V_{H} family III subgroups (V_{H}3-7, V_{H}3-9, V_{H}3-11, V_{H}3-13, V_{H}3-20, V_{H}3-23, V_{H}3-30, V_{H}3-33 and V_{H}3-48). Notable usage of other V_{H} family subgroups was characterized by usage of V_{H}1-18, V_{H}1-69, V_{H}2-5, V_{H}4-59 and V_{H}6-1. For antigens tested in Vκ3-20Jκ1 engineered light chain mice, heavy chain gene usage was characterized by a preponderance of V_{H} family III, V_{H} family IV and V_{H} family V subgroups (V_{H}3-11, V_{H}3-30, V_{H}3-33, V_{H}4-39, V_{H}4-59 and V_{H}5-51). Notable usage of other V_{H} family subgroups was characterized by usage of V_{H}1-18, V_{H}1-69, V_{H}2-70 and V_{H}6-1.

Somatic Hypermutation Frequency. Heavy and light chains from antibodies raised in VELCOIMMUNE^{®} mice and the engineered light chain mice (described above) were aligned to germline sequences according to the heavy and light chain gene usage demonstrated for each heavy and/or light chain. Amino acid changes for each framework region (FW) and complementarity determining region (CDR) for both heavy and light chain of each sequence were calculated. Results are shown in Tables 19 - 22. Percentages in Tables 21 - 24 represent rounded values and in some cases may not equal 100% when added together.

Table 19 sets forth the number of amino acid (AA) changes observed in each FW and CDR region of heavy chains of antibodies from VELCOIMMUNE^{®} mice, heavy chains of antibodies from Vκ1-39 engineered light chain mice (Vκ1-39 Mice) and heavy chains of antibodies from Vκ3-20 engineered light chain mice (Vκ3-20 Mice). Table 20 sets forth the number of amino acid (AA) changes observed in each FW and CDR region of light chains of antibodies from VELCOIMMUNE^{®} mice, the light chain of antibodies from Vκ1-39 engineered mice (Vκ1-39 Mice) and the light chain of antibodies from Vκ3-20 engineered mice (Vκ3-20 Mice). Table 21 sets forth the number of amino acid (AA) changes observed in each FW and CDR region of heavy chains of antibodies from Vκ1-39 engineered light chain mice (Vκ1-39 Mice) for selected immunization groups (Antigens E, F and H). Table 22 sets forth the number of amino acid (AA) changes observed in each FW and CDR region of heavy chains of antibodies from Vκ3-20 engineered light chain mice (Vκ3-20 Mice) for selected immunization groups (Antigens E and G).

**TABLE 16**

| V_{H} Family | VI | VI - Vκ1-39 | Vκ1-39 | VI - Vκ3-20 | Vκ3-20 |
|---|---|---|---|---|---|
| 1 | 9.0 | 14.8 | 3.3 | 7.1 | 4.9 |
| 2 | 2.2 | 1.8 | 4.6 | 0 | 1.6 |
| 3 | 77.8 | 69.8 | 77.3 | 61.4 | 29.5 |
| 4 | 8.4 | 8.3 | 11.2 | 27.1 | 39.3 |
| 5 | 0.9 | 0 | 0.7 | 4.3 | 23.0 |
| 6 | 1.7 | 5.3 | 3.0 | 0 | 1.6 |

**TABLE 17**

| V_{H} Gene | VI | VI - Vκ1-39 | Vκ1-39 | VI - Vκ3-20 | Vκ3-20 |
|---|---|---|---|---|---|
| 1-2 | 3.9 | 8.3 | 0 | 2.9 | 0 |
| 1-3 | 0 | 0 | 0 | 0 | 0 |
| 1-8 | 1.3 | 0.6 | 0 | 1.4 | 0 |
| 1-18 | 3.0 | 0.6 | 1.3 | 2.1 | 1.6 |
| 1-24 | 0.4 | 3.6 | 0 | 0.7 | 0 |
| 1-46 | 0.1 | 0 | 0 | 0 | 0 |
| 1-58 | 0 | 0 | 0 | 0 | 0 |
| 1-69 | 0.3 | 1.8 | 2.0 | 0 | 3.3 |
| 2-5 | 1.9 | 0 | 4.6 | 0 | 0 |
| 2-26 | 0.2 | 1.8 | 0.0 | 0 | 0 |
| 2-70 | 0.1 | 0 | 0 | 0 | 1.6 |
| 3-7 | 3.0 | 14.8 | 0 | 1.4 | 0 |
| 3-9 | 8.5 | 3.6 | 29.6 | 16.4 | 0 |
| 3-11 | 5.4 | 10.7 | 0 | 7.1 | 1.6 |
| 3-13 | 3.2 | 1.8 | 0.7 | 2.1 | 0 |
| 3-15 | 4.0 | 4.7 | 0.3 | 0.7 | 0 |
| 3-20 | 1.0 | 0.6 | 0.3 | 5.0 | 0 |
| 3-21 | 0.8 | 0.6 | 0 | 2.1 | 0 |
| 3-23 | 20.4 | 8.9 | 3.3 | 8.6 | 0 |
| 3-30 | 17.6 | 4.1 | 35.2 | 12.9 | 1.6 |
| 3-33 | 12.6 | 14.8 | 0 | 5.0 | 26.2 |
| 3-43 | 0.2 | 0.6 | 0 | 0 | 0 |
| 3-48 | 0.8 | 1.2 | 7.2 | 0 | 0 |
| 3-53 | 0.3 | 3.6 | 0.3 | 0 | 0 |
| 3-64 | 0 | 0 | 0.3 | 0 | 0 |
| 3-72 | 0 | 0 | 0 | 0 | 0 |
| 3-73 | 0 | 0 | 0 | 0 | 0 |
| 4-31 | 2.7 | 0 | 0.7 | 8.6 | 0 |
| 4-34 | 1.8 | 0.6 | 0.3 | 14.3 | 0 |
| 4-39 | 1.6 | 0.6 | 3.0 | 2.1 | 14.8 |
| 4-59 | 2.3 | 7.1 | 7.2 | 2.1 | 24.6 |
| 5-51 | 0.9 | 0 | 0.7 | 4.3 | 23.0 |
| 6-1 | 1.7 | 5.3 | 3.0 | 0 | 1.6 |

| J_{H} Gene | VI | VI - Vκ1-39 | Vκ1-39 | VI - Vκ3-20 | Vκ3-20 |
|---|---|---|---|---|---|
| 1 | 1.5 | 1.2 | 7.1 | 0 | 0 |
| 2 | 4.5 | 2.4 | 0.7 | 5.0 | 26.9 |
| 3 | 10.5 | 16.6 | 13.1 | 13.6 | 26.9 |
| 4 | 44.0 | 34.3 | 32.3 | 50.7 | 9.6 |
| 5 | 9.6 | 10.1 | 16.8 | 7.9 | 1.9 |
| 6 | 29.7 | 35.5 | 30.0 | 22.9 | 34.6 |

**TABLE 18**

| V_{H} Gene | Vκ1-39 Mice | | | | | Vκ3-20 Mice | |
|---|---|---|---|---|---|---|---|
| | Antigen E | Antigen F | Antigen G | Antigen H | | Antigen E | Antigen G |
| 1-2 | 0 | 0 | 0 | 0 | | 0 | 0 |
| 1-3 | 0 | 0 | 0 | 0 | | 0 | 0 |
| 1-8 | 0 | 0 | 0 | 0 | | 0 | 0 |
| 1-18 | 0 | 0 | 0 | 8.3 | | 0 | 3.1 |
| 1-24 | 0 | 0 | 0 | 0 | | 0 | 0 |
| 1-46 | 0 | 0 | 0 | 0 | | 0 | 0 |
| 1-58 | 0 | 0 | 0 | 0 | | 0 | 0 |
| 1-69 | 2.9 | 0 | 25.0 | 0 | | 0 | 6.3 |
| 2-5 | 8.2 | 0 | 0 | 0 | | 0 | 0 |
| 2-26 | 0 | 0 | 0 | 0 | | 0 | 0 |
| 2-70 | 0 | 0 | 0 | 0 | | 0 | 3.1 |
| 3-7 | 0 | 0 | 0 | 0 | | 0 | 0 |
| 3-9 | 1.2 | 98.8 | 0 | 14.6 | | 0 | 0 |
| 3-11 | 0 | 0 | 0 | 0 | | 0 | 3.1 |
| 3-13 | 0.6 | 0 | 25.0 | 0 | | 0 | 0 |
| 3-15 | 0 | 1.2 | 0 | 0 | | 0 | 0 |
| 3-20 | 0 | 0 | 25.0 | 0 | | 0 | 0 |
| 3-21 | 0 | 0 | 0 | 0 | | 0 | 0 |
| 3-23 | 4.1 | 0 | 25.0 | 4.2 | | 0 | 0 |
| 3-30 | 62.9 | 0 | 0 | 0 | | 3.4 | 0 |
| 3-33 | 0 | 0 | 0 | 0 | | 13.8 | 37.5 |
| 3-43 | 0 | 0 | 0 | 0 | | 0 | 0 |
| 3-48 | 0.6 | 0 | 0 | 43.8 | | 0 | 0 |
| 3-53 | 1.6 | 0 | 0 | 0 | | 0 | 0 |
| 3-64 | 1.6 | 0 | 0 | 0 | | 0 | 0 |
| 3-72 | 0 | 0 | 0 | 0 | | 0 | 0 |
| 3-73 | 0 | 0 | 0 | 0 | | 0 | 0 |
| 4-31 | 0 | 0 | 0 | 4.2 | | 0 | 0 |
| 4-34 | 0 | 0 | 0 | 2.1 | | 0 | 0 |
| 4-39 | 5.3 | 0 | 0 | 0 | | 31.0 | 0 |
| 4-59 | 11.8 | 0 | 0 | 4.2 | | 3.4 | 43.8 |
| 5-51 | 1.2 | 0 | 0 | 0 | | 48.3 | 0 |
| 6-1 | 0 | 0 | 0 | 18.8 | | 0 | 3.1 |

**TABLE 19**

| # AA Changes | Heavy Chains of Antibodies from VELCOIMMUNE^{®} Mice | | | | | |
|---|---|---|---|---|---|---|
| | FW1 | CDR1 | FW2 | CDR2 | FW3 | CDR3 |
| 0 | 63 | 32 | 36 | 26 | 12 | 82 |
| 1 | 23 | 32 | 41 | 31 | 22 | 17 |
| 2 | 9 | 25 | 17 | 23 | 27 | 1 |
| 3 | 4 | 10 | 5 | 16 | 13 | 0 |
| 4 | 0 | 1 | 1 | 3 | 12 | 0 |
| >5 | 1 | 0 | 0 | 1 | 14 | 0 |

| # AA Changes | Heavy Chains of Antibodies from Vκ1-39 Mice | | | | | |
|---|---|---|---|---|---|---|
| | FW1 | CDR1 | FW2 | CDR2 | FW3 | CDR3 |
| 0 | 65 | 8 | 34 | 30 | 9 | 37 |
| 1 | 25 | 26 | 35 | 34 | 19 | 54 |
| 2 | 9 | 44 | 23 | 20 | 33 | 9 |
| 3 | 1 | 19 | 8 | 12 | 22 | 0 |
| 4 | 0 | 3 | 0 | 5 | 11 | 0 |
| >5 | 1 | 0 | 0 | 0 | 7 | 0 |

| # AA Changes | Heavy Chains of Antibodies from Vκ3-20 Mice | | | | | |
|---|---|---|---|---|---|---|
| | FW1 | CDR1 | FW2 | CDR2 | FW3 | CDR3 |
| 0 | 57 | 8 | 54 | 16 | 8 | 93 |
| 1 | 41 | 43 | 34 | 39 | 21 | 7 |
| 2 | 2 | 25 | 10 | 18 | 20 | 0 |
| 3 | 0 | 15 | 2 | 21 | 13 | 0 |
| 4 | 0 | 10 | 0 | 3 | 20 | 0 |
| >5 | 0 | 0 | 0 | 2 | 18 | 0 |

**TABLE 20**

| # AA Changes | Light Chains of Antibodies from VELCOlMMUNE^{®} Mice | | | | | |
|---|---|---|---|---|---|---|
| | FW1 | CDR1 | FW2 | CDR2 | FW3 | CDR3 |
| 0 | 65 | 24 | 49 | 60 | 33 | 23 |
| 1 | 24 | 20 | 34 | 31 | 27 | 38 |
| 2 | 9 | 27 | 16 | 9 | 18 | 28 |
| 3 | 1 | 20 | 1 | 0 | 14 | 7 |
| 4 | 0 | 7 | 0 | 0 | 4 | 3 |
| >5 | 1 | 1 | 0 | 0 | 3 | 0 |

| # AA Changes | Light Chains of Antibodies from Vκ1-39 Mice | | | | | |
|---|---|---|---|---|---|---|
| | FW1 | CDR1 | FW2 | CDR2 | FW3 | CDR3 |
| 0 | 91 | 75 | 80 | 90 | 71 | 63 |
| 1 | 9 | 19 | 17 | 10 | 21 | 27 |
| 2 | 0 | 5 | 1 | 1 | 5 | 8 |
| 3 | 0 | 0 | 1 | 0 | 2 | 1 |
| 4 | 0 | 0 | 0 | 0 | 2 | 1 |
| >5 | 0 | 0 | 0 | 0 | 0 | 0 |

| # AA Changes | Light Chains of Antibodies from Vκ3-20 Mice | | | | | |
|---|---|---|---|---|---|---|
| | FW1 | CDR1 | FW2 | CDR2 | FW3 | CDR3 |
| 0 | 90 | 47 | 93 | 97 | 63 | 57 |
| 1 | 10 | 27 | 3 | 3 | 20 | 43 |
| 2 | 0 | 27 | 3 | 0 | 17 | 0 |
| 3 | 0 | 0 | 0 | 0 | 0 | 0 |
| 4 | 0 | 0 | 0 | 0 | 0 | 0 |
| >5 | 0 | 0 | 0 | 0 | 0 | 0 |

**TABLE 21**

| # AA Changes | Heavy Chains of Anti-Antigen E Antibodies from Vκ1-39 Mice | | | | | |
|---|---|---|---|---|---|---|
| | FW1 | CDR1 | FW2 | CDR2 | FW3 | CDR3 |
| 0 | 75 | 8 | 49 | 41 | 14 | 36 |
| 1 | 21 | 25 | 33 | 35 | 25 | 52 |
| 2 | 4 | 43 | 14 | 18 | 28 | 12 |
| 3 | 0 | 20 | 4 | 5 | 16 | 0 |
| 4 | 0 | 5 | 0 | 1 | 12 | 0 |
| >5 | 1 | 0 | 0 | 0 | 5 | 0 |

| # AA Changes | Heavy Chains of Anti-Antigen F Antibodies from Vκ1-39 Mice | | | | | |
|---|---|---|---|---|---|---|
| | FW1 | CDR1 | FW2 | CDR2 | FW3 | CDR3 |
| 0 | 52 | 0 | 6 | 6 | 2 | 15 |
| 1 | 35 | 24 | 32 | 35 | 15 | 78 |
| 2 | 11 | 59 | 46 | 22 | 49 | 7 |
| 3 | 0 | 17 | 16 | 24 | 29 | 0 |
| 4 | 0 | 0 | 0 | 12 | 4 | 0 |
| >5 | 1 | 0 | 0 | 0 | 1 | 0 |

| # AA Changes | Heavy Chains of Anti-Antigen H Antibodies from Vκ1-39 Mice | | | | | |
|---|---|---|---|---|---|---|
| | FW1 | CDR1 | FW2 | CDR2 | FW3 | CDR3 |
| 0 | 54 | 21 | 29 | 33 | 4 | 77 |
| 1 | 17 | 35 | 50 | 27 | 6 | 23 |
| 2 | 23 | 21 | 15 | 21 | 25 | 0 |
| 3 | 6 | 21 | 4 | 15 | 27 | 0 |
| 4 | 0 | 2 | 2 | 2 | 15 | 0 |
| >5 | 0 | 0 | 0 | 2 | 23 | 0 |

**TABLE 22**

| # AA Changes | Heavy Chains of Anti-Antigen E Antibodies from Vκ3-20 Mice | | | | | |
|---|---|---|---|---|---|---|
| | FW1 | CDR1 | FW2 | CDR2 | FW3 | CDR3 |
| 0 | 79 | 17 | 62 | 24 | 17 | 90 |
| 1 | 21 | 28 | 34 | 55 | 31 | 10 |
| 2 | 0 | 28 | 3 | 21 | 24 | 0 |
| 3 | 0 | 14 | 0 | 0 | 10 | 0 |
| 4 | 0 | 14 | 0 | 0 | 3 | 0 |
| >5 | 0 | 0 | 0 | 0 | 14 | 0 |

| # AA Changes | Heavy Chains of Anti-Antigen G Antibodies from Vκ3-20 Mice | | | | | |
|---|---|---|---|---|---|---|
| | FW1 | CDR1 | FW2 | CDR2 | FW3 | CDR3 |
| 0 | 38 | 0 | 47 | 9 | 0 | 97 |
| 1 | 59 | 56 | 34 | 25 | 13 | 3 |
| 2 | 3 | 22 | 16 | 16 | 16 | 0 |
| 3 | 0 | 16 | 3 | 41 | 16 | 0 |
| 4 | 0 | 6 | 0 | 6 | 34 | 0 |
| >5 | 0 | 0 | 0 | 3 | 22 | 0 |

### Example 13. Binding Affinity of Bispecific Antibodies Having Universal Light Chains

Fully human bispecific antibodies were constructed from cloned human heavy chain variable regions of selected monospecific anti-Antigen E common light chain antibodies (described in Example 5) using standard recombinant DNA techniques known in the art. Table 23 sets forth the pairing of human heavy chains (HC-1 and HC-2) from selected parental monospecific antibodies; each pair employed with a germline rearranged human Vκ1-39/Jκ1 light chain for construction of each bispecific antibody.

Binding of bispecific or parental monospecific anti-Antigen E antibodies to the extracellular domain (ECD) of Antigen E was determined using a real-time surface plasmon resonance biosensor assay on a BIACORE^{™} 2000 instrument (GE Healthcare). A CM5 BIACORE^{™} sensor surface derivatized with anti-c-myc-specific monoclonal antibody (Clone# 9E10) using EDC-NHS chemistry was used to capture the C-terminal myc-myc-hexahistidine tagged ECD of Antigen E (AntigenE-mmh). Around 190 RUs of AntigenE-mmh was captured on the BIACORE^{™} sensor surface, followed by the injection of 300nM and 50nM concentrations of different bispecific or parental monospecific anti-Antigen E antibodies at a flow rate of 50 µl/min. The experiment was performed at 25°C in HBST running buffer (0.01M HEPES pH 7.4, 0.15M NaCl, 3mM EDTA, 0.05% v/v Surfactant P20). The amount of antibody binding to AntigenE-mmh surface at 300nM concentration was recorded three seconds before the end of antibody injection and plotted.

Table 24 and FIG. 8 set forth the binding responses (BIACORE^{™} units; RU) observed for each bispecific antibody (BsAb) and monospecific parental antibody (PAb-1, PAb-2). Since each antibody was injected under saturating conditions over an identical AntigenE-mmh surface, the binding response reflects the binding stoichiometry for each antibody binding to the antigen capture surface.

As shown in this Example, the observed binding response for each bispecific antibody was approximately 2-fold greater than the binding response for each parental monospecific antibody (Table 24 and FIG. 8), demonstrating functional construction of bispecific antibodies using heavy chains of antigen-specific monoclonal antibodies and a common light chain where each Fab arm in the bispecific antibody molecule binds simultaneously to distinct epitopes on the extracelluar domain of a cell surface receptor (Antigen E; see FIG. 7B, bottom left).

**TABLE 23**

| Bispecific Antibody | Parent HC-1 | Parent HC-2 |
|---|---|---|
| 3108 | 2952 | 2978 |
| 3109 | 2978 | 3022 |
| 3111 | 2952 | 3005 |
| 3112 | 3022 | 3005 |

**TABLE 24**

| Bispecific Antibody | Binding Response (RU) | | |
|---|---|---|---|
| | PAb-1 | PAb-2 | BsAb |
| 3108 | 236 | 229 | 485 |
| 3109 | 236 | 197 | 408 |
| 3111 | 202 | 229 | 435 |
| 3112 | 202 | 197 | 345 |

### Example 14. Generation and Analysis of Mice Expressing Two Human Light Chains

Using the methods described above in Example 2, two additional engineered light chain loci containing two human Vκ gene segments (e.g., a human Vκ1-39 and human Vκ3-20 gene segment) were constructed (FIG. 9). One engineered light chain locus contained two human Vκ gene segments and five human Jκ gene segments in unrearranged configuration (DLC-5J). The second engineered light chain locus contained two human Vκ gene segments and one human Jκ gene segment in unrearranged configuration (DLC-1J). For each of the two additional engineered light chain loci, the human gene segments were flanked 3' with recombination signal sequences to allow for *in vivo* rearrangement of the human gene segments in B cells.

Modified BAC DNA clones separately containing each of the the engineered light chain loci operably linked to mouse sequences (*i.e.,* upstream and downstream sequences of the endogenous immunoglobulin κ light chain locus) were confirmed by PCR using primers located at sequences within each engineered light chain locus containing the two human Vκ gene segments followed by electroporation into ES cells to create mice that express either of the two human Vκ gene segments (as described above). Positive ES cell clones that contain either of the engineered light chain loci described above were confirmed by TAQMAN^{™} screening and karyotyping using probes specific for the engineered light chain loci (as described above). Confirmed ES cell clones were then used to implant female mice to give rise to a litter of pups expressing a human light chain variable domain fused with a mouse Cκ domain, referred to herein as Dual Light Chain (DLC) mice.

Alternatively, ES cells bearing the engineered light chain locus may be transfected with a construct that expresses FLP in order to remove the FRTed neomycin cassette introduced by the targeting construct. Optionally, the neomycin cassette is removed by breeding to mice that express FLP recombinase (e.g., US 6,774,279). Optionally, the neomycin cassette is retained in the mice.

Flow Cytometry. B cell populations and B cell development in DLC mice were validated by flow cytometry analysis of splenocyte and bone marrow preparations. Cell suspensions from mice homozygous for two human Vκ gene segments and five human Jκ gene segments (n=4), mice homozygous for two human Vκ gene segments and one human Jκ gene segment (n=4), and wild type mice (n=4) were made using standard methods (described above) and stained with fluorescently labeled antibodies (as described in Example 3).

Briefly, 1x10⁶ cells were incubated with anti-mouse CD16/CD32 (clone 2.4G2, BD Pharmigen) on ice for 10 minutes, followed by labeling with the following antibody cocktail for 30 minutes on ice: APC-H7 conjugated anti-mouse CD19 (clone 1D3, BD Pharmigen), Pacific Blue conjugated anti-mouse CD3 (clone 17A2, BioLegend), FITC conjugated anti-mouse Igκ (clone 187.1, BD Pharmigen) or anti-mouse CD43 (clone 1B11, BioLegend), PE conjugated anti-mouse Igλ (clone RML-42, BioLegend) or anti-mouse c-kit (clone 2B8, BioLegend), PerCP-Cy5.5 conjugated anti-mouse IgD (BioLegend), PE-Cy7 conjugated anti-mouse IgM (clone II/41, eBioscience), APC conjugated anti-mouse B220 (clone RA3-6B2, eBioscience). Following staining, cells were washed and fixed in 2% formaldehyde. Data acquisition was performed on an LSRII flow cytometer and analyzed with FlowJo (Tree Star, Inc.). Gating: total B cells (CD19⁺CD3⁻), Igr⁺ B cells (Igκ⁺Igλ⁻CD19⁺CD3⁻), Igλ⁺ B cells (Igκ⁻ Igλ⁺CD19⁺CD3⁻). Results for the bone marrow compartment are shown in FIG. 10A - FIG. 12B. Results for the splenic compartment are shown in FIG. 13A - FIG. 16.

As shown in this Example, DLC-5J mice demonstrate normal B cell populations within the splenic and bone marrow compartments (FIG. 10A - 16). DLC-5J mice demonstrated immature, mature and pre/pro B cell populations within the bone marrow compartment that are substantially the same as observed in wild-type litter mates. In fact, the DLC-5J locus was capable of competing with the endogenous λ light chain locus to yield a κ:λ ratio that is substantially the same as that observed in wild-type mice (FIG. 14B). Also, DLC-5J mice demonstrate a normal peripheral B cell development as progression of B cells through various stages in the splenic compartment (e.g., immature, mature, T1, T2 T3, marginal zone precursor, marginal zone, follicular-I, follicular-II, *etc*.) occurs in a manner substantially the same as observed in wild type mice (FIG. 15A - 16). In contrast, DLC-1J mice demonstrated a lower overall number of B cells and an increased λ light chain usage as compared to the engineered κ light chain (data not shown).

**Dual Light Chain Expression.** Expression of both human Vκ gene segments was analyzed in homozygous mice using a quantitative PCR assay in accordance with in Example 3. Briefly, CD19⁺ B cells were purified from bone marrow and whole spleens of wild type mice, mice homozygous for a replacement of the mouse heavy chain and κ light chain variable loci with corresponding human heavy chain and κ light chain variable region loci (Hκ), as well as mice homozygous for an engineered κ light chain loci containing two human Vκ gene segments and either five human Jκ gene segments (DLC-5J) or one human Jκ gene segment (DLC-1J). Relative expression was normalized to expression of mouse Cκ region (n=3 to 5 mice per group). Results are shown in FIG. 17 and FIG. 18.

Expression of light chains containing a rearranged human Vκ3-20 or human Vκ1-39 gene segment were detected in both the bone marrow and spleen of DLC-5J and DLC-1J mice (FIG. 17 and FIG. 18). In the bone marrow compartment, expression of both human Vκ3-20-derived and human Vκ1-39-derived light chains in both strains of DLC mice was significantly higher as compared to mice comprising a replacement of mouse Vκ and Jκ gene segment with corresponding human Vκ and Jκ gene segments (Hκ; FIG. 17). Human Vκ3-20-derived light chain expression was observed at about six-fold (DLC-5J) to fifteen-fold (DLC-1J) higher than in Hκ mice. DLC-1J mice demonstrated about two-fold higher expression of human Vκ3-20-derived light chains over DLC-5J mice in the bone marrow compartment. Human Vκ1-39-derived light chain expression was observed at about six-fold (DLC-5J) to thirteen-fold (DLC-1J) higher than in Hκ mice. DLC-1J mice demonstrated about two-fold higher expression of human Vκ1-39-derived light chains over DLC-5J mice in the bone marrow compartment.

In the splenic compartment, expression of both human Vκ3-20-derived and human Vκ1-39-derived light chains in both strains of DLC mice was significantly higher as compared to Hκ mice (FIG. 18). Human Vκ3-20-derived light chain expression was observed at about four-fold (DLC-5J) and eight-fold (DLC-1J) higher than in Hκ mice. DLC-1J mice demonstrated about two-fold higher expression of human Vκ3-20-derived light chains over DLC-5J mice in the splenic compartment. Human Vκ1-39-derived light chain expression was observed at about four-fold (DLC-5J) to five-fold (DLC-1J) higher than in Hκ mice. DLC-1J mice demonstrated similar expression of human Vκ1-39-derived light chains as compared to DLC-5J mice in the splenic compartment.

**Human V_{L}/J_{L} Usage in DLC-5J Mice.** Mice homozygous for two unrearranged human Vκ gene segments and five unrearranged human Jκ gene segments (DLC-5J) were analyzed for human Vκ/Jκ gene segment usage in splenic B cells by reverse-transcriptase polymerase chain reaction (RT-PCR).

Briefly, spleens from homozygous DLC-5J (n=3) and wild type (n=2) mice were harvested and meshed in 10 mL of RPMI 1640 (Sigma) containing 10% heat-inactivated fetal bovine serum using frosted glass slides to create single cell suspensions. Splenocytes were pelleted with a centrifuge (1200 rpm for five minutes) and red blood cells were lysed in 5 mL of ACK lysing buffer (GIBCO) for three minutes. Splenocytes were diluted with PBS (Irvine Scientific), filtered with a 0.7 µm cell strainer and centrifuged again to pellet cells, which was followed by resuspension in 1 mL of PBS.

RNA was isolated from pelleted splenocytes using AllPrep DNA/RNA mini kit (Qiagen) according to manufacturer's specifications. RT-PCR was performed on splenocyte RNA using 5' RACE (Rapid Amplification of cDNA ends) System with primers specific for the mouse Cκ gene according to manufacturer's specifications (Invitrogen). The primers specific for the mouse Cκ gene were 3' mlgκC RACE1 (AAGAAGCACA CGACTGAGGC AC; SEQ ID NO: 34) and mlgκC3'-1 (CTCACTGGAT GGTGGGAAGA TGGA; SEQ ID NO: 35). PCR products were gel-purified and cloned into pCR^{®}2.1-TOPO^{®} vector (TOPO^{®} TA Cloning^{®} Kit, Invitrogen) and sequenced with M13 Forward (GTAAAACGAC GGCCAG; SEQ ID NO: 36) and M13 Reverse (CAGGAAACAG CTATGAC; SEQ ID NO: 37) primers located within the vector at locations flanking the cloning site. Ten clones from each spleen sample were sequenced. Sequences were compared to the mouse and human immunoglobulin sets from the IMGT/V-QUEST reference directory sets to determine Vκ/Jκ usage. Table 25 sets forth the Vκ/Jκ combinations for selected clones observed in RT-PCR clones from each splenocyte sample. Table 26 sets forth the amino acid sequence of the human Vκ/human Jκ and human Jκ/mouse Cκ junctions of selected RT-PCR clones from DLC-5J homozygous mice. Lower case letters indicate mutations in the amino acid sequence of the variable region or non-template additions resulting from N and/or P additions during recombination.

As shown in this Example, mice homozygous for two unrearranged human Vκ gene segments and five unrearranged human Jκ gene segments (DLC-5J) operably linked to the mouse Cκ gene are able to productively recombine both human Vκ gene segments to multiple human Jκ gene segments to produce a limited immunoglobulin light chain repertoire. Among the rearrangements in DLC-5J homozygous mice shown in Table 25, unique human Vκ/Jκ rearrangements were observed for Vκ1-39/Jκ2 (1), Vκ1-39/Jκ3 (1), Vκ3-20/Jκ1 (7), Vκ3-20/Jκ2 (4) and Vκ3-20/Jκ3 (1). Further, such unique rearrangements demonstrated junctional diversity through the presence of unique amino acids within the CDR3 region of the light chain (Table 26) resulting from either mutation and/or the recombination of the human Vκ and Jκ gene segments during development. All the rearrangements showed functional read through into mouse Cκ (Table 26).

Taken together, these data demonstrate that mice engineered to present a choice of no more than two human V_{L} gene segments, both of which are capable of rearranging (e.g., with one or more and, in some embodiments, up to five human J_{L} gene segments) and encoding a human V_{L} domain of an immunoglobulin light chain have B cell numbers and development that is nearly wild-type in all aspects. Such mice produce a collection of antibodies having immunoglobulin light chains that have one of two possible human V_{L} gene segments present in the collection. This collection of antibodies is produced by the mouse in response to antigen challenge and are associated with a diversity of reverse chimeric (human variable/mouse constant) heavy chains.

**TABLE 25**

| Mouse ID No. | Genotype | Clone | Vκ/Jκ Combination |
|---|---|---|---|
| 1089451 | DLC-5J | 1-2 | 1-39/3 |
| | | 1-4 | 3-20/2 |
| | | 1-7 | 3-20/1 |
| | | 1-8 | 3-20/2 |
| 1089452 | DLC-5J | 2-2 | 3-20/1 |
| | | 2-3 | 3-20/1 |
| | | 2-6 | 3-20/2 |
| | | 2-8 | 3-20/2 |
| | | 2-9 | 3-20/1 |
| | | 2-10 | 1-39/2 |
| 1092594 | DLC-5J | 3-1 | 3-20/1 |
| | | 3-2 | 3-20/1 |
| | | 3-4 | 3-20/1 |
| | | 3-6 | 3-20/3 |
| | | 3-9 | 3-20/2 |
| 1092587 | WT | 1-1 | 19-93/1 |
| | | 1-2 | 6-25/1 |
| | | 1-3 | 4-91/5 |
| | | 1-5 | 3-10/4 |
| | | 1-6 | 4-86/4 |
| | | 1-8 | 19-93/1 |
| | | 1-10 | 19-93/2 |
| 1092591 | WT | 2-1 | 19-93/1 |
| | | 2-3 | 6-20/5 |
| | | 2-4 | 6-25/5 |
| | | 2-5 | 1-117/1 |
| | | 2-6 | 8-30/1 |
| | | 2-7 | 8-19/2 |
| | | 2-8 | 8-30/1 |
| | | 2-10 | 1-117/1 |

**TABLE 26**

| Clone | Vκ/Jκ | Sequence of hVκ/hJκ/mCκ Junction (CDR3 underlined, mlgκC italics) | SEQ ID NO: |
|---|---|---|---|
| 2-10 | 1-39/2 | QPEDFATYYCQQSYSTPYTFGQGTKLEI*KRADAAPTVSI* | 38 |
| 1-2 | 1-39/3 | QPEDFATYYCQQSYSTPFTFGPGTKVDI*KRADAAPTVSI* | 39 |
| 1-7 | 3-20/1 | EPEDFAVYYCQQYGSSPRTFGQGTKVEI*KRADAAPTVSI* | 40 |
| 2-2 | 3-20/1 | EPEDFAVYYCQQYGSSrTFGQGTKVEI*KRADAAPTVSI* | 41 |
| 2-3 | 3-20/1 | EPEDFAVYYCQQYGSSPWTFGQGTKVEI*KRADAAPTVSI* | 42 |
| 2-9 | 3-20/1 | dPEDFAVYYCQQYGSSPrTFGQGTKVEI*KRADAAPTVSI* | 44 |
| 3-1 | 3-20/1 | EPEDFAVYYCQQYGSSPrTFGQGTKVEI*KRADAAPTVSI* | 45 |
| 3-2 | 3-20/1 | EPEDFAVYYCQQYGSSPWTFGQGTKVEIK*RADAAPTVSI* | 46 |
| 3-4 | 3-20/1 | EPEDFAVYYCQQYGSSPPTFGQGTKVEIK*RADAAPTVSI* | 47 |
| 3-9 | 3-20/2 | EPEDFAVYYCQQYGSSPYTFGQGTKLEIK*RADAAPTVSI* | 48 |
| 3-6 | 3-20/3 | EPEDFAVYYCQQYGSSiFTFGPGTKVDIK*RADAAPTVSI* | 49 |

## Claims

1. A mouse embryonic stem (ES) cell that has been genetically modified so that it comprises in its genome:
exactly two unrearranged human immunoglobulin Vκ gene segments and five unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment.

2. The genetically modified mouse ES cell of claim 1, further comprising in its genome:
one or more unrearranged human immunoglobulin V_{H} gene segments, one or more unrearranged human immunoglobulin D_{H} gene segments, and one or more unrearranged human immunoglobulin J_{H} gene segments operably linked to a mouse immunoglobulin heavy chain constant region sequence at the endogenous heavy chain loci.

3. The genetically modified ES cell of claim 1 or 2, wherein:
(a) the two unrearranged human immunoglobulin Vκ gene segments and five unrearranged human immunoglobulin Jκ gene segments are present at the endogenous immunoglobulin κ light chain locus;
(b) the genome of the genetically modified mouse ES cell does not comprise a functional λ light chain locus;
(c) the genome of the genetically modified mouse comprises a nonfunctional immunoglobulin λ light chain locus;
(d) the mouse light chain constant region is a mouse Cκ constant region, optionally wherein the mouse Cκ constant region is an endogenous mouse Cκ constant region; or
(e) the human Vκ1-39 gene segment in the genome of the genetically modified mouse ES cell is a human germline Vκ1-39 gene segment and the human Vκ3-20 gene segment in the genome of the genetically modified mouse ES cell is a human germline Vκ3-20 gene segment.

4. The genetically modified mouse ES cell of any one of the preceding claims, wherein the genome of the genetically modified mouse ES cell comprises in order: the human Vκ1-39 gene segment, the human Vκ3-20 gene segment, the human Jκ1 gene segment, the human Jκ2 gene segment, the human Jκ3 gene segment, the human Jκ4 gene segment, and the human Jκ5 gene segment.

5. The genetically modified mouse ES cell of any one of the preceding claims, wherein the genome of the genetically modified mouse ES cell comprises a DLC-5J locus, wherein the DLC-5J locus comprises a hVκ/hJκ/mCκ junction sequence selected from SEQ ID NOs: 38-49.

6. A mouse embryo derived from the genetically modified ES cell of claim 1 or claim 2.

7. A method of making a genetically modified mouse ES cell, the method comprising genetically modifying an ES cell so that it comprises in its genome exactly two unrearranged human immunoglobulin Vκ gene segments and five unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment.

8. The method of claim 7, wherein the genetically modified mouse ES cell further comprising in its genome:
one or more unrearranged human immunoglobulin V_{H} gene segments, one or more unrearranged human immunoglobulin D_{H} gene segments, and one or more unrearranged human immunoglobulin J_{H} gene segments operably linked to a mouse immunoglobulin heavy chain constant region sequence at the endogenous heavy chain loci.

9. The method of claim 7 or 8, wherein:
(a) the two unrearranged human immunoglobulin Vκ gene segments and five unrearranged human immunoglobulin Jκ gene segments are present at the endogenous immunoglobulin κ light chain locus;
(b) the genome of the genetically modified mouse ES cell does not comprise a functional λ light chain locus;
(c) the genome of the genetically modified mouse comprises a nonfunctional immunoglobulin λ light chain locus;
(d) the mouse light chain constant region is a mouse Cκ constant region, optionally wherein the mouse Cκ constant region is an endogenous mouse Cκ constant region; or
(e) the human Vκ1-39 gene segment in genome of the genetically modified mouse ES cell is a human germline Vκ1-39 gene segment and the human Vκ3-20 gene segment in the genome of the genetically modified mouse ES cell is a human germline Vκ3-20 gene segment.

10. The method of any one of claims 7 to 9, wherein the genome of the genetically modified mouse ES cell comprises in order: the human Vκ1-39 gene segment, the human Vκ3-20 gene segment, the human Jκ1 gene segment, the human Jκ2 gene segment, the human Jκ3 gene segment, the human Jκ4 gene segment, and the human Jκ5 gene segment.

11. The method of any one of claims 6 to 10, wherein the genome of the genetically modified mouse ES cell comprises a DLC-5J locus, wherein the DLC-5J locus comprises a hVκ/hJκ/mCκ junction sequence selected from SEQ ID NOs: 38-49.

12. A method of making a genetically modified mouse comprising genetically modifying the germline genome of the mouse so that it includes exactly two unrearranged human immunoglobulin Vκ gene segments and five unrearranged human immunoglobulin Jκ gene segments operably linked to a mouse immunoglobulin light chain constant region sequence, wherein the two unrearranged human immunoglobulin Vκ gene segments are a human Vκ1-39 gene segment and a human Vκ3-20 gene segment, and wherein the five unrearranged human immunoglobulin Jκ gene segments are a human Jκ1 gene segment, a human Jκ2 gene segment, a human Jκ3 gene segment, a human Jκ4 gene segment, and a human Jκ5 gene segment.

13. The method of claim 12, wherein the genetically modified mouse further comprises in its genome:
one or more unrearranged human immunoglobulin V_{H} gene segments, one or more unrearranged human immunoglobulin D_{H} gene segments, and one or more unrearranged human immunoglobulin J_{H} gene segments operably linked to a mouse immunoglobulin heavy chain constant region sequence at the endogenous heavy chain loci.

14. The method of claim 12 or 13, wherein:
(a) the two unrearranged human immunoglobulin Vκ gene segments and five unrearranged human immunoglobulin Jκ gene segments are present at the endogenous immunoglobulin κ light chain locus;
(b) the genome of the genetically modified mouse does not comprise a functional λ light chain locus;
(c) the genome of the genetically modified mouse comprises a nonfunctional immunoglobulin λ light chain locus;
(d) the mouse light chain constant region is a mouse Cκ constant region, optionally wherein the mouse Cκ constant region is an endogenous mouse Cκ constant region; or
(e) the human Vκ1-39 gene segment in genome of the genetically modified mouse ES cell is a human germline Vκ1-39 gene segment and the human Vκ3-20 gene segment in the genome of the genetically modified mouse ES cell is a human germline Vκ3-20 gene segment.

15. The method of any one of claims 12 to 14, wherein the genome of the genetically modified mouse comprises in order: the human Vκ1-39 gene segment, the human Vκ3-20 gene segment, the human Jκ1 gene segment, the human Jκ2 gene segment, the human Jκ3 gene segment, the human Jκ4 gene segment, and the human Jκ5 gene segment.

16. The method of any one of claims 12 to 15, wherein the genome of the genetically modified mouse comprises a DLC-5J locus, wherein the DLC-5J locus comprises a hVκ/hJκ/mCκ junction sequence selected from SEQ ID NOs: 38-49.

## Patentansprüche

1. Embryonale Stammzelle (ES-Zelle) der Maus, die genetisch derart modifiziert wurde, dass sie in ihrem Genom umfasst:
genau zwei nicht neu geordnete menschliche Immunglobulin-Vκ-Gensegmente und fünf nicht neu geordnete menschliche Immunglobulin-Jκ-Gensegmente, die mit einer Sequenz der konstanten Region der leichten Kette des Immunglobulins der Maus wirkverbunden sind, wobei die zwei nicht neu geordneten menschlichen Immunglobulin-Vκ-Gensegmente ein menschliches Vκ1-39-Gensegment und ein menschliches Vκ3-20-Gensegment sind, und wobei die fünf nicht neu geordneten menschlichen Immunglobulin-Jκ-Gensegmente ein menschliches Jκ1-Gensegment, ein menschliches Jκ2-Gensegment, ein menschliches Jκ3-Gensegment, ein menschliches Jκ4-Gensegment und ein menschliches Jκ5-Gensegment sind.

2. Genetisch modifizierte ES-Zelle der Maus nach Anspruch 1, die ferner in ihrem Genom umfasst:
ein oder mehrere nicht neu geordnete menschliche Immunglobulin-V_{H}-Gensegmente, ein oder mehrere nicht neu geordnete menschliche Immunoglobulin-D_{H}-Gensegmente, und ein oder mehrere nicht neu geordnete menschliche Immunoglobulin-J_{H}-Gensegmente, die mit einer Sequenz der konstanten Region der schweren Kette des Immunglobulins der Maus an den endogenen Loci der schweren Kette wirkverbunden sind.

3. Genetisch modifizierte ES-Zelle nach Anspruch 1 oder 2, wobei:
(a) die zwei nicht neu geordneten menschlichen Immunglobulin-Vκ-Gensegmente und fünf nicht neu geordneten menschlichen Immunglobulin-Jκ-Gensegmente an dem endogenen Locus der leichten κ-Kette des Immunglobulins vorhanden sind;
(b) das Genom der genetisch modifizierten ES-Zelle der Maus keinen funktionellen Locus der leichten λ-Kette umfasst;
(c) das Genom der genetisch modifizierten Maus einen nicht funktionellen Locus der leichten λ-Kette des Immunglobulins umfasst;
(d) die konstante Region der leichten Kette der Maus eine konstante Cκ-Region der Maus ist, wobei optional die konstante Cκ-Region der Maus eine endogene konstante Cκ-Region der Maus ist; oder
(e) das menschliche Vκ1-39-Gensegment in dem Genom der genetisch modifizierten ES-Zelle der Maus ein menschliches Keimbahn-Vκ1-39-Gensegment ist und das menschliche Vκ3-20-Gensegment in dem Genom der genetisch modifizierten ES-Zelle der Maus ein menschliches Keimbahn-Vκ3-20-Gensegment ist.

4. Genetisch modifizierte ES-Zelle der Maus nach einem der vorhergehenden Ansprüche, wobei das Genom der genetisch modifizierten ES-Zelle der Maus in dieser Reihenfolge umfasst: das menschliche Vκ1-39-Gensegment, das menschliche Vκ3-20-Gensegment, das menschliche Jκ1-Gensegment, das menschliche Jκ2-Gensegment, das menschliche Jκ3-Gensegment, das menschliche Jκ4-Gensegment und das menschliche Jκ5-Gensegment.

5. Genetisch modifizierte ES-Zelle der Maus nach einem der vorhergehenden Ansprüche, wobei das Genom der genetisch modifizierten ES-Zelle der Maus einen DLC-5J-Locus umfasst, wobei der DLC-5J-Locus eine hVκ/hJκ/mCκ-Knotenpunktsequenz umfasst, ausgewählt aus SEQ ID NO: 38-49.

6. Mausembryo, abgeleitet von der genetisch modifizierten ES-Zelle nach Anspruch 1 oder 2.

7. Verfahren zum Herstellen einer genetisch modifizierten ES-Zelle der Maus, wobei das Verfahren das genetische Modifizieren einer ES-Zelle derart umfasst, dass sie in ihrem Genom genau zwei nicht neu geordnete menschliche Immunglobulin-Vκ-Gensegmente und fünf nicht neu geordnete menschliche Immunglobulin-Jκ-Gensegmente umfasst, die mit einer Sequenz der konstanten Region der leichten Kette des Immunglobulins der Maus wirkverbunden sind, wobei die zwei nicht neu geordneten menschlichen Immunglobulin-Vκ-Gensegmente ein menschliches Vκ1-39-Gensegment und ein menschliches Vκ3-20-Gensegment sind, und wobei die fünf nicht neu geordneten menschlichen Immunglobulin-Jκ-Gensegmente ein menschliches Jκ1-Gensegment, ein menschliches Jκ2-Gensegment, ein menschliches Jκ3-Gensegment, ein menschliches Jκ4-Gensegment und ein menschliches Jκ5-Gensegment sind.

8. Verfahren nach Anspruch 7, wobei die genetisch modifizierte ES-Zelle der Maus ferner in ihrem Genom umfasst:
ein oder mehrere nicht neu geordnete menschliche Immunglobulin-V_{H}-Gensegmente, ein oder mehrere nicht neu geordnete menschliche Immunoglobulin-D_{H}-Gensegmente, und ein oder mehrere nicht neu geordnete menschliche Immunoglobulin-J_{H}-Gensegmente, die mit einer Sequenz der konstanten Region der schweren Kette des Immunglobulins der Maus an den endogenen Loci der schweren Kette wirkverbunden sind.

9. Verfahren nach Anspruch 7 oder 8, wobei:
(a) die zwei nicht neu geordneten menschlichen Immunglobulin-Vκ-Gensegmente und fünf nicht neu geordneten menschlichen Immunglobulin-Jκ-Gensegmente an dem endogenen Locus der leichten κ-Kette des Immunglobulins vorhanden sind;
(b) das Genom der genetisch modifizierten ES-Zelle der Maus keinen funktionellen Locus der leichten λ-Kette umfasst;
(c) das Genom der genetisch modifizierten Maus einen nicht funktionellen Locus der leichten λ-Kette des Immunglobulins umfasst;
(d) die konstante Region der leichten Kette der Maus eine konstante Cκ-Region der Maus ist, wobei optional die konstante Cκ-Region der Maus eine endogene konstante Cκ-Region der Maus ist; oder
(e) das menschliche Vκ1-39-Gensegment in dem Genom der genetisch modifizierten ES-Zelle der Maus ein menschliches Keimbahn-Vκ1-39-Gensegment ist und das menschliche Vκ3-20-Gensegment in dem Genom der genetisch modifizierten ES-Zelle der Maus ein menschliches Keimbahn-Vκ3-20-Gensegment ist.

10. Verfahren nach einem der Ansprüche 7 bis 9, wobei das Genom der genetisch modifizierten ES-Zelle der Maus in dieser Reihenfolge umfasst: das menschliche Vκ1-39-Gensegment, das menschliche Vκ3-20-Gensegment, das menschliche Jκ1-Gensegment, das menschliche Jκ2-Gensegment, das menschliche Jκ3-Gensegment, das menschliche Jκ4-Gensegment und das menschliche Jκ5-Gensegment.

11. Verfahren nach einem der Ansprüche 6 bis 10, wobei das Genom der genetisch modifizierten ES-Zelle der Maus einen DLC-5J-Locus umfasst, wobei der DLC-5J-Locus eine hVκ/hJκ/mCκ-Knotenpunktsequenz umfasst, ausgewählt aus SEQ ID NO: 38-49.

12. Verfahren zum Herstellen einer genetisch modifizierten Maus, umfassend das genetische Modifizieren des Keimbahngenoms der Maus derart, dass es genau zwei nicht neu geordnete menschliche Immunglobulin-Vκ-Gensegmente und fünf nicht neu geordnete menschliche Immunglobulin-Jκ-Gensegmente umfasst, die mit einer Sequenz der konstanten Region der leichten Kette des Immunglobulins der Maus wirkverbunden sind, wobei die zwei nicht neu geordneten menschlichen Immunglobulin-Vκ-Gensegmente ein menschliches Vκ1-39-Gensegment und ein menschliches Vκ3-20-Gensegment sind, und wobei die fünf nicht neu geordneten menschlichen Immunglobulin-Jκ-Gensegmente ein menschliches Jκ1-Gensegment, ein menschliches Jκ2-Gensegment, ein menschliches Jκ3-Gensegment, ein menschliches Jκ4-Gensegment und ein menschliches Jκ5-Gensegment sind.

13. Verfahren nach Anspruch 12, wobei die genetisch modifizierte Maus ferner in ihrem Genom umfasst:
ein oder mehrere nicht neu geordnete menschliche Immunglobulin-V_{H}-Gensegmente, ein oder mehrere nicht neu geordnete menschliche Immunoglobulin-D_{H}-Gensegmente, und ein oder mehrere nicht neu geordnete menschliche Immunoglobulin-J_{H}-Gensegmente, die mit einer Sequenz der konstanten Region der schweren Kette des Immunglobulins der Maus an den endogenen Loci der schweren Kette wirkverbunden sind.

14. Verfahren nach Anspruch 12 oder 13, wobei:
(a) die zwei nicht neu geordneten menschlichen Immunglobulin-Vκ-Gensegmente und fünf nicht neu geordneten menschlichen Immunglobulin-Jκ-Gensegmente an dem endogenen Locus der leichten κ-Kette des Immunglobulins vorhanden sind;
(b) das Genom der genetisch modifizierten ES-Zelle der Maus keinen funktionellen Locus der leichten λ-Kette umfasst;
(c) das Genom der genetisch modifizierten Maus einen nicht funktionellen Locus der leichten λ-Kette des Immunglobulins umfasst;
(d) die konstante Region der leichten Kette der Maus eine konstante Cκ-Region der Maus ist, wobei optional die konstante Cκ-Region der Maus eine endogene konstante Cκ-Region der Maus ist; oder
(e) das menschliche Vκ1-39-Gensegment in dem Genom der genetisch modifizierten ES-Zelle der Maus ein menschliches Keimbahn-Vκ1-39-Gensegment ist und das menschliche Vκ3-20-Gensegment in dem Genom der genetisch modifizierten ES-Zelle der Maus ein menschliches Keimbahn-Vκ3-20-Gensegment ist.

15. Verfahren nach einem der Ansprüche 12 bis 14, wobei das Genom der genetisch modifizierten Maus in dieser Reihenfolge umfasst: das menschliche Vκ1-39-Gensegment, das menschliche Vκ3-20-Gensegment, das menschliche Jκ1-Gensegment, das menschliche Jκ2-Gensegment, das menschliche Jκ3-Gensegment, das menschliche Jκ4-Gensegment und das menschliche Jκ5-Gensegment.

16. Verfahren nach einem der Ansprüche 12 bis 15, wobei das Genom der genetisch modifizierten Maus einen DLC-5J-Locus umfasst, wobei der DLC-5J-Locus eine hVκ/hJκ/mCκ-Knotenpunktsequenz umfasst, ausgewählt aus SEQ ID NO: 38-49.

## Revendications

1. Cellule souche embryonnaire (ES) de souris qui a été génétiquement modifiée de sorte qu'elle comprend dans son génome :
exactement deux segments de gène Vκ d'immunoglobuline humaine non réarrangés et cinq segments de gène Jκ d'immunoglobuline humaine non réarrangés liés de manière opérationnelle à une séquence de région constante de chaîne légère d'immunoglobuline de souris, dans laquelle les deux segments de gène Vκ d'immunoglobuline humaine non réarrangés sont un segment de gène Vκ1-39 humain et un segment de gène Vκ3-20 humain, et dans laquelle les cinq segments de gène Jκ d'immunoglobuline humaine non réarrangés sont un segment de gène Jκ1 humain, un segment de gène Jκ2 humain, un segment de gène Jκ3 humain, un segment de gène Jκ4 humain, et un segment de gène Jκ5 humain.

2. Cellule ES de souris génétiquement modifiée selon la revendication 1, comprenant en outre dans son génome :
un ou plusieurs segments de gène V_{H} d'immunoglobuline humaine non réarrangés, un ou plusieurs segments de gène D_{H} d'immunoglobuline humaine non réarrangés, et un ou plusieurs segments de gène J_{H} d'immunoglobuline humaine non réarrangés liés de manière opérationnelle à une séquence de région constante de chaîne lourde d'immunoglobuline de souris au niveau des loci de chaîne lourde endogènes.

3. Cellule ES génétiquement modifiée selon la revendication 1 ou 2, dans laquelle :
(a) les deux segments de gène Vκ d'immunoglobuline humaine non réarrangés et les cinq segments de gène Jκ d'immunoglobuline humaine non réarrangés sont présents au niveau du locus de chaîne légère κ d'immunoglobuline endogène ;
(b) le génome de la cellule ES de souris génétiquement modifiée ne comprend pas de locus de chaîne légère λ fonctionnel ;
(c) le génome de la souris génétiquement modifiée comprend un locus de chaîne légère λ d'immunoglobuline non fonctionnel ;
(d) la région constante de chaîne légère de souris est une région constante Cκ de souris, éventuellement dans laquelle la région constante Cκ de souris est une région constante Cκ de souris endogène ; ou
(e) le segment de gène Vκ1-39 humain dans le génome de la cellule ES de souris génétiquement modifiée est un segment de gène Vκ1-39 de lignée germinale humaine et le segment de gène Vκ3-20 humain dans le génome de la cellule ES de souris génétiquement modifiée est un segment de gène Vκ3-20 de lignée germinale humaine.

4. Cellule ES de souris génétiquement modifiée selon l'une quelconque des revendications précédentes, dans laquelle le génome de la cellule ES de souris génétiquement modifiée comprend dans l'ordre : le segment de gène Vκ1-39 humain, le segment de gène Vκ3-20 humain, le segment de gène Jκ1 humain, le segment de gène Jκ2 humain, le segment de gène Jκ3 humain, le segment de gène Jκ4 humain, et le segment de gène Jκ5 humain.

5. Cellule ES de souris génétiquement modifiée selon l'une quelconque des revendications précédentes, dans laquelle le génome de la cellule ES de souris génétiquement modifiée comprend un locus DLC-5J, dans laquelle le locus DLC-5J comprend une séquence de jonction hVκ/hJκ/mCκ sélectionnée parmi SEQ ID N° : 38 à 49.

6. Embryon de souris dérivé de la cellule ES génétiquement modifiée de la revendication 1 ou de la revendication 2.

7. Procédé de production d'une cellule ES de souris génétiquement modifiée, le procédé comprenant la modification génétique d'une cellule ES de sorte qu'elle comprend dans son génome exactement deux segments de gène Vκ d'immunoglobuline humaine non réarrangés et cinq segments de gène Jκ d'immunoglobuline humaine non réarrangés liés de manière opérationnelle à une séquence de région constante de chaîne légère d'immunoglobuline de souris, dans lequel les deux segments de gène Vκ d'immunoglobuline humaine non réarrangés sont un segment de gène Vκ1-39 humain et un segment de gène Vκ3-20 humain, et dans lequel les cinq segments de gène Jκ d'immunoglobuline humaine non réarrangés sont un segment de gène Jκ1 humain, un segment de gène Jκ2 humain, un segment de gène Jκ3 humain, un segment de gène Jκ4 humain, et un segment de gène Jκ5 humain.

8. Procédé selon la revendication 7, dans lequel la cellule ES de souris génétiquement modifiée comprend en outre dans son génome :
un ou plusieurs segments de gène V_{H} d'immunoglobuline humaine non réarrangés, un ou plusieurs segments de gène D_{H} d'immunoglobuline humaine non réarrangés, et un ou plusieurs segments de gène J_{H} d'immunoglobuline humaine non réarrangés, liés de manière opérationnelle à une séquence de région constante de chaîne lourde d'immunoglobuline de souris au niveau des loci de chaîne lourde endogènes.

9. Procédé selon la revendication 7 ou 8, dans lequel :
(a) les deux segments de gène Vκ d'immunoglobuline humaine non réarrangés et les cinq segments de gène Jκ d'immunoglobuline humaine non réarrangés sont présents au niveau du locus de chaîne légère κ d'immunoglobuline endogène ;
(b) le génome de la cellule ES de souris génétiquement modifiée ne comprend pas de locus de chaîne légère λ fonctionnel ;
(c) le génome de la souris génétiquement modifiée comprend un locus de chaîne légère λ d'immunoglobuline non fonctionnel ;
(d) la région constante de chaîne légère de souris est une région constante Cκ de souris, éventuellement dans lequel la région constante Cκ de souris est une région constante Cκ de souris endogène ; ou
(e) le segment de gène Vκ1-39 humain dans le génome de la cellule ES de souris génétiquement modifiée est un segment de gène Vκ1-39 de lignée germinale humaine et le segment de gène Vκ3-20 humain dans le génome de la cellule ES de souris génétiquement modifiée est un segment de gène Vκ3-20 de lignée germinale humaine.

10. Procédé selon l'une quelconque des revendications 7 à 9, dans lequel le génome de la cellule ES de souris génétiquement modifiée comprend dans l'ordre : le segment de gène Vκ1-39 humain, le segment de gène Vκ3-20 humain, le segment de gène Jκ1 humain, le segment de gène Jκ2 humain, le segment de gène Jκ3 humain, le segment de gène Jκ4 humain, et le segment de gène Jκ5 humain.

11. Procédé selon l'une quelconque des revendications 6 à 10, dans lequel le génome de la cellule ES de souris génétiquement modifiée comprend un locus DLC-5J, dans lequel le locus DLC-5J comprend une séquence de jonction hVκ/hJκ/mCκ sélectionnée parmi SEQ ID N° : 38 à 49.

12. Procédé de production d'une souris génétiquement modifiée comprenant la modification génétique du génome germinal de la souris pour qu'il inclue exactement deux segments de gène Vκ d'immunoglobuline humaine non réarrangés et cinq segments de gène Jκ d'immunoglobuline humaine non réarrangés, liés de manière opérationnelle à une séquence de région constante de chaîne légère d'immunoglobuline de souris, dans lequel les deux segments de gène Vκ d'immunoglobuline humaine non réarrangés sont un segment de gène Vκ1-39 humain et un segment de gène Vκ3-20 humain, et dans lequel les cinq segments de gène Jκ d'immunoglobuline humaine non réarrangés sont un segment de gène Jκ1 humain, un segment de gène Jκ2 humain, un segment de gène Jκ3 humain, un segment de gène Jκ4 humain, et un segment de gène Jκ5 humain.

13. Procédé selon la revendication 12, dans lequel la souris génétiquement modifiée comprend en outre dans son génome :
un ou plusieurs segments de gène V_{H} d'immunoglobuline humaine non réarrangés, un ou plusieurs segments de gène D_{H} d'immunoglobuline humaine non réarrangés, et un ou plusieurs segments de gène J_{H} d'immunoglobuline humaine non réarrangés liés de manière opérationnelle à une séquence de région constante de chaîne lourde d'immunoglobuline de souris au niveau des loci de chaîne lourde endogènes.

14. Procédé selon la revendication 12 ou 13, dans lequel :
(a) les deux segments de gène Vκ d'immunoglobuline humaine non réarrangés et les cinq segments de gène Jκ d'immunoglobuline humaine non réarrangés sont présents au niveau du locus de chaîne légère κ d'immunoglobuline endogène ;
(b) le génome de la souris génétiquement modifiée ne comprend pas de locus de chaîne légère λ fonctionnel ;
(c) le génome de la souris génétiquement modifiée comprend un locus de chaîne légère λ d'immunoglobuline non fonctionnel ;
(d) la région constante de chaîne légère de souris est une région constante Cκ de souris, éventuellement dans lequel la région constante Cκ de souris est une région constante Cκ de souris endogène ; ou
(e) le segment de gène Vκ1-39 humain dans le génome de la cellule ES de souris génétiquement modifiée est un segment de gène Vκ1-39 de lignée germinale humaine et le segment de gène Vκ3-20 humain dans le génome de la cellule ES de souris génétiquement modifiée est un segment de gène Vκ3-20 de lignée germinale humaine.

15. Procédé selon l'une quelconque des revendications 12 à 14, dans lequel le génome de la souris génétiquement modifiée comprend dans l'ordre : le segment de gène Vκ1-39 humain, le segment de gène Vκ3-20 humain, le segment de gène Jκ1 humain, le segment de gène Jκ2 humain, le segment de gène Jκ3 humain, le segment de gène Jκ4 humain, et le segment de gène Jκ5 humain.

16. Procédé selon l'une quelconque des revendications 12 à 15, dans lequel le génome de la souris génétiquement modifiée comprend un locus DLC-5J, dans lequel le locus DLC-5J comprend une séquence de jonction hVκ/hJκ/mCκ sélectionnée parmi SEQ ID N° : 38 à 49.
